# EUROPEAN PATENT APPLICATION

(11) **EP 4 559 900 A1**
(43) Date of publication of application: **28.05.2025**
(21) Application number: 23383186.6
(22) Date of filing: 21.11.2023
(51) Int. Cl.: C07D 233/20, A61P 27/00, C07C 309/02

(54) **PHENTOLAMINE MESYLATE AND THERAPEUTIC USES THEREOF**

(71) Applicant: Opus Genetics, Inc., Farmington Hills, MI 48335 (US)
(72) Inventor: ONICIU, Daniela Carmen, Gainesville, 32605 (US); ESPINOSA FERRER, Rosa Maria, 08028 Barcelona (ES); PINA CORONADO, Clara, 08028 Barcelona (ES); BENET CONGOST, Joan, 08028 Barcelona (ES)
(74) Representative: Graham Watt & Co LLP

(57) **Abstract**

This invention provides phentolamine mesylate that exhibits a particular x-ray powder diffraction (XRPD) pattern or exhibits one or more particular XRPD peaks. The invention also provides compositions comprising the phentolamine mesylate and compositions in which the phentolamine mesylate is dissolved. The invention also provides methods for treating or preventing an ocular disease or condition, comprising administering to a subject in need thereof an effective amount of the phentolamine mesylate, a composition comprising the phentolamine mesylate or a composition in which the phentolamine mesylate is dissolved.

## Description

### FIELD OF THE INVENTION

This invention provides phentolamine mesylate that exhibits a particular x-ray powder diffraction (XRPD) pattern or exhibits one or more particular XRPD peaks. The invention also provides compositions comprising the phentolamine mesylate and compositions in which the phentolamine mesylate is dissolved. The invention also provides methods for treating or preventing an ocular disease or condition, comprising administering to a subject in need thereof an effective amount of the phentolamine mesylate, a composition comprising the phentolamine mesylate or a composition in which the phentolamine mesylate is dissolved.

### BACKGROUND OF THE INVENTION

RYZUMVI, an ophthalmic solution of phentolamine mesylate, was approved by the U.S. Food and Drug Administration (FDA) for treatment of pharmacologically induced mydriasis produced by adrenergic agonists or parasympatholytic agents. Phentolamine mesylate for injection was approved by the FDA for use in reversing soft-tissue anesthesia, for use in preventing or controlling hypertensive episodes in patients with pheochromocytoma, and for treatment of dermal necrosis following intravenous administration or extravasation of norepinephrine.

### SUMMARY OF THE INVENTION

The present invention provides phentolamine mesylate that exhibits an XRPD pattern comprising a peak at 8.3 ± 0.2 degrees 2-theta, a peak at 13.1 ± 0.2 degrees 2-theta and a peak at 16.3 ± 0.2 degrees 2-theta.

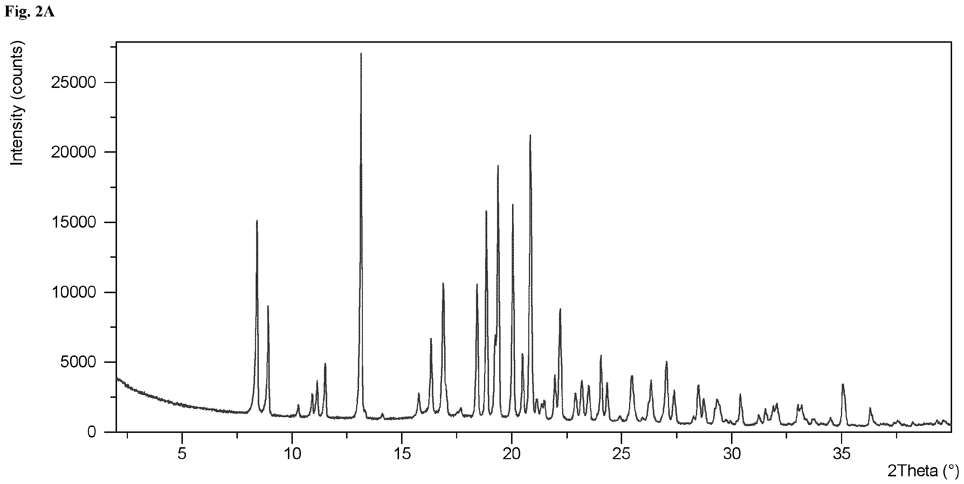

The invention further provides phentolamine mesylate that exhibits an XRPD pattern that is substantially the same as that depicted in **Fig. 2A****.**

The invention further provides phentolamine mesylate that exhibits an XRPD pattern comprising the **Table 3** peaks having at least 50% relative intensity.

The present invention provides phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta.

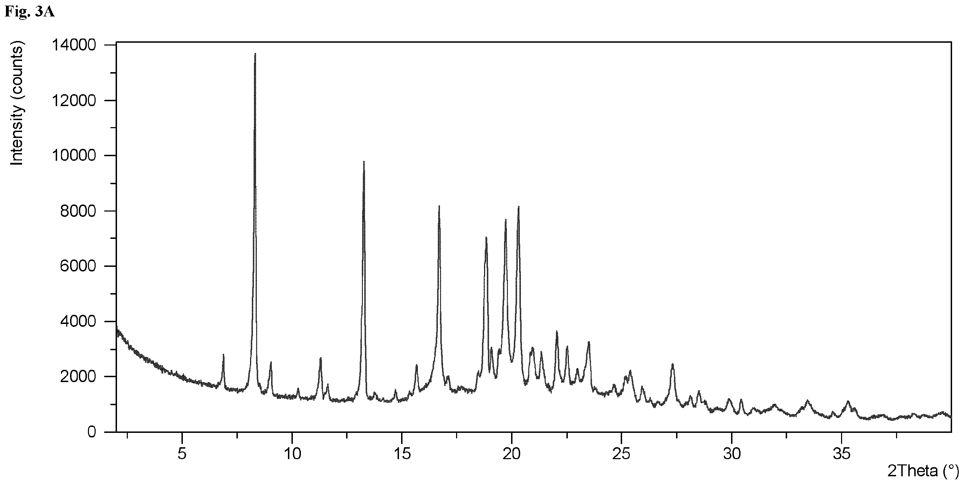

The invention further provides phentolamine mesylate that exhibits an XRPD pattern that is substantially the same as that depicted in **Fig. 3A****.**

The invention further provides phentolamine mesylate that exhibits an XRPD pattern comprising the **Table 4** peaks having at least 50% relative intensity.

Each phentolamine mesylate above is a "compound of the invention".

The present invention further provides compositions comprising a compound of the invention and a pharmaceutically acceptable carrier or vehicle and compositions in which a compound of the invention is dissolved (each composition being a "composition of the invention").

The present invention further provides a sealed container containing a compound of the invention and an inert gas.

The present invention further provides methods for treating or reversing pharmacologically induced mydriasis, comprising administering to a subject in need thereof an effective amount of a compound of the invention or a composition of the invention.

The present invention further provides methods for inhibiting contraction of smooth muscle of a subject's iris, comprising administering to a subject in need thereof an effective amount of a compound of the invention or a composition of the invention.

The present invention further provides methods for reducing a subject's pupil diameter, comprising administering to a subject in need thereof an effective amount of a compound of the invention or a composition of the invention.

The present invention further provides methods for improving visual contrast sensitivity or visual acuity, comprising administering to a subject in need thereof an effective amount of a compound of the invention or a composition of the invention.

The present invention further provides methods for treating or preventing an ocular disease or condition, comprising administering to a subject in need thereof an effective amount of a compound of the invention or a composition of the invention.

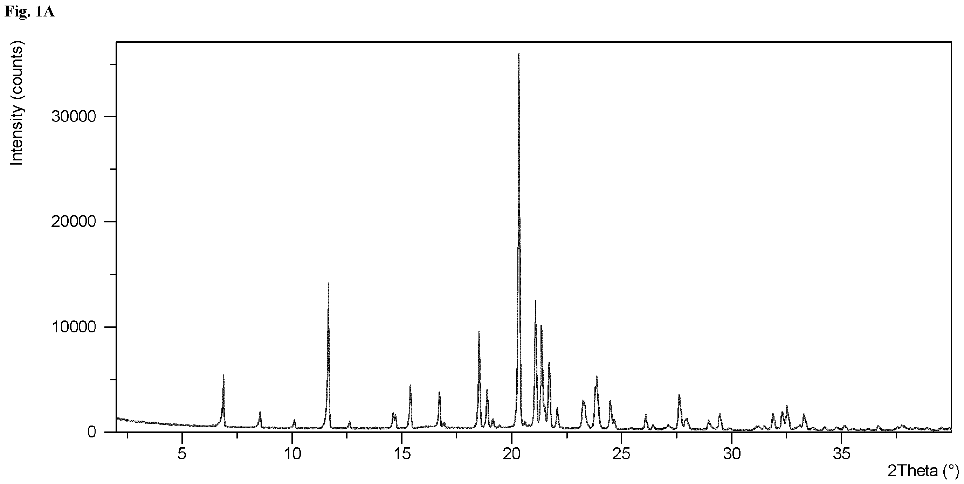

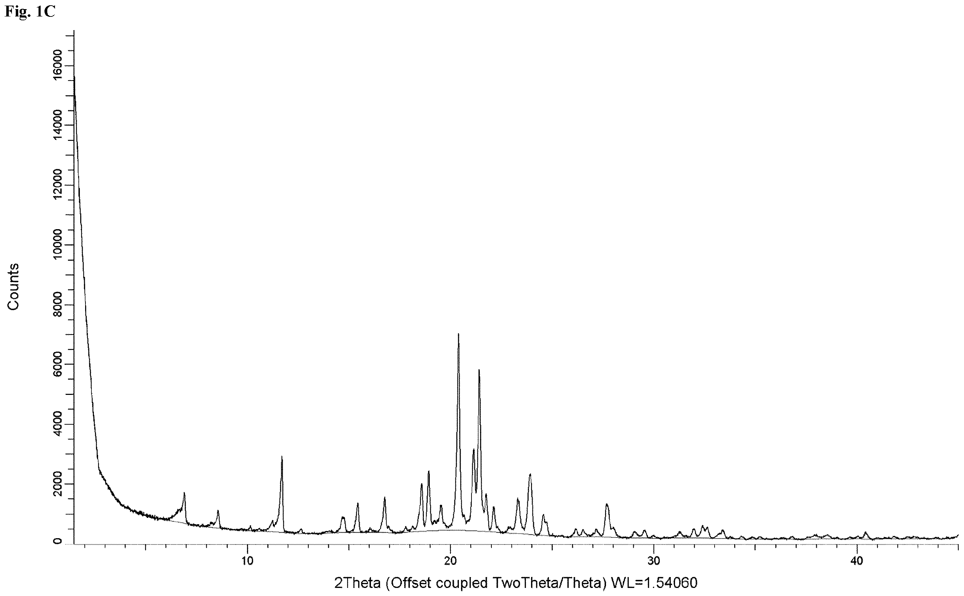

The present invention further provides methods for treating or preventing diabetic retinal disease, retinopathy of prematurity, pathological myopia, hypertensive retinopathy, occlusive vasculitis, polypoidal choroidal vasculopathy, diabetic macular edema, uveitic macular edema, retinal vein occlusion, ocular neovascularization, ocular histoplasmosis, neovascular glaucoma, retinoblastoma, macular degeneration, retrolental fibroplasias, retinal angiomatous proliferation, dry eye disease, uveitis, thyroid eye disease, sickle cell retinopathy, geographic atrophy, choroidal neovascularization, or corneal graft rejection, comprising administering to a subject in need thereof an effective amount of: phentolamine mesylate that exhibits an XRPD pattern that is substantially the same as that depicted in **Fig. 1A** or **Fig. 1C****;** a composition comprising (i) phentolamine mesylate that exhibits an XRPD pattern that is substantially the same as that depicted in **Fig. 1A** or **Fig. 1C** and (ii) a pharmaceutically acceptable carrier or vehicle; or a composition in which phentolamine mesylate that exhibits an XRPD pattern that is substantially the same as that depicted in **Fig. 1A** or **Fig. 1C** is dissolved.

Each of the above methods is an "ocular method of the invention".

The present invention further provides methods for making phentolamine mesylate that exhibits an XRPD pattern comprising a peak at 8.4 ± 0.2 degrees 2-theta and a peak at 16.3 ± 0.2 degrees 2-theta, comprising: (a) heating a mixture comprising phentolamine mesylate comprising an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta; methane sulfonic acid; acetone; and water at about 45 °C for about thirty minutes to provide a heated mixture; (b) allowing the heated mixture to cool to room temperature to provide a cooled mixture; (c) diluting the cooled mixture with t-butyl methyl ether to provide a t-butyl methyl ether mixture; and (d) cooling the t-butyl methyl ether mixture to about -20 °C to precipitate the phentolamine mesylate that exhibits an XRPD pattern comprising a peak at 8.4 ± 0.2 degrees 2-theta and a peak at 16.3 ± 0.2 degrees 2-theta.

The present invention further provides methods for making phentolamine mesylate that exhibits an XRPD pattern comprising a peak at 8.4 ± 0.2 degrees 2-theta and a peak at 16.3 ± 0.2 degrees 2-theta, comprising: (a) heating a mixture comprising phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta and dichloromethane at about 50 °C for about two hours to provide a heated mixture; (b) allowing the heated mixture to cool at about 25 °C for about two hours to provide cooled phentolamine mesylate; and (c) repeating steps (a) and (b) for up to about three days to provide the phentolamine mesylate that exhibits an XRPD pattern comprising a peak at 8.4 ± 0.2 degrees 2-theta and a peak at 16.3 ± 0.2 degrees 2-theta.

The present invention further provides methods for making phentolamine mesylate that exhibits an XRPD pattern comprising a peak at 8.4 ± 0.2 degrees 2-theta and a peak at 16.3 ± 0.2 degrees 2-theta, comprising: (a) dissolving phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta in a mixture comprising methyl ethyl ketone and water at about 80 °C to provide a solution; (b) cooling the solution to about 45 °C and adding one or more seeds of phentolamine mesylate that exhibits an XRPD pattern comprising a peak at 8.4 ± 0.2 degrees 2-theta and a peak at 16.3 ± 0.2 degrees 2-theta to provide a seeded mixture; (c) cooling the seeded mixture at 10 °C for about thirty minutes to provide a cooled mixture; (d) heating the cooled mixture at 35°C under vacuum to reduce the volume of the mixture by about 33% and provide a reduced mixture; and (e) cooling the reduced mixture at 10 °C for about forty minutes to provide the phentolamine mesylate that exhibits an XRPD pattern comprising a peak at 8.4 ± 0.2 degrees 2-theta and a peak at 16.3 ± 0.2 degrees 2-theta.

The present invention further provides methods for making phentolamine mesylate that exhibits an XRPD pattern comprising a peak at 8.4 ± 0.2 degrees 2-theta and a peak at 16.3 ± 0.2 degrees 2-theta, comprising: (a) heating a mixture comprising phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta; methyl ethyl ketone; and water to 77 °C to provide a heated mixture; (b) cooling the heated mixture to 10 °C to provide a cooled mixture; and (c) repeating steps (a) and (b) twice to provide the phentolamine mesylate that exhibits an XRPD pattern comprising a peak at 8.4 ± 0.2 degrees 2-theta and a peak at 16.3 ± 0.2 degrees 2-theta.

The present invention further provides methods for making phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta, comprising (a) heating phentolamine mesylate that exhibits an XRPD pattern comprising a peak at 8.4 ± 0.2 degrees 2-theta and a peak at 16.3 ± 0.2 degrees 2-theta to about 126 °C to provide heated phentolamine mesylate; and (b) allowing the heated phentolamine mesylate to cool to room temperature to provide the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta.

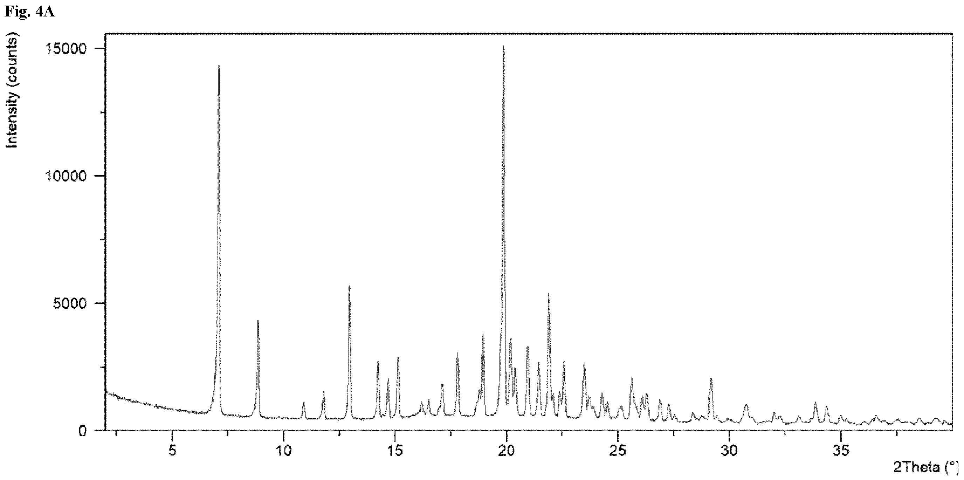

The present invention further provides methods for making phentolamine mesylate that exhibits an XRPD pattern that is substantially the same as that depicted in **Fig. 4A****,** comprising (a) heating a mixture comprising phentolamine mesylate that exhibits an XRPD pattern that is substantially the same as that depicted in **Fig. 1A** or **Fig. 1C** and acetone at about 50 °C for about two hours to provide a heated mixture; (b) allowing the heated mixture to cool at about 25 °C for about two hours to provide cooled phentolamine mesylate; and (c) repeating steps (a) and (b) for up to about three days to provide the phentolamine mesylate that exhibits an XRPD pattern that is substantially the same as that depicted in **Fig. 4A****.**

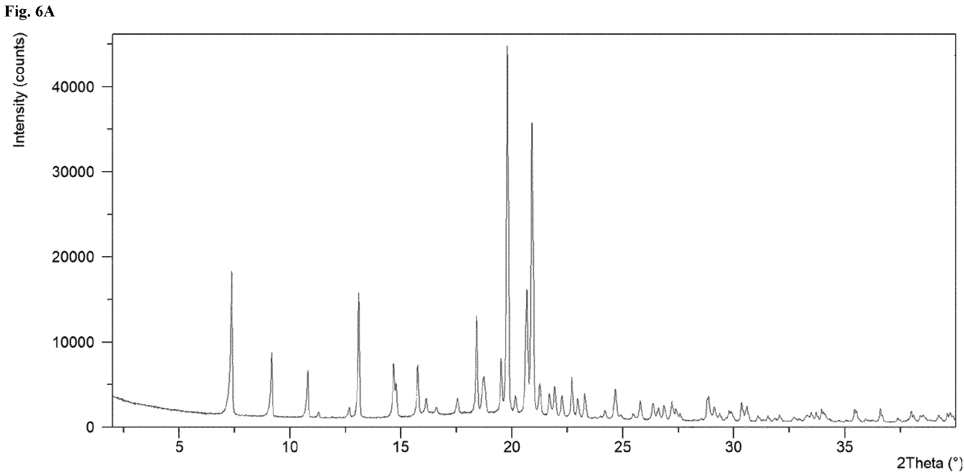

The present invention further provides methods for making phentolamine mesylate that exhibits an XRPD pattern that is substantially the same as that depicted in **Fig. 6A****,** comprising (a) pulverizing phentolamine mesylate that exhibits an XRPD pattern that is substantially the same as that depicted in **Fig. 1A** or **Fig. 1C** in the presence of an excess of 1,4-dioxane solvent to provide a pulverized mixture; and (b) removing the 1,4-dioxane solvent from the pulverized mixture to provide the phentolamine mesylate that exhibits an XRPD pattern that is substantially the same as that depicted in **Fig. 6A****.**

Each of the above methods is a "synthesis method of the invention".

### BRIEF DESCRIPTION OF THE DRAWINGS

**Fig. 1A** depicts an XRPD pattern of phentolamine mesylate United States Pharmacopeia (USP) reference standard purchased from Sigma-Aldrich, Inc. (Product No. 1530004) ("Reference A"). **Fig. 1B** shows a differential scanning calorimetry (DSC) thermogram of the Reference A.
**Fig. 1C** depicts an XRPD pattern of phentolamine mesylate purchased from Chemo Iberica S.A. (Code 690001) ("Reference B"). **Fig. 1D** shows an overlay of a thermogravimetric (TG) thermogram and a DSC thermogram of Reference B.
**Fig. 2A** shows an XRPD pattern of the phentolamine mesylate obtained as described in Example 1. **Fig. 2B** shows a DSC thermogram of the phentolamine mesylate obtained as described in Example 1.
**Fig. 3A** shows an XRPD pattern of the phentolamine mesylate obtained as described in Example 2. **Fig. 3B** shows a DSC thermogram of the phentolamine mesylate obtained as described in Example 2.
**Fig. 4A** shows an XRPD pattern of the phentolamine mesylate acetone solvate obtained as described in Example 3. **Fig. 4B** shows a DSC thermogram of the phentolamine mesylate acetone solvate obtained as described in Example 3. **Fig. 4C** shows a TG thermogram of the phentolamine mesylate acetone solvate obtained as described in Example 3. **Fig. 4D** shows a ¹H NMR spectrum of the phentolamine mesylate acetone solvate obtained as described in Example 3.
**Fig. 5A** shows an XRPD pattern of the phentolamine mesylate obtained as described in Example 4. **Fig. 5B** shows a DSC thermogram of the phentolamine mesylate obtained as described in Example 4. **Fig. 5C** shows a TG thermogram of the phentolamine mesylate obtained as described in Example 4.
**Fig. 6A** shows an XRPD pattern of the phentolamine mesylate dioxane solvate obtained as described in Example 5. **Fig. 6B** shows a DSC thermogram of the phentolamine mesylate dioxane solvate obtained as described in Example 5. **Fig. 6C** shows a TG thermogram of the phentolamine mesylate dioxane solvate obtained as described in Example 5. **Fig. 6D** shows a ¹H NMR spectrum of the phentolamine mesylate dioxane solvate obtained as described in Example 5.
**Fig. 7** shows an XRPD pattern of the phentolamine mesylate obtained as described in Example 6.
**Fig. 8** shows an overlay of an XRPD pattern of the phentolamine mesylate obtained as described in Example 7 (top) and the XRPD pattern of the phentolamine mesylate of **Fig. 7** (bottom).
**Fig. 9** shows an XRPD pattern of the phentolamine mesylate obtained as described in Example 8.
**Fig. 10** shows an overlay of an XRPD pattern of the phentolamine mesylate obtained as described in Example 9 (bottom) and the XRPD pattern of the phentolamine mesylate of **Fig. 2A** (top).
**Fig. 11** shows an XRPD pattern of the phentolamine mesylate obtained as described in Example 10.
**Fig. 12** shows an XRPD pattern of the phentolamine mesylate obtained as described in Example 11.
**Fig. 13** shows an XRPD pattern of the phentolamine mesylate obtained as described in Example 12.

### DETAILED DESCRIPTION OF THE INVENTION

### Definitions

The term "about" when immediately preceding a numerical value means ± 10% of the numerical value.

Throughout the present specification, numerical ranges are provided for certain quantities. These ranges comprise all subranges therein. Thus, the range "from 50 to 80" includes all possible ranges therein (e.g., 51-79, 52-78, 53-77, 54-76, 55-75, 60-70, etc.). Furthermore, all values within a given range may be an endpoint for the range encompassed thereby (e.g., the range 50-80 includes the ranges with endpoints such as 55-80, 50-75, etc.).

As used herein, "effective amount" refers to an amount of a compound or composition that is effective to treat or reverse pharmacologically induced mydriasis; inhibit contraction of smooth muscle of a subject's iris; reduce a subject's pupil diameter; improve visual contrast sensitivity or visual acuity; treat or prevent an ocular disease or condition; or treat or prevent diabetic retinal disease, retinopathy of prematurity, pathological myopia, hypertensive retinopathy, occlusive vasculitis, polypoidal choroidal vasculopathy, diabetic macular edema, uveitic macular edema, retinal vein occlusion, ocular neovascularization, ocular histoplasmosis, neovascular glaucoma, retinoblastoma, macular degeneration, retrolental fibroplasias, retinal angiomatous proliferation, dry eye disease, uveitis, thyroid eye disease, sickle cell retinopathy, geographic atrophy, choroidal neovascularization, or corneal graft rejection.

All weight percentages (i.e., "% by weight" and "wt. %" and w/w) referenced herein, unless otherwise indicated, are relative to the total weight of the compound or composition, as the case may be.

As used herein in connection with a compound of the invention, an "impurity" is a compound or substance other than phentolamine mesylate.

As used herein, "isolated" means isolated from a chemical synthesis reaction mixture. In some embodiments, an isolated compound is at least 95% pure and comprises no more than 5% of one or more impurities by weight, moles, or volume. By "x% pure" means that a compound includes no more than (100-x)% of one or more impurities by weight, moles, or volume. In some embodiments, an isolated compound is at least 96%, at least 97%, at least 98%, or at least 99% pure, and comprises no more than 4%, no more than 3%, no more than 2%, or no more than 1% of an impurity, respectively, by weight, moles, or volume. In some embodiments, the one or more impurities, if any, are present in the compound as a percent by weight. In some embodiments, the one or more impurities, if any, are present in the compound as a percent by mole. In some embodiments, the one or more impurities, if any, are present in the compound as a percent by volume.

As used herein, "room temperature" when used in connection with the synthesis methods of the invention is a temperature from about 20 °C to about 25 °C.

As used herein, "substantially the same as" when used in connection with an XRPD pattern means that each peak (having an at least 2% relative intensity) of the XRPD pattern differs from a corresponding peak of a reference or comparative XRPD pattern by no more than ± 0.2 degrees 2-theta. The most intense peak in an XRPD pattern is assigned 100% relative peak intensity and the intensity of all other peaks in the XRPD pattern is measured relative to the XRPD pattern's most intense peak (relative intensity).

As used herein, "substantially the same as" when used in connection with a DSC thermogram means that each peak of the DSC thermogram differs from a corresponding peak of a reference or comparative DSC thermogram by no more than ± 3 °C.

As used herein, "substantially the same as" when used in connection with a TG thermogram means that each peak of the TG thermogram differs from a corresponding peak of a reference or comparative TG thermogram by no more than ± 2%.

As used herein, "stable" when used in connection with a compound means that the compound comprises no more than 10% of a degradation product by weight of the compound.

As used herein, "subject" a mammal. In some embodiments, the mammal is a human.

### Compounds of the Invention

The present invention provides phentolamine mesylate that exhibits a particular XRPD pattern or exhibits one or more particular XRPD peaks.

Phentolamine mesylate has the structure:

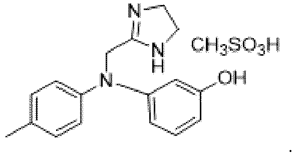

In some embodiments, the phentolamine mesylate is crystalline. In some embodiments, the phentolamine mesylate is a phentolamine mesylate solvate. In some embodiments, the phentolamine mesylate solvate is crystalline.

In some embodiments, the compound of the invention is phentolamine mesylate that exhibits an XRPD pattern comprising a peak at 8.4 ± 0.2 degrees 2-theta and a peak at 16.3 ± 0.2 degrees 2-theta. In some embodiments, the XRPD pattern further comprises a peak at 13.1 ± 0.2 degrees 2-theta, a peak at 19.4 ± 0.2 degrees 2-theta, or a peak at 20.8 ± 0.2 degrees 2-theta. In some embodiments, the XRPD pattern further comprises a peak at 18.8 ± 0.2 degrees 2-theta or a peak at 20.0 ± 0.2 degrees 2-theta. In some embodiments, the XRPD pattern further comprises a peak at 16.9 ± 0.2 degrees 2-theta or a peak at 18.4 ± 0.2 degrees 2-theta. In some embodiments, the XRPD pattern further comprises a peak at 8.9 ± 0.2 degrees 2-theta or a peak at 22.2 ± 0.2 degrees 2-theta. In some embodiments, the XRPD pattern further comprises a peak at 11.1 ± 0.2 degrees 2-theta or a peak at 19.2 ± 0.2 degrees 2-theta.

In some embodiments, the compound of the invention is phentolamine mesylate that exhibits an XRPD pattern comprising a peak at 8.4 ± 0.2 degrees 2-theta, a peak at 13.1 ± 0.2 degrees 2-theta, a peak at 16.3 ± 0.2 degrees 2-theta, a peak at 19.4 ± 0.2 degrees 2-theta, and a peak at 20.8 ± 0.2 degrees 2-theta. In some embodiments, the XRPD pattern further comprises a peak at 18.8 ± 0.2 degrees 2-theta or a peak at 20.0 ± 0.2 degrees 2-theta. In some embodiments, the XRPD pattern further comprises a peak at 16.9 ± 0.2 degrees 2-theta or a peak at 18.4 ± 0.2 degrees 2-theta. In some embodiments, the XRPD pattern further comprises a peak at 8.9 ± 0.2 degrees 2-theta or a peak at 22.2 ± 0.2 degrees 2-theta. In some embodiments, the XRPD pattern further comprises a peak at 11.1 ± 0.2 degrees 2-theta or a peak at 19.2 ± 0.2 degrees 2-theta.

In some embodiments, the compound of the invention is phentolamine mesylate that exhibits an XRPD pattern comprising a peak at 8.4 ± 0.2 degrees 2-theta, a peak at 13.1 ± 0.2 degrees 2-theta, a peak at 16.3 ± 0.2 degrees 2-theta, a peak at 18.8 ± 0.2 degrees 2-theta, and a peak at 20.0 ± 0.2 degrees 2-theta. In some embodiments, the XRPD pattern further comprises a peak at 19.4 ± 0.2 degrees 2-theta or a peak at 20.8 ± 0.2 degrees 2-theta. In some embodiments, the XRPD pattern further comprises a peak at 16.9 ± 0.2 degrees 2-theta or a peak at 18.4 ± 0.2 degrees 2-theta. In some embodiments, the XRPD pattern further comprises a peak at 8.9 ± 0.2 degrees 2-theta or a peak at 22.2 ± 0.2 degrees 2-theta. In some embodiments, the XRPD pattern further comprises a peak at 11.1 ± 0.2 degrees 2-theta or a peak at 19.2 ± 0.2 degrees 2-theta.

In some embodiments, the compound of the invention is phentolamine mesylate that exhibits an XRPD pattern comprising a peak at 13.1 ± 0.2 degrees 2-theta, a peak at 16.3 ± 0.2 degrees 2-theta, a peak at 16.9 ± 0.2 degrees 2-theta, and a peak at 18.4 ± 0.2 degrees 2-theta. In some embodiments, the XRPD pattern further comprises a peak at 8.4 ± 0.2 degrees 2-theta, a peak at 19.4 ± 0.2 degrees 2-theta, or a peak at 20.8 ± 0.2 degrees 2-theta. In some embodiments, the XRPD pattern further comprises a peak at 18.8 ± 0.2 degrees 2-theta or a peak at 20.0 ± 0.2 degrees 2-theta. In some embodiments, the XRPD pattern further comprises a peak at 8.9 ± 0.2 degrees 2-theta or a peak at 22.2 ± 0.2 degrees 2-theta. In some embodiments, the XRPD pattern further comprises a peak at 11.1 ± 0.2 degrees 2-theta or a peak at 19.2 ± 0.2 degrees 2-theta.

In some embodiments, the compound of the invention is phentolamine mesylate that exhibits an XRPD pattern comprising a peak at 8.4 ± 0.2 degrees 2-theta, a peak at 13.1 ± 0.2 degrees 2-theta, a peak at 16.3 ± 0.2 degrees 2-theta, a peak at 18.8 ± 0.2 degrees 2-theta, a peak at 19.4 ± 0.2 degrees 2-theta, a peak at 20.0 ± 0.2 degrees 2-theta, and a peak at 20.8 ± 0.2 degrees 2-theta. In some embodiments, the XRPD pattern further comprises a peak at 16.9 ± 0.2 degrees 2-theta or a peak at 18.4 ± 0.2 degrees 2-theta. In some embodiments, the XRPD pattern further comprises a peak at 8.9 ± 0.2 degrees 2-theta or a peak at 22.2 ± 0.2 degrees 2-theta. In some embodiments, the XRPD pattern further comprises a peak at 11.1 ± 0.2 degrees 2-theta or a peak at 19.2 ± 0.2 degrees 2-theta.

In some embodiments, the compound of the invention is phentolamine mesylate that exhibits an XRPD pattern comprising a peak at 8.4 ± 0.2 degrees 2-theta, a peak at 13.1 ± 0.2 degrees 2-theta, a peak at 16.3 ± 0.2 degrees 2-theta, a peak at 19.4 ± 0.2 degrees 2-theta, and a peak at 20.8 ± 0.2 degrees 2-theta. In some embodiments, the XRPD pattern further comprises a peak at 8.9 ± 0.2 degrees 2-theta, a peak at 10.3 ± 0.2 degrees 2-theta, a peak at 10.9 ± 0.2 degrees 2-theta, a peak at 11.1 ± 0.2 degrees 2-theta, a peak at 16.9 ± 0.2 degrees 2-theta, a peak at 18.4 ± 0.2 degrees 2-theta, a peak at 18.8 ± 0.2 degrees 2-theta, a peak at 19.2 ± 0.2 degrees 2-theta, a peak at 20.0 ± 0.2 degrees 2-theta or a peak at 22.2 ± 0.2 degrees 2-theta. In some embodiments, the XRPD pattern further comprises a peak at 8.9 ± 0.2 degrees 2-theta, a peak at 10.3 ± 0.2 degrees 2-theta, a peak at 10.9 ± 0.2 degrees 2-theta, a peak at 11.1 ± 0.2 degrees 2-theta, a peak at 16.9 ± 0.2 degrees 2-theta, a peak at 18.4 ± 0.2 degrees 2-theta, a peak at 18.8 ± 0.2 degrees 2-theta, a peak at 19.2 ± 0.2 degrees 2-theta, a peak at 20.0 ± 0.2 degrees 2-theta and a peak at 22.2 ± 0.2 degrees 2-theta.

In some embodiments, the compound of the invention is phentolamine mesylate that exhibits an XRPD pattern comprising a peak at 8.4 ± 0.2 degrees 2-theta and a peak at 16.3 ± 0.2 degrees 2-theta, wherein the peak at 8.4 ± 0.2 degrees 2-theta and the peak at 16.3 ± 0.2 degrees 2-theta have a relative peak intensity (%) of greater than 10%. In some embodiments, the XRPD pattern further comprises a peak at 13.1 ± 0.2 degrees 2-theta, a peak at 19.4 ± 0.2 degrees 2-theta, or a peak at 20.8 ± 0.2 degrees 2-theta, wherein the peak at 8.4 ± 0.2 degrees 2-theta, the peak at 19.4 ± 0.2 degrees 2-theta, or the peak at 20.8 ± 0.2 degrees 2-theta has a relative peak intensity (%) of greater than 30%. In some embodiments, the XRPD pattern further comprises a peak at 18.8 ± 0.2 degrees 2-theta or a peak at 20.0 ± 0.2 degrees 2-theta, wherein the peak at 18.8 ± 0.2 degrees 2-theta or the peak at 20.0 ± 0.2 degrees 2-theta has a relative peak intensity (%) of greater than 40%. In some embodiments, the XRPD pattern further comprises a peak at 16.9 ± 0.2 degrees 2-theta or a peak at 18.4 ± 0.2 degrees 2-theta, wherein the peak at 16.9 ± 0.2 degrees 2-theta or the peak at 18.4 ± 0.2 degrees 2-theta has a relative peak intensity (%) of greater than 30%. In some embodiments, the XRPD pattern further comprises a peak at 8.9 ± 0.2 degrees 2-theta or a peak at 22.2 ± 0.2 degrees 2-theta, wherein the peak at 8.9 ± 0.2 degrees 2-theta or the peak at 22.2 ± 0.2 degrees 2-theta has a relative peak intensity (%) of greater than 20%. In some embodiments, the XRPD pattern further comprises a peak at 11.1 ± 0.2 degrees 2-theta or a peak at 19.2 ± 0.2 degrees 2-theta, wherein the peak at 11.1 ± 0.2 degrees 2-theta or the peak at 19.2 ± 0.2 degrees 2-theta has a relative peak intensity (%) of greater than 10%.

In some embodiments, the compound of the invention is phentolamine mesylate that exhibits an XRPD pattern comprising a peak at 16.3 ± 0.2 degrees 2-theta. In some embodiments, the peak at 16.3 ± 0.2 degrees 2-theta has a relative peak intensity (%) of greater than 10%. In some embodiments, the peak at 16.3 ± 0.2 degrees 2-theta has a relative peak intensity (%) of greater than 15%.

In some embodiments, the compound of the invention is phentolamine mesylate that exhibits an XRPD pattern comprising a peak at 13.1 ± 0.2 degrees 2-theta, wherein the peak at 13.1 ± 0.2 degrees 2-theta has a relative peak intensity (%) of greater than 75%. In some embodiments, the peak at 13.1 ± 0.2 degrees 2-theta has a relative peak intensity (%) of greater than 80%. In some embodiments, the peak at 13.1 ± 0.2 degrees 2-theta is the XRPD pattern's most intense peak.

In some embodiments, the compound of the invention is phentolamine mesylate that exhibits an XRPD pattern comprising a peak at 8.4 ± 0.2 degrees 2-theta and a peak at 16.3 ± 0.2 degrees 2-theta, wherein the peak at 8.4 ± 0.2 degrees 2-theta has a relative peak intensity (%) of greater than 30% and the peak at 16.3 ± 0.2 degrees 2-theta has a relative peak intensity (%) of greater than 10%.

In some embodiments, the compound of the invention is phentolamine mesylate that exhibits an XRPD pattern comprising a peak at 8.4 ± 0.2 degrees 2-theta and 1 other peak, 2 other peaks, 3 other peaks, 4 other peaks, 5 other peaks, 6 other peaks, 7 other peaks, 8 other peaks, or 9 other peaks of **Table 3.** In some embodiments, the phentolamine mesylate exhibits an XRPD pattern comprising a peak at 8.4 ± 0.2 degrees 2-theta and 1 peak, 2 peaks, 3 peaks, 4 peaks, 5 peaks, 6 peaks, 7 peaks, 8 peaks, 9 peaks or 10 peaks of the following peaks: a peak at 8.9 ± 0.2 degrees 2-theta, a peak at 13.1 ± 0.2 degrees 2-theta, a peak at 16.3 ± 0.2 degrees 2-theta, a peak at 16.9 ± 0.2 degrees 2-theta, a peak at 18.4 ± 0.2 degrees 2-theta, a peak at 18.8 ± 0.2 degrees 2-theta, a peak at 19.4 ± 0.2 degrees 2-theta, a peak at 20.0 ± 0.2 degrees 2-theta, a peak at 20.8 ± 0.2 degrees 2-theta, and a peak at 22.2 ± 0.2 degrees 2-theta.

In some embodiments, the phentolamine mesylate exhibits an XRPD pattern comprising a **Table 3** peak having a relative peak intensity (%) of greater than 50%. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 30%. In some embodiments, the phentolamine mesylate exhibits an XRPD pattern comprising a **Table 3** peak having a relative peak intensity (%) of greater than 20%. In some embodiments, the phentolamine mesylate exhibits an XRPD pattern comprising a **Table 3** peak having a relative peak intensity (%) of greater than 15%. In some embodiments, the phentolamine mesylate exhibits an XRPD pattern comprising a **Table 3** peak having a relative peak intensity (%) of greater than 10%.

In some embodiments, the phentolamine mesylate exhibits an XRPD pattern that is substantially the same as that depicted in **Fig. 2A****.**

In some embodiments, the phentolamine mesylate exhibits a DSC thermogram comprising an endothermic peak having a peak maximum at a temperature of from about 110 °C to about 130 °C. In some embodiments, the phentolamine mesylate exhibits a DSC thermogram comprising an endothermic peak having a peak maximum at a temperature of from about 115 °C to about 125 °C. In some embodiments, the phentolamine mesylate exhibits a DSC thermogram comprising an endothermic peak having a peak maximum at a temperature of about 120 °C.

In some embodiments, the phentolamine mesylate exhibits a DSC thermogram comprising an endothermic peak having a peak maximum at a temperature of from about 120 °C to about 140 °C. In some embodiments, the phentolamine mesylate exhibits a DSC thermogram comprising an endothermic peak having a peak maximum at a temperature of from about 125 °C to about 135 °C. In some embodiments, the phentolamine mesylate exhibits a DSC thermogram comprising an endothermic peak having a peak maximum at a temperature of about 133 °C.

In some embodiments, the phentolamine mesylate exhibits a DSC thermogram comprising an endothermic peak having a peak maximum at a temperature of from about 170 °C to about 190 °C. In some embodiments, the phentolamine mesylate exhibits a DSC thermogram comprising an endothermic peak having a peak maximum at a temperature of from about 175 °C to about 185 °C. In some embodiments, the phentolamine mesylate exhibits a DSC thermogram comprising an endothermic peak having a peak maximum at a temperature of about 180 °C.

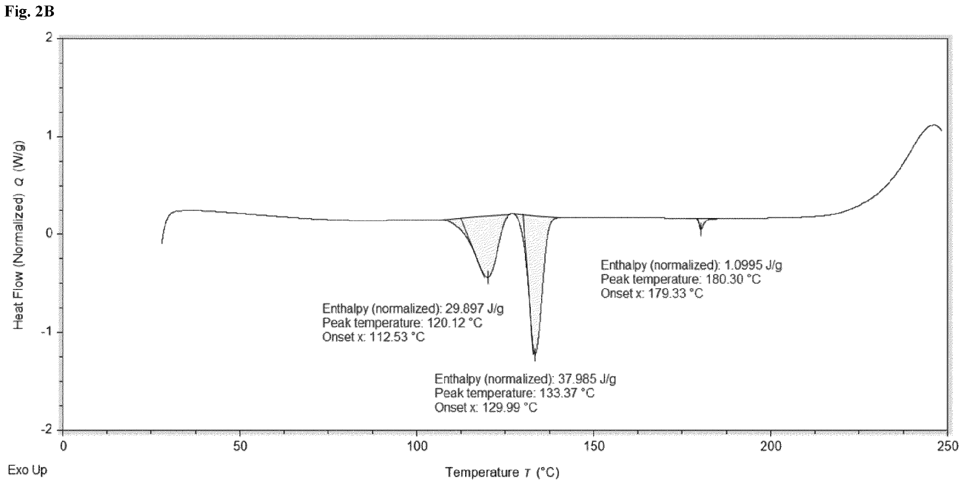

In some embodiments, the phentolamine mesylate exhibits a DSC thermogram that is substantially the same as that depicted in **Fig. 2B****.**

In some embodiments, the compound of the invention is phentolamine mesylate obtained according to Example 1.

In some embodiments, the phentolamine mesylate obtained according to Example 1 is at least about 95% pure by weight. In some embodiments, the phentolamine mesylate obtained according to Example 1 is at least about 98% pure by weight. In some embodiments, the phentolamine mesylate obtained according to Example 1 is at least about 99% pure by weight.

In some embodiments, the phentolamine mesylate obtained according to Example 1 is at least about 90% pure by weight after being exposed to 25 °C/60% relative humidity (RH) for 3 weeks. In some embodiments, the phentolamine mesylate obtained according to Example 1 is at least about 95% pure by weight after being exposed to 25 °C/60% RH for 3 weeks. In some embodiments, the phentolamine mesylate obtained according to Example 1 is at least about 98% pure by weight after being exposed to 25 °C/60% RH for 3 weeks.

In some embodiments, the compound of the invention is phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta. In some embodiments, the XRPD pattern further comprises a peak at 16.7 ± 0.2 degrees 2-theta or a peak at 20.3 ± 0.2 degrees 2-theta. In some embodiments, the XRPD pattern further comprises a peak at 18.8 ± 0.2 degrees 2-theta. In some embodiments, the XRPD pattern further comprises a peak at 18.9 ± 0.2 degrees 2-theta. In some embodiments, the XRPD pattern further comprises a peak at 22.0 ± 0.2 degrees 2-theta or a peak at 23.5 ± 0.2 degrees 2-theta. In some embodiments, the XRPD pattern further comprises a peak at 11.3 ± 0.2 degrees 2-theta or a peak at 19.1 ± 0.2 degrees 2-theta.

In some embodiments, the compound of the invention is phentolamine mesylate that exhibits an XRPD pattern comprising a peak at 8.3 ± 0.2 degrees 2-theta, a peak at 13.3 ± 0.2 degrees 2-theta, a peak at 16.7 ± 0.2 degrees 2-theta, a peak at 19.7 ± 0.2 degrees 2-theta, and a peak at 20.3 ± 0.2 degrees 2-theta. In some embodiments, the XRPD pattern further comprises a peak at 18.8 ± 0.2 degrees 2-theta. In some embodiments, the XRPD pattern further comprises a peak at 18.9 ± 0.2 degrees 2-theta. In some embodiments, the XRPD pattern further comprises a peak at 22.0 ± 0.2 degrees 2-theta or a peak at 23.5 ± 0.2 degrees 2-theta. In some embodiments, the XRPD pattern further comprises a peak at 11.3 ± 0.2 degrees 2-theta or a peak at 19.1 ± 0.2 degrees 2-theta.

In some embodiments, the compound of the invention is phentolamine mesylate that exhibits an XRPD pattern comprising a peak at 8.3 ± 0.2 degrees 2-theta, a peak at 13.3 ± 0.2 degrees 2-theta, a peak at 18.8 ± 0.2 degrees 2-theta, and a peak at 19.7 ± 0.2 degrees 2-theta. In some embodiments, the XRPD pattern further comprises a peak at 16.7 ± 0.2 degrees 2-theta or a peak at 20.3 ± 0.2 degrees 2-theta. In some embodiments, the XRPD pattern further comprises a peak at 18.9 ± 0.2 degrees 2-theta. In some embodiments, the XRPD pattern further comprises a peak at 22.0 ± 0.2 degrees 2-theta or a peak at 23.5 ± 0.2 degrees 2-theta. In some embodiments, the XRPD pattern further comprises a peak at 11.3 ± 0.2 degrees 2-theta or a peak at 19.1 ± 0.2 degrees 2-theta.

In some embodiments, the compound of the invention is phentolamine mesylate that exhibits an XRPD pattern comprising a peak at 8.3 ± 0.2 degrees 2-theta, a peak at 13.3 ± 0.2 degrees 2-theta, a peak at 19.7 ± 0.2 degrees 2-theta, a peak at 22.0 ± 0.2 degrees 2-theta, and a peak at 23.5 ± 0.2 degrees 2-theta. In some embodiments, the XRPD pattern further comprises a peak at 16.7 ± 0.2 degrees 2-theta or a peak at 20.3 ± 0.2 degrees 2-theta. In some embodiments, the XRPD pattern further comprises a peak at 18.8 ± 0.2 degrees 2-theta. In some embodiments, the XRPD pattern further comprises a peak at 18.9 ± 0.2 degrees 2-theta. In some embodiments, the XRPD pattern further comprises a peak at 11.3 ± 0.2 degrees 2-theta or a peak at 19.1 ± 0.2 degrees 2-theta.

In some embodiments, the compound of the invention is phentolamine mesylate that exhibits an XRPD pattern comprising a peak at 8.3 ± 0.2 degrees 2-theta, a peak at 13.3 ± 0.2 degrees 2-theta, a peak at 16.7 ± 0.2 degrees 2-theta, a peak at 18.8 ± 0.2 degrees 2-theta, a peak at 19.7 ± 0.2 degrees 2-theta, and a peak at 20.3 ± 0.2 degrees 2-theta. In some embodiments, the XRPD pattern further comprises a peak at 18.9 ± 0.2 degrees 2-theta. In some embodiments, the XRPD pattern further comprises a peak at 22.0 ± 0.2 degrees 2-theta or a peak at 23.5 ± 0.2 degrees 2-theta. In some embodiments, the XRPD pattern further comprises a peak at 11.3 ± 0.2 degrees 2-theta or a peak at 19.1 ± 0.2 degrees 2-theta.

In some embodiments, the XRPD pattern comprising a peak at 8.3 ± 0.2 degrees 2-theta, a peak at 13.3 ± 0.2 degrees 2-theta and a peak at 19.7 ± 0.2 degrees 2-theta does not comprise a peak at 6.9 ± 0.2 degrees 2-theta or a peak at 11.6 ± 0.2 degrees 2-theta. In some embodiments, the XRPD pattern comprising a peak at 8.3 ± 0.2 degrees 2-theta, a peak at 13.3 ± 0.2 degrees 2-theta and a peak at 19.7 ± 0.2 degrees 2-theta does not comprise a peak at 6.9 ± 0.2 degrees 2-theta and a peak at 11.6 ± 0.2 degrees 2-theta.

In some embodiments, the compound of the invention is phentolamine mesylate that exhibits an XRPD pattern comprising a peak at a peak at 8.3 ± 0.2 degrees 2-theta, a peak at 11.6 ± 0.2 degrees 2-theta, a peak at 13.3 ± 0.2 degrees 2-theta, a peak at 16.7 ± 0.2 degrees 2-theta, and a peak at 19.7 ± 0.2 degrees 2-theta. In some embodiments, the XRPD pattern further comprises a peak at 9.0 ± 0.2 degrees 2-theta, a peak at 10.3 ± 0.2 degrees 2-theta, a peak at 11.3 ± 0.2 degrees 2-theta, a peak at 18.9 ± 0.2 degrees 2-theta, a peak at 19.1 ± 0.2 degrees 2-theta, a peak at 20.3 ± 0.2 degrees 2-theta, a peak at 22.0 ± 0.2 degrees 2-theta, or a peak at 23.5 ± 0.2 degrees 2-theta. In some embodiments, the XRPD pattern further comprises a peak at 9.0 ± 0.2 degrees 2-theta, a peak at 10.3 ± 0.2 degrees 2-theta, a peak at 11.3 ± 0.2 degrees 2-theta, a peak at 18.9 ± 0.2 degrees 2-theta, a peak at 19.1 ± 0.2 degrees 2-theta, a peak at 20.3 ± 0.2 degrees 2-theta, a peak at 22.0 ± 0.2 degrees 2-theta, and a peak at 23.5 ± 0.2 degrees 2-theta.

In some embodiments, the compound of the invention is phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta, wherein the peak at 8.3 ± 0.2 degrees 2-theta, the peak at 13.3 ± 0.2 degrees 2-theta and the peak at 19.7 ± 0.2 degrees 2-theta have a relative peak intensity (%) of greater than 40%. In some embodiments, the XRPD pattern further comprises a peak at 16.7 ± 0.2 degrees 2-theta or a peak at 20.3 ± 0.2 degrees 2-theta, wherein the peak at 16.7 ± 0.2 degrees 2-theta or the peak at 20.3 ± 0.2 degrees 2-theta has a relative peak intensity (%) of greater than 30%. In some embodiments, the XRPD pattern further comprises a peak at 18.8 ± 0.2 degrees 2-theta, wherein the peak at 18.8 ± 0.2 degrees 2-theta has a relative peak intensity (%) of greater than 30%. In some embodiments, the XRPD pattern further comprises a peak at 18.9 ± 0.2 degrees 2-theta, wherein the peak at 18.9 ± 0.2 degrees 2-theta has a relative peak intensity (%) of greater than 30%. In some embodiments, the XRPD pattern further comprises a peak at 22.0 ± 0.2 degrees 2-theta or a peak at 23.5 ± 0.2 degrees 2-theta, wherein the peak at 22.0 ± 0.2 degrees 2-theta or the peak at 23.5 ± 0.2 degrees 2-theta has a relative peak intensity (%) of greater than 10%. In some embodiments, the XRPD pattern further comprises a peak at 11.3 ± 0.2 degrees 2-theta or a peak at 19.1 ± 0.2 degrees 2-theta, wherein the peak at 11.3 ± 0.2 degrees 2-theta or the peak at 19.1 ± 0.2 degrees 2-theta has a relative peak intensity (%) of greater than 10%.

In some embodiments, the compound of the invention is phentolamine mesylate that exhibits an XRPD pattern comprising a peak at 8.3 ± 0.2 degrees 2-theta and a peak at 19.7 ± 0.2 degrees 2-theta, wherein the peak at 19.7 ± 0.2 degrees 2-theta has a relative peak intensity (%) of greater than 30%. In some embodiments, the peak at 19.7 ± 0.2 degrees 2-theta has a relative peak intensity (%) of greater than 40%.

In some embodiments, the compound of the invention is phentolamine mesylate that exhibits an XRPD pattern comprising a peak at 8.3 ± 0.2 degrees 2-theta, wherein the peak at 8.3 ± 0.2 degrees 2-theta has a relative peak intensity (%) of greater than 70%. In some embodiments, the peak at 8.3 ± 0.2 degrees 2-theta has a relative peak intensity (%) of greater than 80%. In some embodiments, the peak at 8.3 ± 0.2 degrees 2-theta is the XRPD pattern's most intense peak.

In some embodiments, the compound of the invention is phentolamine mesylate that exhibits an XRPD pattern comprising a peak at 8.3 ± 0.2 degrees 2-theta and a peak at 13.3 ± 0.2 degrees 2-theta, wherein the peak at 8.3 ± 0.2 degrees 2-theta and the peak at 13.3 ± 0.2 degrees 2-theta have a relative peak intensity (%) of greater than 55%. In some embodiments, the compound of the invention is phentolamine mesylate that exhibits an XRPD pattern comprising a peak at 8.3 ± 0.2 degrees 2-theta and a peak at 13.3 ± 0.2 degrees 2-theta, wherein the peak at 8.3 ± 0.2 degrees 2-theta and the peak at 13.3 ± 0.2 degrees 2-theta have a relative peak intensity (%) of greater than 65%.

In some embodiments, the compound of the invention is phentolamine mesylate that exhibits an XRPD pattern comprising a peak at 8.3 ± 0.2 degrees 2-theta, a peak at 13.3 ± 0.2 degrees 2-theta, a peak at 19.7± 0.2 degrees 2-theta and 1 other peak, 2 other peaks, 3 other peaks, 4 other peaks, 5 other peaks, 6 other peaks, 7 other peaks, 8 other peaks, or 9 other peaks of **Table** 4. In some embodiments, the phentolamine mesylate exhibits an XRPD pattern comprising a peak at 8.3 ± 0.2 degrees 2-theta, a peak at 13.3 ± 0.2 degrees 2-theta, and a peak at 19.7 ± 0.2 degrees 2-theta, and 1 peak, 2 peaks, 3 peaks, 4 peaks, 5 peaks, 6 peaks, 7 peaks, or 8 peaks of the following peaks: a peak at 11.3 ± 0.2 degrees 2-theta, a peak at 16.7 ± 0.2 degrees 2-theta, a peak at 18.8 ± 0.2 degrees 2-theta, a peak at 18.9 ± 0.2 degrees 2-theta, a peak at 19.1 ± 0.2 degrees 2-theta, a peak at 20.3 ± 0.2 degrees 2-theta, a peak at 22.0 ± 0.2 degrees 2-theta, and a peak at 23.5 ± 0.2 degrees 2-theta.

In some embodiments, the phentolamine mesylate exhibits an XRPD pattern comprising a **Table 4** peak having a relative peak intensity (%) of greater than 50%. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 30%. In some embodiments, the phentolamine mesylate exhibits an XRPD pattern comprising a **Table 4** peak having a relative peak intensity (%) of greater than 20%. In some embodiments, the phentolamine mesylate exhibits an XRPD pattern comprising a **Table 4** peak having a relative peak intensity (%) of greater than 15%. In some embodiments, the phentolamine mesylate exhibits an XRPD pattern comprising a **Table 4** peak having a relative peak intensity (%) of greater than 10%.

In some embodiments, the phentolamine mesylate exhibits an XRPD pattern that is substantially the same as that depicted in **Fig. 3A****.**

In some embodiments, the phentolamine mesylate exhibits a DSC thermogram comprising an endothermic peak having a peak maximum at a temperature of from about 120 °C to about 140 °C. In some embodiments, the phentolamine mesylate exhibits a DSC thermogram comprising an endothermic peak having a peak maximum at a temperature of from about 125 °C to about 135 °C. In some embodiments, the phentolamine mesylate exhibits a DSC thermogram comprising an endothermic peak having a peak maximum at a temperature of about 133 °C.

In some embodiments, the phentolamine mesylate exhibits a DSC thermogram comprising an exothermic peak having a peak maximum at a temperature of from about 130 °C to about 150 °C. In some embodiments, the phentolamine mesylate exhibits a DSC thermogram comprising an exothermic peak having a peak maximum at a temperature of from about 135 °C to about 140 °C. In some embodiments, the phentolamine mesylate exhibits a DSC thermogram comprising an exothermic peak having a peak maximum at a temperature of about 138 °C.

In some embodiments, the phentolamine mesylate exhibits a DSC thermogram comprising an endothermic peak having a peak maximum at a temperature of from about 170 °C to about 190 °C. In some embodiments, the phentolamine mesylate exhibits a DSC thermogram comprising an endothermic peak having a peak maximum at a temperature of from about 175 °C to about 185 °C. In some embodiments, the phentolamine mesylate exhibits a DSC thermogram comprising an endothermic peak having a peak maximum at a temperature of about 180 °C.

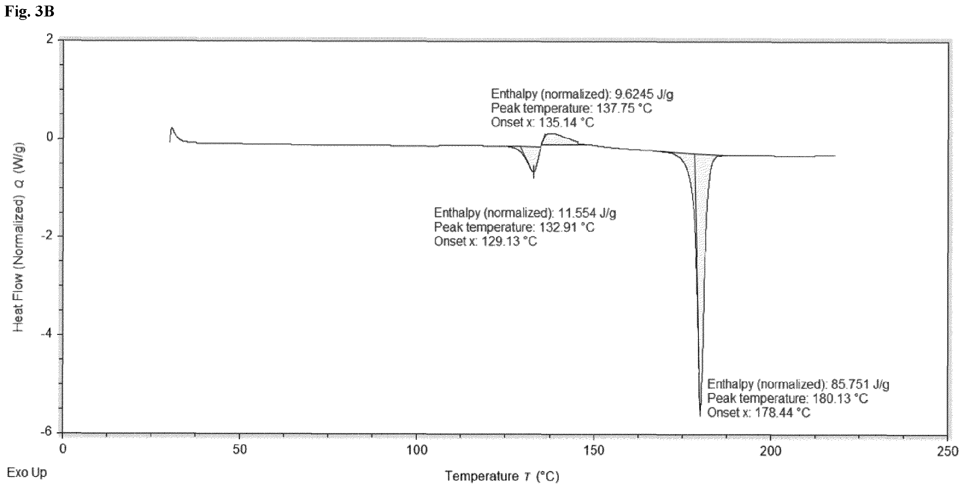

In some embodiments, the phentolamine mesylate exhibits a DSC thermogram that is substantially the same as that depicted in **Fig. 3B****.**

In some embodiments, the compound of the invention is phentolamine mesylate obtained according to Example 2.

In some embodiments, the phentolamine mesylate obtained according to Example 2 is at least about 80% pure by weight. In some embodiments, the phentolamine mesylate obtained according to Example 2 is at least about 85% pure by weight. In some embodiments, the phentolamine mesylate obtained according to Example 2 is at least about 90% pure by weight. In some embodiments, the phentolamine mesylate obtained according to this Example 2 is at least about 95% pure by weight. In some embodiments, the phentolamine mesylate obtained according to Example 2 is at least about 98% pure by weight. In some embodiments, the phentolamine mesylate obtained according to Example 2 is at least about 99% pure by weight.

In some embodiments, the compound of the invention is phentolamine mesylate that exhibits an XRPD pattern comprising a peak at 7.1 ± 0.2 degrees 2-theta, a peak at 8.9 ± 0.2 degrees 2-theta, a peak at 13.0 ± 0.2 degrees 2-theta, a peak at 15.1 ± 0.2 degrees 2-theta, and a peak at 19.9 ± 0.2 degrees 2-theta. In some embodiments, the XRPD pattern further comprises a peak at 11.8 ± 0.2 degrees 2-theta, a peak at 17.8 ± 0.2 degrees 2-theta, a peak at 19.0 ± 0.2 degrees 2-theta, a peak at 20.2 ± 0.2 degrees 2-theta, a peak at 21.0 ± 0.2 degrees 2-theta, or a peak at 21.9 ± 0.2 degrees 2-theta. In some embodiments, the XRPD pattern further comprises a peak at 11.8 ± 0.2 degrees 2-theta, a peak at 17.8 ± 0.2 degrees 2-theta, a peak at 19.0 ± 0.2 degrees 2-theta, a peak at 20.2 ± 0.2 degrees 2-theta, a peak at 21.0 ± 0.2 degrees 2-theta, and a peak at 21.9 ± 0.2 degrees 2-theta.

In some embodiments, the compound of the invention is phentolamine mesylate that exhibits an XRPD pattern comprising a peak at 15.1 ± 0.2 degrees 2-theta and 1 other peak, 2 other peaks, 3 other peaks, 4 other peaks, 5 other peaks, 6 other peaks, 7 other peaks, 8 other peaks, or 9 other peaks of **Table 5.** In some embodiments, the phentolamine mesylate exhibits an XRPD pattern comprising a peak at 15.1 ± 0.2 degrees 2-theta and 1 peak, 2 peaks, 3 peaks, 4 peaks, 5 peaks, 6 peaks, 7 peaks, 8 peaks, 9 peaks or 10 peaks of the following peaks: a peak at 7.1 ± 0.2 degrees 2-theta, a peak at 8.9 ± 0.2 degrees 2-theta, a peak at 11.8 ± 0.2 degrees 2-theta, a peak at 13.0 ± 0.2 degrees 2-theta, a peak at 17.8 ± 0.2 degrees 2-theta, a peak at 19.0 ± 0.2 degrees 2-theta, a peak at 19.9 ± 0.2 degrees 2-theta, a peak at 20.2 ± 0.2 degrees 2-theta, a peak at 21.0 ± 0.2 degrees 2-theta, and a peak at 21.9 ± 0.2 degrees 2-theta.

In some embodiments, the compound of the invention is phentolamine mesylate that exhibits an XRPD pattern that is substantially the same as that depicted in **Fig. 4A****.**

In some embodiments, the phentolamine mesylate exhibits a DSC thermogram comprising an endothermic peak having a peak maximum at a temperature of from about 90 °C to about 110 °C. In some embodiments, the phentolamine mesylate exhibits a DSC thermogram comprising an endothermic peak having a peak maximum at a temperature of from about 95 °C to about 105 °C. In some embodiments, the phentolamine mesylate exhibits a DSC thermogram comprising an endothermic peak having a peak maximum at a temperature of about 100 °C.

In some embodiments, the phentolamine mesylate exhibits a DSC thermogram comprising an endothermic peak having a peak maximum at a temperature of from about 170 °C to about 190 °C. In some embodiments, the phentolamine mesylate exhibits a DSC thermogram comprising an endothermic peak having a peak maximum at a temperature of from about 175 °C to about 185 °C. In some embodiments, the phentolamine mesylate exhibits a DSC thermogram comprising an endothermic peak having a peak maximum at a temperature of about 181 °C.

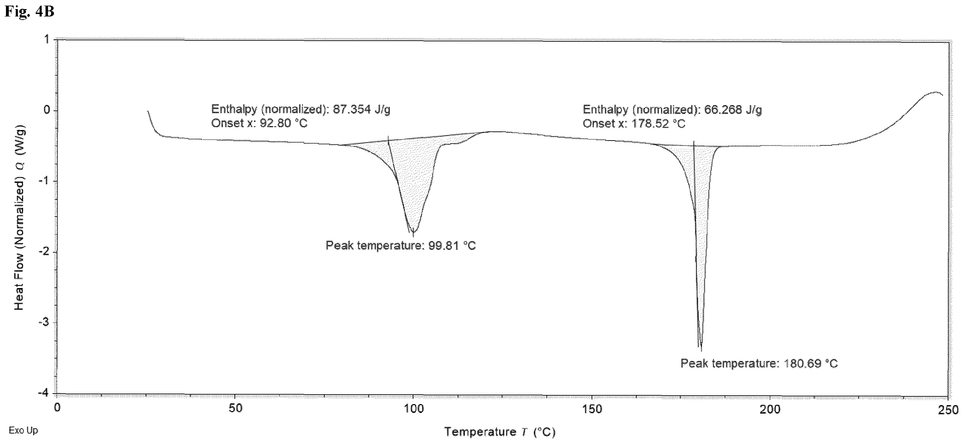

In some embodiments, the phentolamine mesylate exhibits a DSC thermogram that is substantially the same as that depicted in **Fig. 4B****.**

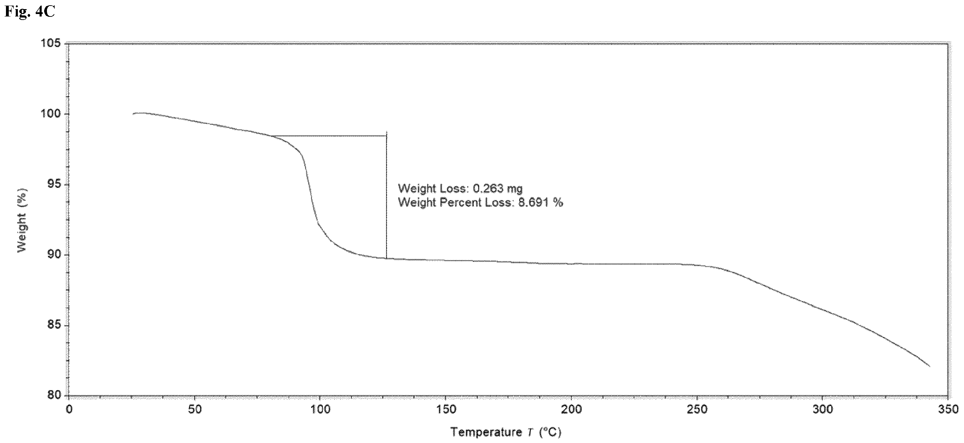

In some embodiments, the phentolamine mesylate exhibits a TG curve that is substantially the same as that depicted in **Fig. 4C****.**

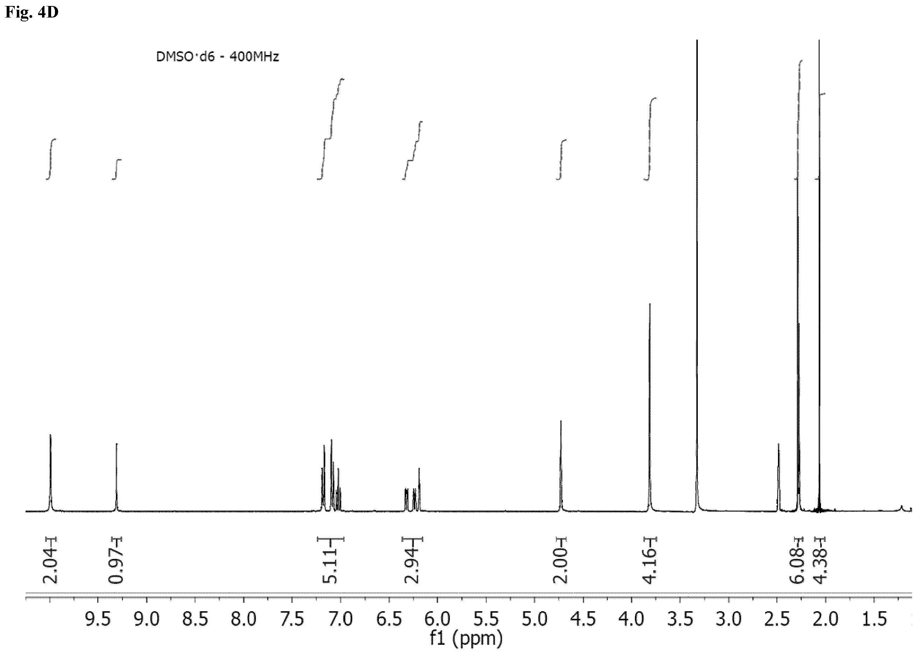

In some embodiments, the phentolamine mesylate exhibits a ¹H-NMR spectrum that is substantially the same as that depicted in **Fig. 4D****.**

In some embodiments, the compound of the invention is phentolamine mesylate obtained according to Example 3.

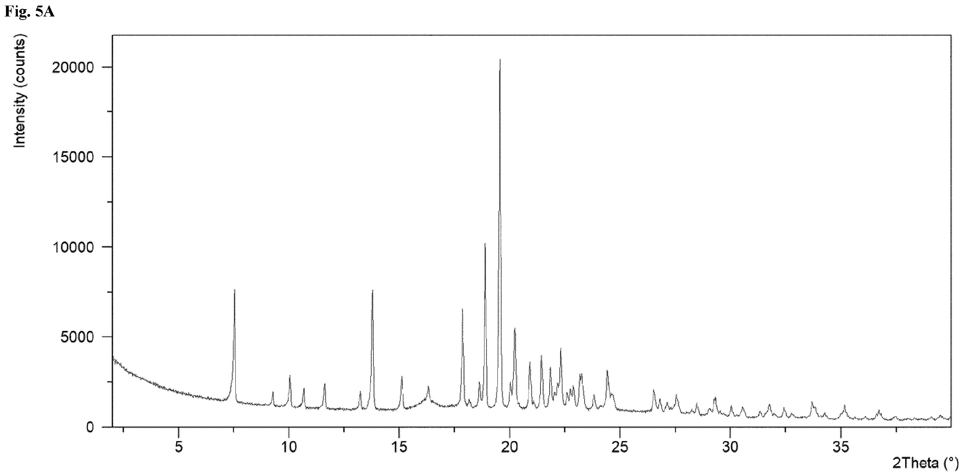

In some embodiments, the compound of the invention is phentolamine mesylate that exhibits an XRPD pattern that is substantially the same as that depicted in **Fig. 5A****.**

In some embodiments, the phentolamine mesylate exhibits a DSC thermogram comprising an endothermic peak having a peak maximum at a temperature of from about 45 °C to about 50 °C. In some embodiments, the phentolamine mesylate exhibits a DSC thermogram comprising an endothermic peak having a peak maximum at a temperature of about 52 °C.

In some embodiments, the phentolamine mesylate exhibits a DSC thermogram comprising an endothermic peak having a peak maximum at a temperature of from about 90 °C to about 105 °C. In some embodiments, the phentolamine mesylate exhibits a DSC thermogram comprising an endothermic peak having a peak maximum at a temperature of about 99°C.

In some embodiments, the phentolamine mesylate exhibits a DSC thermogram comprising an endothermic peak having a peak maximum at a temperature of from about 110 °C to about 120 °C. In some embodiments, the phentolamine mesylate exhibits a DSC thermogram comprising an endothermic peak having a peak maximum at a temperature of about 114 °C.

In some embodiments, the phentolamine mesylate exhibits a DSC thermogram comprising an exothermic peak having a peak maximum at a temperature of from about 125 °C to about 145 °C. In some embodiments, the phentolamine mesylate exhibits a DSC thermogram comprising an exothermic peak having a peak maximum at a temperature of from about 130 °C to about 140 °C. In some embodiments, the phentolamine mesylate exhibits a DSC thermogram comprising an exothermic peak having a peak maximum at a temperature of about 133 °C.

In some embodiments, the phentolamine mesylate exhibits a DSC thermogram comprising an endothermic peak having a peak maximum at a temperature of from about 170 °C to about 190 °C. In some embodiments, the phentolamine mesylate exhibits a DSC thermogram comprising an endothermic peak having a peak maximum at a temperature of from about 175 °C to about 185 °C. In some embodiments, the phentolamine mesylate exhibits a DSC thermogram comprising an endothermic peak having a peak maximum at a temperature of about 180 °C.

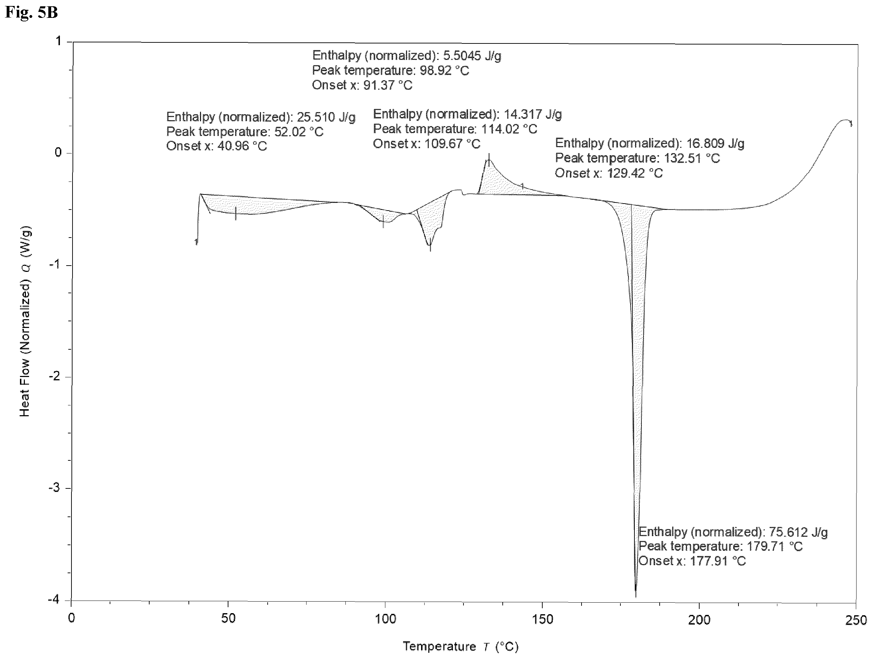

In some embodiments, the phentolamine mesylate exhibits a DSC thermogram that is substantially the same as that depicted in **Fig. 5B****.**

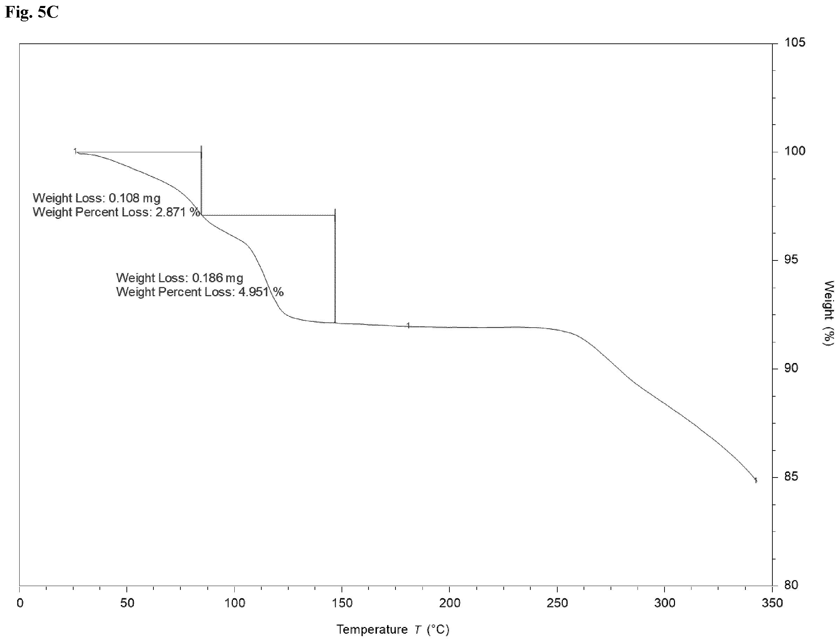

In some embodiments, the phentolamine mesylate exhibits a TG curve that is substantially the same as that depicted in **Fig. 5C****.**

In some embodiments, the compound of the invention is phentolamine mesylate obtained according to Example 4.

In some embodiments, the compound of the invention is phentolamine mesylate that exhibits an XRPD pattern comprising a peak at 7.4 ± 0.2 degrees 2-theta, a peak at 13.1 ± 0.2 degrees 2-theta, a peak at 19.8 ± 0.2 degrees 2-theta, and a peak at 20.9 ± 0.2 degrees 2-theta. In some embodiments, the XRPD pattern further comprises a peak at 9.2 ± 0.2 degrees 2-theta, a peak at 10.8 ± 0.2 degrees 2-theta, a peak at 14.7 ± 0.2 degrees 2-theta, a peak at 15.8 ± 0.2 degrees 2-theta, a peak at 18.4 ± 0.2 degrees 2-theta, a peak at 19.5 ± 0.2 degrees 2-theta, or a peak at 20.7 ± 0.2 degrees 2-theta. In some embodiments, the XRPD pattern further comprises a peak at 9.2 ± 0.2 degrees 2-theta, a peak at 10.8 ± 0.2 degrees 2-theta, a peak at 14.7 ± 0.2 degrees 2-theta, a peak at 15.8 ± 0.2 degrees 2-theta, a peak at 18.4 ± 0.2 degrees 2-theta, a peak at 19.5 ± 0.2 degrees 2-theta, and a peak at 20.7 ± 0.2 degrees 2-theta.

In some embodiments, the compound of the invention is phentolamine mesylate that exhibits an XRPD pattern comprising a peak at 7.4 ± 0.2 degrees 2-theta and 1 other peak, 2 other peaks, 3 other peaks, 4 other peaks, 5 other peaks, 6 other peaks, 7 other peaks, 8 other peaks, or 9 other peaks of **Table 6.** In some embodiments, the phentolamine mesylate exhibits an XRPD pattern comprising a peak at 7.4 ± 0.2 degrees 2-theta and 1 peak, 2 peaks, 3 peaks, 4 peaks, 5 peaks, 6 peaks, 7 peaks, 8 peaks, 9 peaks or 10 peaks of the following peaks: a peak at 9.2 ± 0.2 degrees 2-theta, a peak at 10.8 ± 0.2 degrees 2-theta, a peak at 13.1 ± 0.2 degrees 2-theta, a peak at 14.7 ± 0.2 degrees 2-theta, a peak at 15.8 ± 0.2 degrees 2-theta, a peak at 18.4 ± 0.2 degrees 2-theta, a peak at 19.5 ± 0.2 degrees 2-theta, a peak at 19.8 ± 0.2 degrees 2-theta, a peak at 20.7 ± 0.2 degrees 2-theta, and a peak at 20.9 ± 0.2 degrees 2-theta.

In some embodiments, the compound of the invention is phentolamine mesylate that exhibits an XRPD pattern that is substantially the same as that depicted in **Fig. 6A****.**

In some embodiments, the phentolamine mesylate exhibits a DSC thermogram comprising an endothermic peak having a peak maximum at a temperature of from about 130 °C to about 155 °C. In some embodiments, the phentolamine mesylate exhibits a DSC thermogram comprising an endothermic peak having a peak maximum at a temperature of from about 140 °C to about 150 °C. In some embodiments, the phentolamine mesylate exhibits a DSC thermogram comprising an endothermic peak having a peak maximum at a temperature of about 143 °C.

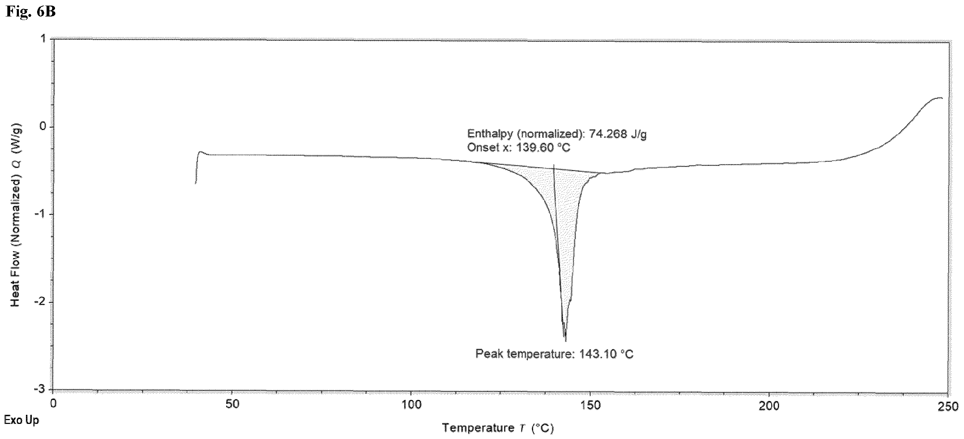

In some embodiments, the phentolamine mesylate exhibits a DSC thermogram that is substantially the same as that depicted in **Fig. 6B****.**

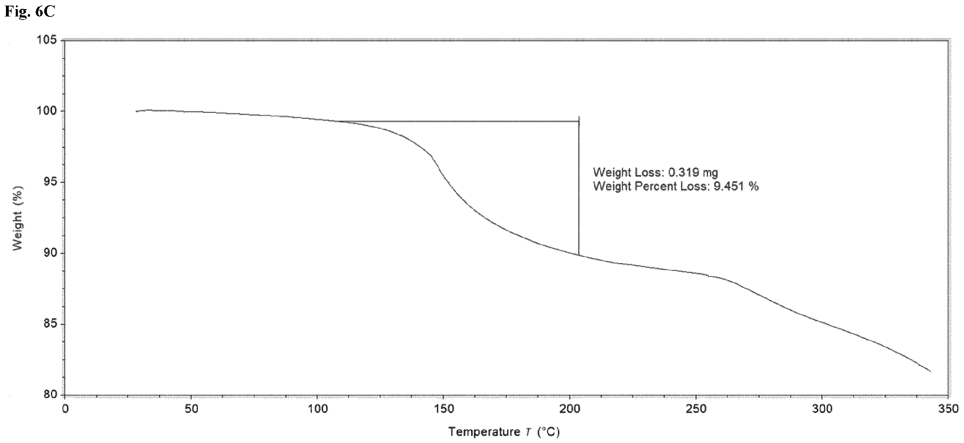

In some embodiments, the phentolamine mesylate exhibits a TG curve that is substantially the same as that depicted in **Fig. 6C****.**

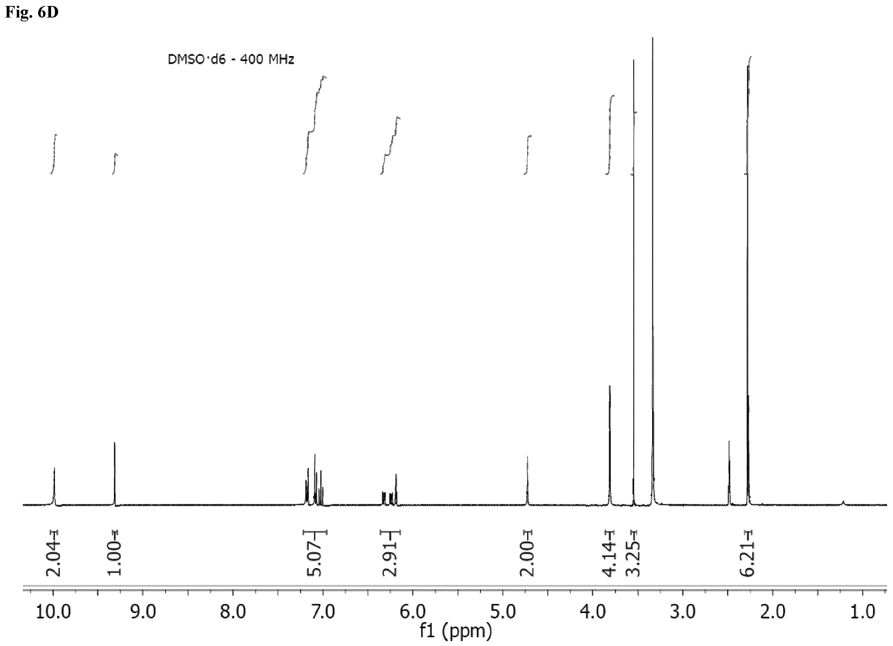

In some embodiments, the phentolamine mesylate exhibits a ¹H-NMR spectrum that is substantially the same as that depicted in **Fig. 6D****.**

In some embodiments, the compound of the invention is phentolamine mesylate obtained according to Example 5.

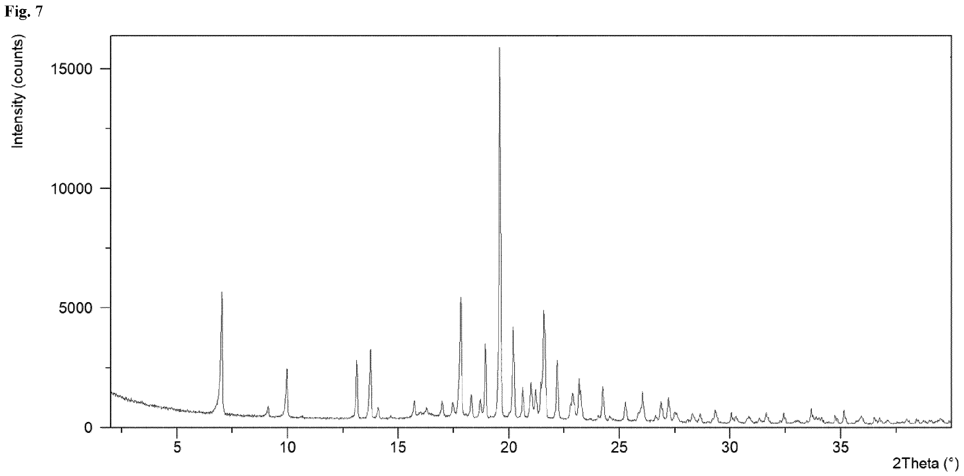

In some embodiments, the compound of the invention is phentolamine mesylate that exhibits an XRPD pattern that is substantially the same as that depicted in **Fig. 7****.**

In some embodiments, the compound of the invention is phentolamine mesylate obtained according to Example 6.

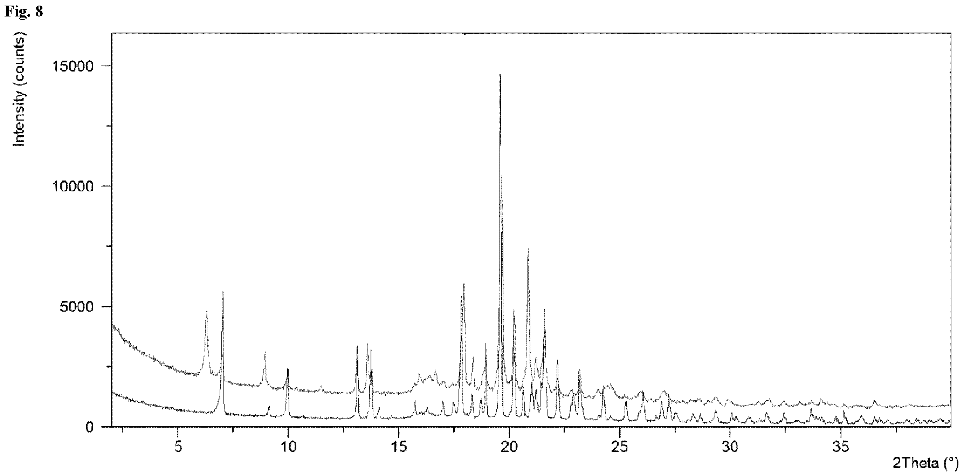

In some embodiments, the compound of the invention is phentolamine mesylate that exhibits an XRPD pattern that is substantially the same as that depicted in **Fig. 8****.**

In some embodiments, the compound of the invention is phentolamine mesylate obtained according to Example 7. In some embodiments, the phentolamine mesylate obtained according to Example 7 comprises the phentolamine mesylate obtained according to Example 6.

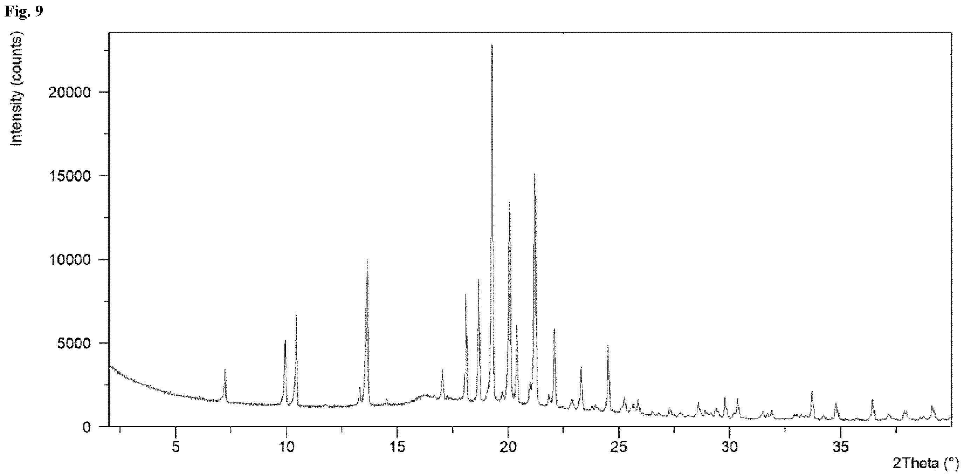

In some embodiments, the compound of the invention is phentolamine mesylate that exhibits an XRPD pattern that is substantially the same as that depicted in **Fig. 9****.**

In some embodiments, the compound of the invention is phentolamine mesylate obtained according to Example 8.

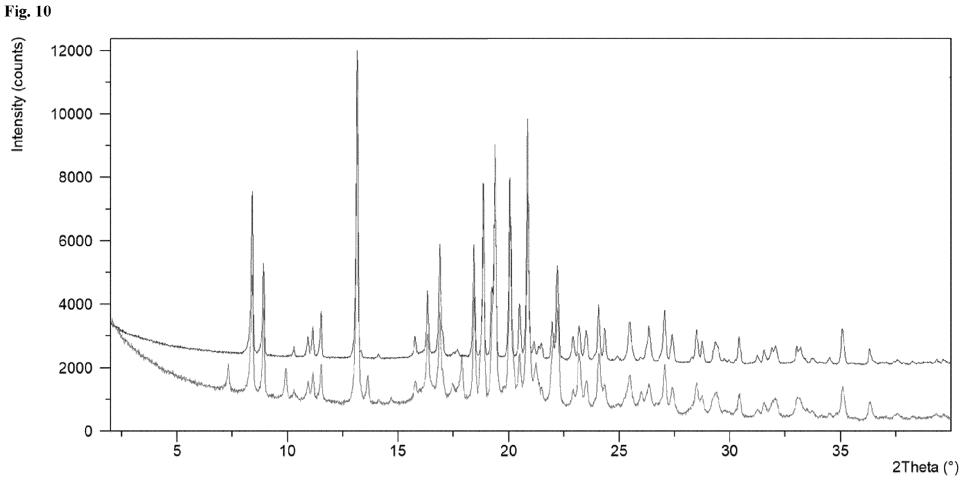

In some embodiments, the compound of the invention is phentolamine mesylate that exhibits an XRPD pattern that is substantially the same as that depicted in **Fig. 10****.**

In some embodiments, the compound of the invention is phentolamine mesylate obtained according to Example 9. In some embodiments, the phentolamine mesylate obtained according to Example 9 comprises the phentolamine mesylate obtained according to Example 1.

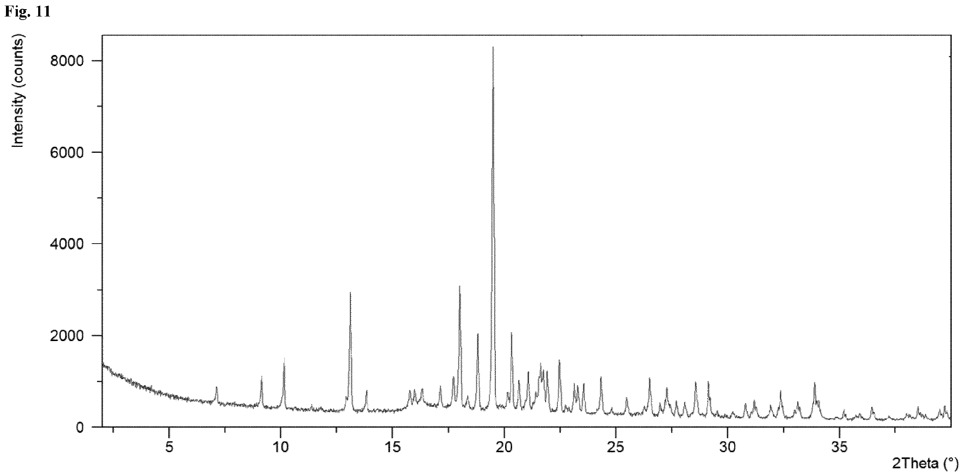

In some embodiments, the compound of the invention is phentolamine mesylate that exhibits an XRPD pattern that is substantially the same as that depicted in **Fig. 11****.**

In some embodiments, the compound of the invention is phentolamine mesylate obtained according to Example 10.

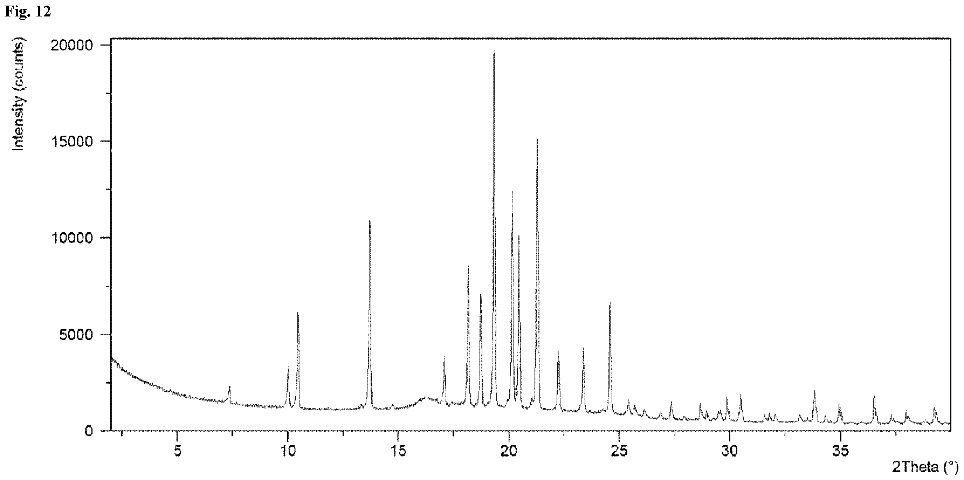

In some embodiments, the compound of the invention is phentolamine mesylate that exhibits an XRPD pattern that is substantially the same as that depicted in **Fig. 12****.**

In some embodiments, the compound of the invention is phentolamine mesylate obtained according to Example 11.

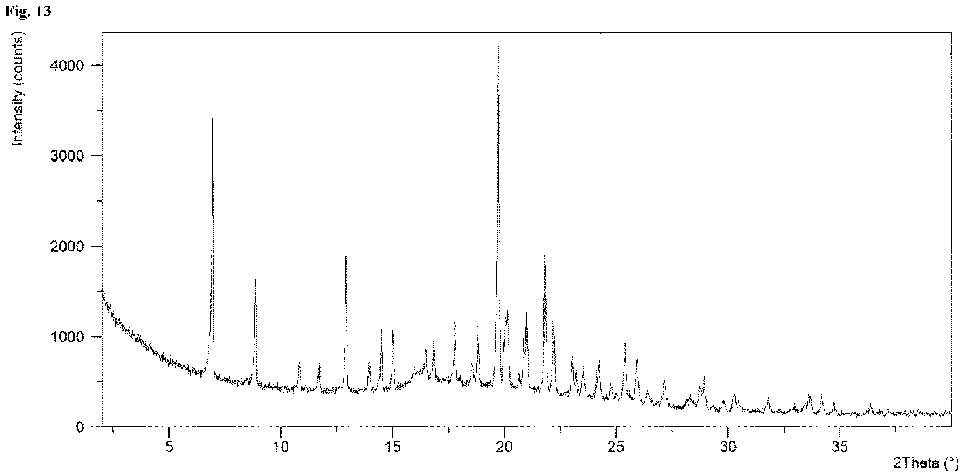

In some embodiments, the compound of the invention is phentolamine mesylate that exhibits an XRPD pattern that is substantially the same as that depicted in **Fig. 13****.**

In some embodiments, the compound of the invention is phentolamine mesylate obtained according to Example 12.

In some embodiments, the phentolamine mesylate is at least about 98% pure by weight, and the phentolamine mesylate comprises no more than about 2% of an impurity by weight of the phentolamine mesylate. In some embodiments, the phentolamine mesylate is about 95.0% to 100% pure by weight, and the phentolamine mesylate comprises 0% to about 5% of an impurity by weight of the phentolamine mesylate. In some embodiments, the phentolamine mesylate is about 98% to 100% pure by weight, and the phentolamine mesylate comprises 0% to about 2% of an impurity by weight of the phentolamine mesylate. In some embodiments, the phentolamine mesylate is about 98%, about 98.5%, about 99%, about 99.5%, or 100% pure by weight, and the phentolamine mesylate comprises about 2%, about 1.5%, about 1%, about 0.5%, or 0%, respectively, of an impurity by weight of the phentolamine mesylate. In some embodiments, the phentolamine mesylate is about 99.5%, about 99.9%, or about 99.95% pure by weight, and the phentolamine mesylate comprises about 0.5%, about 0.1%, or about 0.05%, respectively, of an impurity by weight of the phentolamine mesylate. In some embodiments, the purity or the impurity are determined by high-performance liquid chromatography (HPLC). In some embodiments, the purity or impurity is determined by titration.

In some embodiments, the phentolamine mesylate comprises less than 5% of phentolamine mesylate that exhibits an XRPD pattern that is substantially the same as that depicted in **Fig. 1A** or **Fig. 1C****.** In some embodiments, the phentolamine mesylate comprises less than 3% of phentolamine mesylate that exhibits an XRPD pattern that is substantially the same as that depicted in **Fig. 1A** or **Fig. 1C****.** In some embodiments, the phentolamine mesylate comprises less than 1% of phentolamine mesylate that exhibits an XRPD pattern that is substantially the same as that depicted in **Fig. 1A** or **Fig. 1C****.**

In some embodiments, the phentolamine mesylate comprises less than 5% of phentolamine mesylate that exhibits an XRPD pattern that is substantially the same as that depicted in **Fig. 1A****.** In some embodiments, the phentolamine mesylate comprises less than 3% of phentolamine mesylate that exhibits an XRPD pattern that is substantially the same as that depicted in **Fig. 1A****.** In some embodiments, the phentolamine mesylate comprises less than 1% of phentolamine mesylate that exhibits an XRPD pattern that is substantially the same as that depicted in **Fig. 1A****.**

In some embodiments, the phentolamine mesylate comprises less than 5% of phentolamine mesylate that exhibits an XRPD pattern that is substantially the same as that depicted in **Fig. 1C****.** In some embodiments, the phentolamine mesylate comprises less than 3% of phentolamine mesylate that exhibits an XRPD pattern that is substantially the same as that depicted in **Fig. 1C****.** In some embodiments, the phentolamine mesylate comprises less than 1% of phentolamine mesylate that exhibits an XRPD pattern that is substantially the same as that depicted in **Fig. 1C****.**

In some embodiments, the phentolamine mesylate comprises less than 5% of phentolamine mesylate that exhibits an XRPD pattern substantially the same as that of Reference A. In some embodiments, the phentolamine mesylate comprises less than 3% of phentolamine mesylate that exhibits an XRPD pattern substantially the same as that of Reference A. In some embodiments, the phentolamine mesylate comprises less than 1% of phentolamine mesylate that exhibits an XRPD pattern substantially the same as that of Reference A.

In some embodiments, the phentolamine mesylate comprises less than 5% of phentolamine mesylate that exhibits an XRPD pattern substantially the same as that of Reference B. In some embodiments, the phentolamine mesylate comprises less than 3% of phentolamine mesylate that exhibits an XRPD pattern substantially the same as that of Reference B. In some embodiments, the phentolamine mesylate comprises less than 1% of phentolamine mesylate that exhibits an XRPD pattern substantially the same as that of Reference B.

In some embodiments, the compound of invention is dried.

In some embodiments, the compound of invention is purified.

In some embodiments, the compound of invention is isolated.

In some embodiments, the compound of invention is isolated and purified.

The compounds of the invention are useful in the ocular methods of the invention.

### Synthesis Methods of the Invention

The present invention provides methods of making phentolamine mesylate that exhibits a particular XRPD pattern or exhibits one or more particular XRPD peaks.

The present invention further provides isolating the phentolamine mesylate prepared according to any one of the synthesis methods of the invention.

The present invention provides a method of making phentolamine mesylate according to Example 1. In some embodiments, phentolamine mesylate that exhibits an XRPD pattern comprising a peak at 8.4 ± 0.2 degrees 2-theta and a peak at 16.3 ± 0.2 degrees 2-theta is made according to Example 1. In some embodiments, the method further comprises isolating the phentolamine mesylate that exhibits an XRPD pattern comprising a peak at 8.4 ± 0.2 degrees 2-theta and a peak at 16.3 ± 0.2 degrees 2-theta.

In some embodiments, a method for making phentolamine mesylate that exhibits an XRPD pattern comprising a peak at 8.4 ± 0.2 degrees 2-theta and a peak at 16.3 ± 0.2 degrees 2-theta comprises: (a) heating a mixture comprising phentolamine mesylate comprising an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta; methane sulfonic acid; acetone; and water at about 45 °C for about thirty minutes to provide a heated mixture; (b) allowing the heated mixture to cool to room temperature to provide a cooled mixture; (c) diluting the cooled mixture with t-butyl methyl ether to provide a t-butyl methyl ether mixture; and (d) cooling the t-butyl methyl ether mixture to about -20 °C to precipitate the phentolamine mesylate that exhibits an XRPD pattern comprising a peak at 8.4 ± 0.2 degrees 2-theta and a peak at 16.3 ± 0.2 degrees 2-theta. In some embodiments, the method further comprises (e) isolating the phentolamine mesylate that exhibits an XRPD pattern comprising a peak at 8.4 ± 0.2 degrees 2-theta and a peak at 16.3 ± 0.2 degrees 2-theta.

In some embodiments, a method for making phentolamine mesylate that exhibits an XRPD pattern comprising a peak at 8.4 ± 0.2 degrees 2-theta and a peak at 16.3 ± 0.2 degrees 2-theta comprises: (a) heating a mixture comprising phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta and dichloromethane at about 50 °C for about two hours to provide a heated mixture; (b) allowing the heated mixture to cool at about 25 °C for about two hours to provide cooled phentolamine mesylate; and (c) repeating steps (a) and (b) for up to about three days to provide the phentolamine mesylate that exhibits an XRPD pattern comprising a peak at 8.4 ± 0.2 degrees 2-theta and a peak at 16.3 ± 0.2 degrees 2-theta. In some embodiments, the method further comprises (d) isolating the phentolamine mesylate that exhibits an XRPD pattern comprising a peak at 8.4 ± 0.2 degrees 2-theta and a peak at 16.3 ± 0.2 degrees 2-theta. In some embodiments, the heating in step (a) is at 1 °C/min. In some embodiments, the cooling in step (b) is at 1 °C/min.

In some embodiments, a method for making phentolamine mesylate that exhibits an XRPD pattern comprising a peak at 8.4 ± 0.2 degrees 2-theta and a peak at 16.3 ± 0.2 degrees 2-theta comprises: (a) dissolving phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta in a mixture comprising methyl ethyl ketone and water at about 80 °C to provide a solution; (b) cooling the solution to about 45 °C and adding seeds of phentolamine mesylate that exhibits an XRPD pattern comprising a peak at 8.4 ± 0.2 degrees 2-theta and a peak at 16.3 ± 0.2 degrees 2-theta to provide a seeded mixture; (c) cooling the seeded mixture at 10 °C for about thirty minutes to provide a cooled mixture; (d) heating the cooled mixture at 35°C under vacuum to reduce the volume of the mixture by about 33% and provide a reduced mixture; and (e) cooling the reduced mixture at 10 °C for about forty minutes to provide the phentolamine mesylate that exhibits an XRPD pattern comprising a peak at 8.4 ± 0.2 degrees 2-theta and a peak at 16.3 ± 0.2 degrees 2-theta. In some embodiments, the method further comprises (f) isolating the phentolamine mesylate that exhibits an XRPD pattern comprising a peak at 8.4 ± 0.2 degrees 2-theta and a peak at 16.3 ± 0.2 degrees 2-theta. In some embodiments, the mixture of methyl ethyl ketone and water in step (a) is 96:4 w/w. In some embodiments, the cooling in step (b) is at 0.5 °C/min.

In some embodiments, a method for making phentolamine mesylate that exhibits an XRPD pattern comprising a peak at 8.4 ± 0.2 degrees 2-theta and a peak at 16.3 ± 0.2 degrees 2-theta comprises: (a) heating a mixture comprising phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta, methyl ethyl ketone, and water to about 77 °C to provide a heated mixture; (b) cooling the heated mixture to 10 °C to provide a cooled mixture; and (c) repeating steps (a) and (b) twice to provide the phentolamine mesylate that exhibits an XRPD pattern comprising a peak at 8.4 ± 0.2 degrees 2-theta and a peak at 16.3 ± 0.2 degrees 2-theta. In some embodiments, the method further comprises (d) isolating the phentolamine mesylate that exhibits an XRPD pattern comprising a peak at 8.4 ± 0.2 degrees 2-theta and a peak at 16.3 ± 0.2 degrees 2-theta. In some embodiments, the ratio of methyl ethyl ketone and water in step (a) is 96:4 w/w. In some embodiments, the heating in step (a) is at 0.5 °C/min. In some embodiments, the cooling in step (b) is at 0.5 °C/min.

The present invention provides a method of making phentolamine mesylate according to this Example 2. In some embodiments, phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is made according to this Example 2. In some embodiments, the method further comprises isolating the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta.

In some embodiments, a method for making phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta comprises: (a) heating phentolamine mesylate that exhibits an XRPD pattern comprising a peak at 8.4 ± 0.2 degrees 2-theta and a peak at 16.3 ± 0.2 degrees 2-theta to about 126 °C to provide heated phentolamine mesylate; and (b) allowing the heated phentolamine mesylate to cool to room temperature to provide the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta. In some embodiments, the method further comprises (c) isolating the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta. In some embodiments, the heating in step (a) is at a rate of about 10 °C/min.

The present invention provides a method of making phentolamine mesylate according to Example 3. In some embodiments, phentolamine mesylate that exhibits an XRPD pattern that is substantially the same as that depicted in **Fig. 4A** is made according to Example 3. In some embodiments, the method further comprises isolating the phentolamine mesylate that exhibits an XRPD pattern that is substantially the same as that depicted in **Fig. 4A****.**

In some embodiments, a method for making phentolamine mesylate that exhibits an XRPD pattern that is substantially the same as that depicted in **Fig. 4A****,** comprises: (a) heating a mixture comprising phentolamine mesylate that exhibits an XRPD pattern that is substantially the same as that depicted in **Fig. 1A** or **Fig. 1C****,** and acetone at about 50 °C for about two hours to provide a heated mixture; (b) allowing the heated mixture to cool at about 25 °C for about two hours to provide cooled phentolamine mesylate; and (c) repeating steps (a) and (b) for up to about three days to provide the phentolamine mesylate that exhibits an XRPD pattern that is substantially the same as that depicted in **Fig. 4A****.** In some embodiments, the method further comprises (c) isolating the phentolamine mesylate that exhibits an XRPD pattern that is substantially the same as that depicted in **Fig. 4A****.** In some embodiments, the heating in step (a) is at a rate of about 1 °C/min. In some embodiments, the cooling in step (b) is at a rate of about 1 °C/min. In some embodiments, the phentolamine mesylate in step (a) exhibits an XRPD pattern that is substantially the same as that depicted in **Fig. 1A****.** In some embodiments, the phentolamine mesylate in step (a) exhibits an XRPD pattern that is substantially the same as that depicted in **Fig. 1C****.**

The present invention provides a method of making phentolamine mesylate according to Example 4. In some embodiments, phentolamine mesylate that exhibits an XRPD pattern that is substantially the same as that depicted in **Fig. 5A** is made according to Example 4. In some embodiments, the method further comprises isolating the phentolamine mesylate that exhibits an XRPD pattern that is substantially the same as that depicted in **Fig. 5A****.**

The present invention provides a method of making phentolamine mesylate according to Example 5. In some embodiments, phentolamine mesylate that exhibits an XRPD pattern that is substantially the same as that depicted in **Fig. 6A** is made according to Example 5. In some embodiments, the method further comprises isolating the phentolamine mesylate that exhibits an XRPD pattern that is substantially the same as that depicted in **Fig. 6A****.**

In some embodiments, a method for making phentolamine mesylate that exhibits an XRPD pattern that is substantially the same as that depicted in **Fig. 6A** comprises: (a) pulverizing phentolamine mesylate that exhibits an XRPD pattern that is substantially the same as that depicted in **Fig. 1A** or **Fig. 1C** in the presence of an excess of 1,4-dioxane solvent to provide a pulverized mixture; and (b) removing the 1,4-dioxane solvent from the pulverized mixture to provide the phentolamine mesylate that exhibits an XRPD pattern that is substantially the same as that depicted in **Fig. 6A****..** In some embodiments, the method further comprises (c) isolating the phentolamine mesylate that exhibits an XRPD pattern that is substantially the same as that depicted in **Fig. 6A****.** In some embodiments, the pulverizing in step (a) is with stainless steel balls. In some embodiments, the pulverizing in step (a) is at about 400 rpm. In some embodiments, the phentolamine mesylate in step (a) exhibits an XRPD pattern that is substantially the same as that depicted in **Fig. 1A****.** In some embodiments, the phentolamine mesylate in step (a) exhibits an XRPD pattern that is substantially the same as that depicted in **Fig. 1C****.**

The present invention provides a method of making phentolamine mesylate according to Example 6. In some embodiments, phentolamine mesylate that exhibits an XRPD pattern that is substantially the same as that depicted in **Fig. 7** is made according to Example 6. In some embodiments, the method further comprises isolating the phentolamine mesylate that exhibits an XRPD pattern that is substantially the same as that depicted in **Fig. 7****.**

The present invention provides a method of making phentolamine mesylate according to Example 7. In some embodiments, phentolamine mesylate that exhibits an XRPD pattern that is substantially the same as that depicted in **Fig. 8** is made according to Example 7. In some embodiments, the method further comprises isolating the phentolamine mesylate that exhibits an XRPD pattern that is substantially the same as that depicted in **Fig. 8****.**

The present invention provides a method of making phentolamine mesylate according to Example 8. In some embodiments, phentolamine mesylate that exhibits an XRPD pattern that is substantially the same as that depicted in **Fig. 9** is made according to Example 8. In some embodiments, the method further comprises isolating the phentolamine mesylate that exhibits an XRPD pattern that is substantially the same as that depicted in **Fig. 9****.**

The present invention provides a method of making phentolamine mesylate according to Example 9. In some embodiments, phentolamine mesylate that exhibits an XRPD pattern that is substantially the same as that depicted in **Fig. 10** is made according to Example 9. In some embodiments, the method further comprises isolating the phentolamine mesylate that exhibits an XRPD pattern that is substantially the same as that depicted in **Fig. 10****.**

The present invention provides a method of making phentolamine mesylate according to Example 10. In some embodiments, phentolamine mesylate that exhibits an XRPD pattern that is substantially the same as that depicted in **Fig. 11** is made according to Example 10. In some embodiments, the method further comprises isolating the phentolamine mesylate that exhibits an XRPD pattern that is substantially the same as that depicted in **Fig. 11****.**

The present invention provides a method of making phentolamine mesylate according to Example 11. In some embodiments, phentolamine mesylate that exhibits an XRPD pattern that is substantially the same as that depicted in **Fig. 12** is made according to Example 11. In some embodiments, the method further comprises isolating the phentolamine mesylate that exhibits an XRPD pattern that is substantially the same as that depicted in **Fig. 12****.**

The present invention provides a method of making phentolamine mesylate according to Example 12. In some embodiments, phentolamine mesylate that exhibits an XRPD pattern that is substantially the same as that depicted in **Fig. 13** is made according to Example 12. In some embodiments, the method further comprises isolating the phentolamine mesylate that exhibits an XRPD pattern that is substantially the same as that depicted in **Fig. 13****.**

### Other Compounds Useful in the Ocular Methods of the Invention

In some embodiments, a compound useful in the ocular methods of the invention is Reference A. In some embodiments, a compound useful in the ocular methods of the invention exhibits an XRPD pattern substantially the same as that of Reference A.

In some embodiments, a compound useful in the ocular methods of the invention is Reference B. In some embodiments, a compound useful in the ocular methods of the invention exhibits an XRPD pattern substantially the same as that of Reference B.

In some embodiments, a compound useful in the ocular methods of the invention is phentolamine mesylate that exhibits an XRPD pattern that is substantially the same as that depicted in **Fig. 1A** or **Fig. 1C****.** In some embodiments, a compound useful in the ocular methods of the invention is phentolamine mesylate that exhibits an XRPD pattern that is substantially the same as that depicted in **Fig. 1A****.** In some embodiments, a compound useful in the ocular methods of the invention is phentolamine mesylate that exhibits an XRPD pattern that is substantially the same as that depicted in **Fig. 1C****.**

In some embodiments, a compound useful in the ocular methods of the invention is phentolamine mesylate that exhibits an XRPD pattern comprising a peak at 20.3 ± 0.2 degrees 2-theta and a peak at 21.1 ± 0.2 degrees 2-theta, wherein the peak at 20.3 ± 0.2 degrees 2-theta has a relative intensity of greater than 80%. In some embodiments, the XRPD pattern further comprises a peak at 6.9 ± 0.2 degrees 2-theta, a peak at 11.6 ± 0.2 degrees 2-theta, or a peak at 15.6 ± 0.2 degrees 2-theta.

In some embodiments, a compound useful in the ocular methods of the invention is phentolamine mesylate that exhibits an XRPD pattern comprising a peak at 6.9 ± 0.2 degrees 2-theta, a peak at 11.6 ± 0.2 degrees 2-theta, a peak at 15.4 ± 0.2 degrees 2-theta, a peak at 20.3 ± 0.2 degrees 2-theta, and a peak at 21.7 ± 0.2 degrees 2-theta. %. In some embodiments, the XRPD pattern further comprises a peak at 8.5 ± 0.2 degrees 2-theta, a peak at 10.1 ± 0.2 degrees 2-theta, a peak at 18.5 ± 0.2 degrees 2-theta, a peak at 21.3 ± 0.2 degrees 2-theta, a peak at 23.8 ± 0.2 degrees 2-theta, or a peak at 23.9 ± 0.2 degrees 2-theta. In some embodiments, the XRPD pattern further comprises a peak at 8.5 ± 0.2 degrees 2-theta, a peak at 10.1 ± 0.2 degrees 2-theta, a peak at 18.5 ± 0.2 degrees 2-theta, a peak at 21.3 ± 0.2 degrees 2-theta, a peak at 23.8 ± 0.2 degrees 2-theta, and a peak at 23.9 ± 0.2 degrees 2-theta.

In some embodiments, a compound useful in the ocular methods of the invention is phentolamine mesylate that exhibits an XRPD pattern that is substantially the same as that depicted in **Fig. 1A****.**

In some embodiments, a compound useful in the ocular methods of the invention is phentolamine mesylate that exhibits an XRPD pattern comprising the peaks of **Table 1** having at least 30% relative intensity. In some embodiments, a compound useful in the ocular methods of the invention is phentolamine mesylate that exhibits an XRPD pattern comprising the peaks of Table 1 having at least 20% relative intensity. In some embodiments, a compound useful in the ocular methods of the invention is phentolamine mesylate that exhibits an XRPD pattern comprising the peaks of Table 1 having at least 15% relative intensity. In some embodiments, a compound useful in the ocular methods of the invention is phentolamine mesylate that exhibits an XRPD pattern comprising the peaks of **Table 1** having at least 10% relative intensity.

In some embodiments, a compound useful in the ocular methods of the invention is phentolamine mesylate that exhibits an XRPD pattern that is substantially the same as that depicted in **Fig. 1C****.**

In some embodiments, a compound useful in the ocular methods of the invention is phentolamine mesylate that exhibits an XRPD pattern comprising the peaks of **Table 2** having at least 30% relative intensity. In some embodiments, a compound useful in the ocular methods of the invention is phentolamine mesylate that exhibits an XRPD pattern comprising the peaks of Table 2 having at least 20% relative intensity. In some embodiments, a compound useful in the ocular methods of the invention is phentolamine mesylate that exhibits an XRPD pattern comprising the peaks of **Table 2** having at least 15% relative intensity. In some embodiments, a compound useful in the ocular methods of the invention is phentolamine mesylate that exhibits an XRPD pattern comprising the peaks of **Table 2** having at least 10% relative intensity.

In some embodiments, a compound useful in the ocular methods of the invention is phentolamine mesylate that exhibits an XRPD pattern comprising a peak at 20.3 ± 0.3 degrees 2-theta, wherein the peak at 20.3 ± 0.3 degrees 2-theta has a relative peak intensity (%) of greater than 85%. In some embodiments, the phentolamine mesylate exhibits an XRPD pattern comprising a peak at 20.3 ± 0.3 degrees 2-theta, wherein the peak at 20.3 ± 0.3 degrees 2-theta has a relative peak intensity (%) of greater than 90%. In some embodiments, the phentolamine mesylate exhibits an XRPD pattern comprising a peak at 20.3 ± 0.3 degrees 2-theta, wherein the peak at 20.3 ± 0.3 degrees 2-theta is the XRPD pattern's most intense peak.

In some embodiments, a compound useful in the ocular methods of the invention is phentolamine mesylate that exhibits an XRPD pattern comprising a peak at 20.3 ± 0.2 degrees 2-theta, wherein the peak at 20.3 ± 0.2 degrees 2-theta has a relative peak intensity (%) of greater than 85%. In some embodiments, the phentolamine mesylate exhibits an XRPD pattern comprising a peak at 20.3 ± 0.2 degrees 2-theta, wherein the peak at 20.3 ± 0.2 degrees 2-theta has a relative peak intensity (%) of greater than 90%. In some embodiments, the phentolamine mesylate exhibits an XRPD pattern comprising a peak at 20.3 ± 0.2 degrees 2-theta, wherein the peak at 20.3 ± 0.2 degrees 2-theta is the XRPD pattern's most intense peak.

In some embodiments, a compound useful in the ocular methods of the invention is phentolamine mesylate that exhibits an XRPD pattern comprising a peak at 21.1 ± 0.2 degrees 2-theta, wherein the peak at 21.1 ± 0.2 degrees 2-theta has a relative peak intensity (%) of greater than 30%.

In some embodiments, a compound useful in the ocular methods of the invention is phentolamine mesylate that exhibits an XRPD pattern comprising a peak at 11.6 ± 0.2 degrees 2-theta, wherein the peak at 11.6 ± 0.2 degrees 2-theta has a relative peak intensity (%) of greater than 30%.

In some embodiments, a compound useful in the ocular methods of the invention is phentolamine mesylate that exhibits an XRPD pattern comprising a peak at 20.3 ± 0.2 degrees 2-theta, a peak at 21.1 ± 0.2 degrees 2-theta, and a peak at 21.1 ± 0.2 degrees 2-theta, wherein the peak at 20.3 ± 0.2 degrees 2-theta has a relative peak intensity (%) of greater than 80% and the peak at 21.1 ± 0.2 degrees 2-theta and the peak at 21.1 ± 0.2 degrees 2-theta each has a relative peak intensity (%) of greater than 30%.

### Ocular Methods of the Invention

In some embodiments, the compound of the invention is useful for treating or reversing pharmacologically induced mydriasis. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

Accordingly, the invention further provides methods for treating or reversing pharmacologically induced mydriasis, comprising administering to a subject in need thereof an effective amount of a compound of the invention or a composition of the invention. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis produced by an adrenergic agonist, comprising administering to a subject in need thereof an effective amount of phentolamine mesylate that exhibits an XRPD pattern that is substantially the same as that depicted in **Fig. 1A** or **Fig. 1C** or a composition comprising (i) phentolamine mesylate that exhibits an XRPD pattern that is substantially the same as that depicted in **Fig. 1A** or **Fig. 1C** and (ii) a pharmaceutically acceptable carrier or vehicle. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the adrenergic agonist is phenylephrine. In some embodiments, the administering is topically instilling into one or both eyes of the subject.

The invention further provides methods for treating or reversing pharmacologically induced mydriasis produced by a parasympatholytic agent, comprising administering to a subject in need thereof an effective amount of phentolamine mesylate that exhibits an XRPD pattern that is substantially the same as that depicted in **Fig. 1A** or **Fig. 1C** or a composition comprising (i) phentolamine that exhibits an XRPD pattern that is substantially the same as that depicted in **Fig. 1A** or **Fig. 1C** and (ii) a pharmaceutically acceptable carrier or vehicle. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of phentolamine mesylate that exhibits an XRPD pattern comprising a peak at 8.4 ± 0.2 degrees 2-theta and a peak at 16.3 ± 0.2 degrees 2-theta. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of phentolamine mesylate that exhibits an XRPD pattern that is substantially the same as that depicted in **Fig. 2A****.** In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of phentolamine mesylate that exhibits an XRPD pattern comprising a **Table 3** peak having a relative peak intensity (%) of greater than 30%. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of phentolamine mesylate that exhibits an XRPD pattern that is substantially the same as that depicted in **Fig. 3A****.** In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of phentolamine mesylate that exhibits an XRPD pattern comprising a **Table 4** peak having a relative peak intensity (%) of greater than 30%. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of a composition comprising (i) phentolamine mesylate that exhibits an XRPD pattern comprising a peak at 8.4 ± 0.2 degrees 2-theta and a peak at 16.3 ± 0.2 degrees 2-theta and (ii) a pharmaceutically acceptable carrier or vehicle. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of a composition comprising (i) phentolamine mesylate that exhibits an XRPD pattern that is substantially the same as that depicted in **Fig. 2A** and (ii) a pharmaceutically acceptable carrier or vehicle. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of a composition comprising (i) phentolamine mesylate that exhibits an XRPD pattern comprising a **Table 3** peak having a relative peak intensity (%) of greater than 30% and (ii) a pharmaceutically acceptable carrier or vehicle. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of a composition comprising (i) phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta and (ii) a pharmaceutically acceptable carrier or vehicle. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject. In some embodiments, the administering is topically instilling into one or both eyes of the subj ect.

The invention further provides methods for treating or reversing pharmacologically induced mydriasis, comprising administering to a subject in need thereof an effective amount of a composition comprising (i) phentolamine mesylate that exhibits an XRPD pattern that is substantially the same as that depicted in **Fig. 3A** and (ii) a pharmaceutically acceptable carrier or vehicle. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide.

The invention further provides methods for treating or reversing pharmacologically induced mydriasis, comprising administering to a subject in need thereof an effective amount of a composition comprising (i) phentolamine mesylate that exhibits an XRPD pattern comprising a **Table 4** peak having a relative peak intensity (%) of greater than 30% and (ii) a pharmaceutically acceptable carrier or vehicle. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****.** In some embodiments, the pharmacologically induced mydriasis produced by an adrenergic agonist. In some embodiments, the adrenergic agonist is phenylephrine. In some embodiments, the pharmacologically induced mydriasis produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of a composition comprising (i) phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (ii) a pharmaceutically acceptable carrier or vehicle. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which a compound of the invention is dissolved. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which phentolamine mesylate that exhibits an XRPD pattern comprising a peak at 8.4 ± 0.2 degrees 2-theta and a peak at 16.3 ± 0.2 degrees 2-theta is dissolved. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which phentolamine mesylate that exhibits an XRPD pattern that is substantially the same as that depicted in **Fig. 2A** is dissolved. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which phentolamine mesylate that exhibits an XRPD pattern comprising a **Table 3** peak having a relative peak intensity (%) of greater than 30% is dissolved. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which phentolamine mesylate that exhibits an XRPD pattern that is substantially the same as that depicted in **Fig. 3A** is dissolved. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which phentolamine mesylate that exhibits an XRPD pattern comprising a **Table 4** peak having a relative peak intensity (%) of greater than 30% is dissolved. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis, comprising administering to a subject in need thereof an effective amount of an ophthalmic solution in which phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13** is dissolved. In some embodiments, the pharmacologically induced mydriasis produced by an adrenergic agonist. In some embodiments, the adrenergic agonist is phenylephrine. In some embodiments, the pharmacologically induced mydriasis produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis in a subject in need thereof, comprising (a) dissolving in a pharmaceutically acceptable aqueous carrier or vehicle a compound of the invention to provide an ophthalmic solution in which the compound of the invention is dissolved and (b) administering to the subject the ophthalmic solution. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing in a subject in need thereof pharmacologically induced mydriasis produced by an adrenergic agonist, comprising (a) dissolving in a pharmaceutically acceptable aqueous carrier or vehicle phentolamine mesylate that exhibits an XRPD pattern that is substantially the same as that depicted in **Fig. 1A** or **Fig. 1C** to provide an ophthalmic solution in which the phentolamine mesylate that exhibits an XRPD pattern that is substantially the same as that depicted in **Fig. 1A** or **Fig. 1C** is dissolved and (b) administering to the subject an effective amount of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the adrenergic agonist is phenylephrine. In some embodiments, the administering is topically instilling into one or both eyes of the subject.

The invention further provides methods for treating or reversing in a subject in need thereof pharmacologically induced mydriasis produced by a parasympatholytic agent, comprising (a) dissolving in a pharmaceutically acceptable aqueous carrier or vehicle phentolamine mesylate that exhibits an XRPD pattern that is substantially the same as that depicted in **Fig. 1A** or **Fig. 1C** to provide an ophthalmic solution in which the phentolamine mesylate that exhibits an XRPD pattern that is substantially the same as that depicted in **Fig. 1A** or **Fig. 1C** is dissolved and (b) administering to the subject an effective amount of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the parasympatholytic agent is tropicamide. In some embodiments, the administering is topically instilling into one or both eyes of the subj ect.

The invention further provides methods for treating or reversing pharmacologically induced mydriasis in a subject in need thereof, comprising (a) dissolving in a pharmaceutically acceptable aqueous carrier or vehicle phentolamine mesylate that exhibits an XRPD pattern comprising a peak at 8.4 ± 0.2 degrees 2-theta and a peak at 16.3 ± 0.2 degrees 2-theta to provide an ophthalmic solution in which the phentolamine mesylate that exhibits an XRPD pattern comprising a peak at 8.4 ± 0.2 degrees 2-theta and a peak at 16.3 ± 0.2 degrees 2-theta is dissolved and (b) administering to the subject an effective amount of the ophthalmic solution. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis in a subject in need thereof, comprising (a) dissolving in a pharmaceutically acceptable aqueous carrier or vehicle phentolamine mesylate that exhibits an XRPD pattern that is substantially the same as that depicted in **Fig. 2A** to provide an ophthalmic solution in which the phentolamine mesylate that exhibits an XRPD pattern that is substantially the same as that depicted in **Fig. 2A** is dissolved and (b) administering to the subject an effective amount of the ophthalmic solution. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis in a subject in need thereof, comprising (a) dissolving in a pharmaceutically acceptable aqueous carrier or vehicle phentolamine mesylate that exhibits an XRPD pattern comprising a **Table 3** peak having a relative peak intensity (%) of greater than 30% to provide an ophthalmic solution in which the phentolamine mesylate that exhibits an XRPD pattern comprising a **Table 3** peak having a relative peak intensity (%) of greater than 30% is dissolved and (b) administering to the subject an effective amount of the ophthalmic solution. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis in a subject in need thereof, comprising (a) dissolving in a pharmaceutically acceptable aqueous carrier or vehicle phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta to provide an ophthalmic solution in which the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved and (b) administering to the subject an effective amount of the ophthalmic solution. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis in a subject in need thereof, comprising (a) dissolving in a pharmaceutically acceptable aqueous carrier or vehicle phentolamine mesylate that exhibits an XRPD pattern that is substantially the same as that depicted in **Fig. 3A** to provide an ophthalmic solution in which the phentolamine mesylate that exhibits an XRPD pattern that is substantially the same as that depicted in **Fig. 3A** is dissolved and (b) administering to the subject an effective amount of the ophthalmic solution. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis in a subject in need thereof, comprising (a) dissolving in a pharmaceutically acceptable aqueous carrier or vehicle phentolamine mesylate that exhibits an XRPD pattern comprising a **Table 4** peak having a relative peak intensity (%) of greater than 30% to provide an ophthalmic solution in which the phentolamine mesylate that exhibits an XRPD pattern comprising a **Table 4** peak having a relative peak intensity (%) of greater than 30% is dissolved and (b) administering to the subject an effective amount of the ophthalmic solution. In some embodiments, the pharmacologically induced mydriasis is produced by an adrenergic agonist. In some embodiments, the adrenergic agonist is phenylephrine. In some embodiments, the pharmacologically induced mydriasis is produced by a parasympatholytic agent. In some embodiments, the parasympatholytic agent is tropicamide. In some embodiments, the administering is topically instilling into one or both eyes of the subject.

The invention further provides methods for treating or reversing pharmacologically induced mydriasis in a subject in need thereof, comprising (a) dissolving in a pharmaceutically acceptable aqueous carrier or vehicle phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (b) administering to the subject an effective amount of the ophthalmic solution. In some embodiments, the pharmacologically induced mydriasis produced by an adrenergic agonist. In some embodiments, the adrenergic agonist is phenylephrine. In some embodiments, the pharmacologically induced mydriasis produced by a parasympatholytic agent. In some embodiments, the parasympatholytic agent is tropicamide. In some embodiments, the administering is topically instilling into one or both eyes of the subject.

In some embodiments, adrenergic agonist is an alpha-1 adrenergic agonist. In some embodiments, adrenergic agonist is 6-fluoronorepinephrine, amidephrine, buspirone, cirazoline, corbadrine, deoxyepinephrine, desglymidodrine, dexisometheptene, dipivefrine, dopamine, droxidopa (L-DOPS), epinephrine, etilefrine, etilevodopa, ethylnorepinephrine, ibopamine, indanidine, isometheptene, levodopa, L-phenylalanine, L-tyrosine, melevodopa, metaraminol, methoxamine, methyldopa, midodrine, naphazoline, norepinephrine, octopamine, oxymetazoline, phenylephrine, phenylpropanolamine, synephrine, tetryzoline, tiamenidine, XP21279, or xylometazoline. In some embodiments, the adrenergic agonist is phenylephrine, methoxamine, midodrine, or oxymetazoline. In some embodiments, the adrenergic agonist is phenylephrine.

In some embodiments, the adrenergic agonist is an alpha-2 adrenergic agonist. In some embodiments, the adrenergic agonist is (R)-3-nitrobiphenyline, (S)-naphthylmedetomidine, 6-fluoronorepinephrine, amitraz, apraclonidine, brimonidine, clonidine, corbadrine, deoxyepinephrine, detomidine, dexmedetomidine, dihydroergotamine, dipivefrine, dopamine, droxidopa (L-DOPS), etilevodopa, ergotamine, epinephrine, etilefrine, ethylnorepinephrine, guanabenz, guanfacine, guanoxabenz, ibopamine, levodopa, L-phenylalanine, L-tyrosine, lofexidine, medetomidine, melevodopa, methyldopa, mivazerol, moxonidine, naphazoline, norepinephrine, oxymetazoline, phenylpropanolamine, piperoxan, N-(pyridin-4-yl)-7-chloronaphthalen-1-amine, rezatomidine, rilmenidine, romifidine, talipexole, tasipimidine, tetryzoline, tiamenidine, tizanidine, tolonidine, urapidil, vatinoxan, XP21279, xylazine, or xylometazoline. In some embodiments, the adrenergic agonist is α-methyldopa, clonidine, brimonidine, or dexmedetomidine.

In some embodiments, the adrenergic agonist is a selective agonist. In some embodiments, the adrenergic agonist is a non-selective agonist. In some embodiments, the adrenergic agonist is adrenaline, noradrenaline, or dopamine.

In some embodiments, the parasympatholytic agent is atropine, cyclopentolate, homatropine, scopolamine or tropicamide.

In some embodiments, the compound of the invention is useful for treating or preventing an ocular disease or condition.

Accordingly, the invention further provides methods for treating or preventing an ocular disease or condition, comprising administering to a subject in need thereof an effective amount of a compound of the invention or a composition of the invention. In some embodiments, the administering is topically instilling into one or both eyes of the subject.

The invention further provides methods for treating or preventing an ocular disease or condition, comprising administering to a subject in need thereof an effective amount of phentolamine mesylate that exhibits an XRPD pattern comprising a peak at 8.4 ± 0.2 degrees 2-theta and a peak at 16.3 ± 0.2 degrees 2-theta. In some embodiments, the administering is topically instilling into one or both eyes of the subject.

The invention further provides methods for treating or preventing an ocular disease or condition, comprising administering to a subject in need thereof an effective amount of phentolamine mesylate that exhibits an XRPD pattern that is substantially the same as that depicted in **Fig. 2A****.** In some embodiments, the administering is topically instilling into one or both eyes of the subject.

The invention further provides methods for treating or preventing an ocular disease or condition, comprising administering to a subject in need thereof an effective amount of phentolamine mesylate that exhibits an XRPD pattern comprising a **Table 3** peak having a relative peak intensity (%) of greater than 30%. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for treating or preventing an ocular disease or condition, comprising administering to a subject in need thereof an effective amount of phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta.

The invention further provides methods for treating or preventing an ocular disease or condition, comprising administering to a subject in need thereof an effective amount of phentolamine mesylate that exhibits an XRPD pattern that is substantially the same as that depicted in **Fig. 3A****.** In some embodiments, the administering is topically instilling into one or both eyes of the subject.

The invention further provides methods for treating or preventing an ocular disease or condition, comprising administering to a subject in need thereof an effective amount of phentolamine mesylate that exhibits an XRPD pattern comprising a **Table 4** peak having a relative peak intensity (%) of greater than 30%. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for treating or preventing an ocular disease or condition, comprising administering to a subject in need thereof an effective amount of a composition of the invention. In some embodiments, the administering is topically instilling into one or both eyes of the subject.

The invention further provides methods for treating or preventing an ocular disease or condition, comprising administering to a subject in need thereof an effective amount of a composition comprising (i) phentolamine mesylate that exhibits an XRPD pattern comprising a peak at 8.4 ± 0.2 degrees 2-theta and a peak at 16.3 ± 0.2 degrees 2-theta and (ii) a pharmaceutically acceptable carrier or vehicle. In some embodiments, the administering is topically instilling into one or both eyes of the subject.

The invention further provides methods for treating or preventing an ocular disease or condition, comprising administering to a subject in need thereof an effective amount of a composition comprising (i) phentolamine mesylate that exhibits an XRPD pattern that is substantially the same as that depicted in **Fig. 2A** and (ii) a pharmaceutically acceptable carrier or vehicle. In some embodiments, the administering is topically instilling into one or both eyes of the subject.

The invention further provides methods for treating or preventing an ocular disease or condition, comprising administering to a subject in need thereof an effective amount of a composition comprising (i) phentolamine mesylate that exhibits an XRPD pattern comprising a **Table 3** peak having a relative peak intensity (%) of greater than 30% and (ii) a pharmaceutically acceptable carrier or vehicle. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for treating or preventing an ocular disease or condition, comprising administering to a subject in need thereof an effective amount of a composition comprising (i) phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta and (ii) a pharmaceutically acceptable carrier or vehicle. In some embodiments, the administering is topically instilling into one or both eyes of the subject.

The invention further provides methods for treating or preventing an ocular disease or condition, comprising administering to a subject in need thereof an effective amount of a composition comprising (i) phentolamine mesylate that exhibits an XRPD pattern that is substantially the same as that depicted in **Fig. 3A** and (ii) a pharmaceutically acceptable carrier or vehicle. In some embodiments, the administering is topically instilling into one or both eyes of the subject.

The invention further provides methods for treating or preventing an ocular disease or condition, comprising administering to a subject in need thereof an effective amount of a composition comprising (i) phentolamine mesylate that exhibits an XRPD pattern comprising a **Table 4** peak having a relative peak intensity (%) of greater than 30% and (ii) a pharmaceutically acceptable carrier or vehicle. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for treating or preventing an ocular disease or condition, comprising administering to a subject in need thereof an effective amount of phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****.** In some embodiments, the administering is topically instilling into one or both eyes of the subj ect.

The invention further provides methods for treating or preventing an ocular disease or condition, comprising administering to a subject in need thereof an effective amount of a composition comprising (i) phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (ii) a pharmaceutically acceptable carrier or vehicle.

The invention further provides methods for treating or preventing an ocular disease or condition, comprising administering to a subject in need thereof an effective amount of an ophthalmic solution in which a compound of the invention is dissolved. In some embodiments, the administering is topically instilling into one or both eyes of the subject.

The invention further provides methods for treating or preventing an ocular disease or condition, comprising administering to a subject in need thereof an effective amount of an ophthalmic solution in which phentolamine mesylate that exhibits an XRPD pattern comprising a peak at 8.4 ± 0.2 degrees 2-theta and a peak at 16.3 ± 0.2 degrees 2-theta is dissolved. In some embodiments, the administering is topically instilling into one or both eyes of the subject.

The invention further provides methods for treating or preventing an ocular disease or condition, comprising administering to a subject in need thereof an effective amount of an ophthalmic solution in which phentolamine mesylate that exhibits an XRPD pattern that is substantially the same as that depicted in **Fig. 2A** is dissolved. In some embodiments, the administering is topically instilling into one or both eyes of the subject.

The invention further provides methods for treating or preventing an ocular disease or condition, comprising administering to a subject in need thereof an effective amount of an ophthalmic solution in which phentolamine mesylate that exhibits an XRPD pattern comprising a **Table 3** peak having a relative peak intensity (%) of greater than 30% is dissolved. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for treating or preventing an ocular disease or condition, comprising administering to a subject in need thereof an effective amount of an ophthalmic solution in which phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved. In some embodiments, the administering is topically instilling into one or both eyes of the subject.

The invention further provides methods for treating or preventing an ocular disease or condition, comprising administering to a subject in need thereof an effective amount of an ophthalmic solution in which phentolamine mesylate that exhibits an XRPD pattern that is substantially the same as that depicted in **Fig. 3A** is dissolved. In some embodiments, the administering is topically instilling into one or both eyes of the subject.

The invention further provides methods for treating or preventing an ocular disease or condition, comprising administering to a subject in need thereof an effective amount of an ophthalmic solution in which phentolamine mesylate that exhibits an XRPD pattern comprising a **Table 4** peak having a relative peak intensity (%) of greater than 30% is dissolved. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for treating or preventing an ocular disease or condition, comprising administering to a subject in need thereof an effective amount of an ophthalmic solution in which phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13** is dissolved. In some embodiments, the administering is topically instilling into one or both eyes of the subject.

The invention further provides methods for treating or preventing an ocular disease or condition in a subject in need thereof, comprising (a) dissolving in a pharmaceutically acceptable aqueous carrier or vehicle a compound of the invention to provide an ophthalmic solution in which the compound of the invention is dissolved and (b) administering to the subject the ophthalmic solution. In some embodiments, the administering is topically instilling into one or both eyes of the subject.

The invention further provides methods for treating or preventing an ocular disease or condition in a subject in need thereof, comprising (a) dissolving in a pharmaceutically acceptable aqueous carrier or vehicle phentolamine mesylate that exhibits an XRPD pattern comprising a peak at 8.4 ± 0.2 degrees 2-theta and a peak at 16.3 ± 0.2 degrees 2-theta to provide an ophthalmic solution in which the phentolamine mesylate that exhibits an XRPD pattern comprising a peak at 8.4 ± 0.2 degrees 2-theta and a peak at 16.3 ± 0.2 degrees 2-theta is dissolved and (b) administering to the subject an effective amount of the ophthalmic solution. In some embodiments, the administering is topically instilling into one or both eyes of the subject.

The invention further provides methods for treating or preventing an ocular disease or condition in a subject in need thereof, comprising (a) dissolving in a pharmaceutically acceptable aqueous carrier or vehicle phentolamine mesylate that exhibits an XRPD pattern that is substantially the same as that depicted in **Fig. 2A** to provide an ophthalmic solution in which the phentolamine mesylate that exhibits an XRPD pattern that is substantially the same as that depicted in **Fig. 2A** is dissolved and (b) administering to the subject an effective amount of the ophthalmic solution. In some embodiments, the administering is topically instilling into one or both eyes of the subject.

The invention further provides methods for treating or preventing an ocular disease or condition in a subject in need thereof, comprising (a) dissolving in a pharmaceutically acceptable aqueous carrier or vehicle phentolamine mesylate that exhibits an XRPD pattern comprising a **Table 3** peak having a relative peak intensity (%) of greater than 30% to provide an ophthalmic solution in which the phentolamine mesylate that exhibits an XRPD pattern comprising a **Table 3** peak having a relative peak intensity (%) of greater than 30% is dissolved and (b) administering to the subject an effective amount of the ophthalmic solution. In some embodiments, the administering is topically instilling into one or both eyes of the subj ect.

The invention further provides for treating or preventing an ocular disease or condition in a subject in need thereof, comprising (a) dissolving in a pharmaceutically acceptable aqueous carrier or vehicle phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta to provide an ophthalmic solution in which the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved and (b) administering to the subject an effective amount of the ophthalmic solution. In some embodiments, the administering is topically instilling into one or both eyes of the subj ect.

The invention further provides methods for treating or preventing an ocular disease or condition in a subject in need thereof, comprising (a) dissolving in a pharmaceutically acceptable aqueous carrier or vehicle phentolamine mesylate that exhibits an XRPD pattern that is substantially the same as that depicted in **Fig. 3A** to provide an ophthalmic solution in which the phentolamine mesylate that exhibits an XRPD pattern that is substantially the same as that depicted in **Fig. 3A** is dissolved and (b) administering to the subject an effective amount of the ophthalmic solution. In some embodiments, the administering is topically instilling into one or both eyes of the subject.

The invention further provides methods for treating or preventing an ocular disease or condition in a subject in need thereof, comprising (a) dissolving in a pharmaceutically acceptable aqueous carrier or vehicle phentolamine mesylate that exhibits an XRPD pattern comprising a **Table 4** peak having a relative peak intensity (%) of greater than 30% to provide an ophthalmic solution in which the phentolamine mesylate that exhibits an XRPD pattern comprising a **Table 4** peak having a relative peak intensity (%) of greater than 30% is dissolved and (b) administering to the subject an effective amount of the ophthalmic solution. In some embodiments, the administering is topically instilling into one or both eyes of the subj ect.

The invention further provides methods for treating or preventing an ocular disease or condition in a subject in need thereof, comprising (a) dissolving in a pharmaceutically acceptable aqueous carrier or vehicle phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (b) administering to the subject an effective amount of the ophthalmic solution. In some embodiments, the administering is topically instilling into one or both eyes of the subject.

In some embodiments, the ocular disease or condition is a dim or night vision disturbance.

In some embodiments, the ocular disease or condition is presbyopia.

In some embodiments, the ocular disease or condition is diabetic retinal disease. In some embodiments, diabetic retinal disease is diabetic retinopathy (DR) or diabetic macular edema (DME). In some embodiments, the DR is moderately severe non-proliferative DR or mild proliferative DR. In some embodiments, the DME is DME without loss of central vision.

In some embodiments, the ocular disease or condition is retinopathy of prematurity, DR, pathological myopia, hypertensive retinopathy, occlusive vasculitis, polypoidal choroidal vasculopathy, diabetic macular edema, uveitic macular edema, retinal vein occlusion, ocular neovascularization, ocular histoplasmosis, neovascular glaucoma, retinoblastoma, macular degeneration, retrolental fibroplasias, retinal angiomatous proliferation, dry eye disease, uveitis, thyroid eye disease, or sickle cell retinopathy. In some embodiments, the ocular disease or condition is DR, and the DR is proliferative diabetic retinopathy. In some embodiments, the ocular disease or condition is macular degeneration, and the macular degeneration is advanced macular degeneration. In some embodiments, the macular degeneration is wet age-related macular degeneration. In some embodiments, the macular degeneration is dry age-related macular degeneration. In some embodiments, the ocular disease or condition is ocular neovascularization, and the ocular neovascularization is corneal neovascularization or retinal neovascularization. In some embodiments, retinal vein occlusion is central retinal vein occlusion or branch retinal vein occlusion.

In some embodiments, the ocular disease or condition is geographic atrophy, choroidal neovascularization, or corneal graft rejection.

The invention further provides methods for treating or preventing diabetic retinal disease, retinopathy of prematurity, pathological myopia, hypertensive retinopathy, occlusive vasculitis, polypoidal choroidal vasculopathy, diabetic macular edema, uveitic macular edema, retinal vein occlusion, ocular neovascularization, ocular histoplasmosis, neovascular glaucoma, retinoblastoma, macular degeneration, retrolental fibroplasias, retinal angiomatous proliferation, dry eye disease, uveitis, thyroid eye disease, sickle cell retinopathy, geographic atrophy, choroidal neovascularization, or corneal graft rejection, comprising administering to a subject in need thereof an effective amount of: a compound of the invention; a composition of the invention; phentolamine mesylate that exhibits an XRPD pattern that is substantially the same as that depicted in **Fig. 1A** or **Fig. 1C****;** a composition comprising (i) phentolamine mesylate that exhibits an XRPD pattern that is substantially the same as that depicted in **Fig. 1A** or **Fig. 1C** and (ii) a pharmaceutically acceptable carrier or vehicle; or a composition comprising a pharmaceutically acceptable aqueous carrier or vehicle in which phentolamine mesylate that exhibits an XRPD pattern that is substantially the same as that depicted in **Fig. 1A** or **Fig. 1C** is dissolved. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, diabetic retinal disease is diabetic retinopathy (DR) or diabetic macular edema (DME). In some embodiments, the DR is moderately severe non-proliferative DR or mild proliferative DR. In some embodiments, the DME is DME without loss of central vision. In some embodiments, the administering is topically instilling into one or both eyes of the subject.

The invention further provides methods for treating or preventing diabetic retinal disease, retinopathy of prematurity, pathological myopia, hypertensive retinopathy, occlusive vasculitis, polypoidal choroidal vasculopathy, diabetic macular edema, uveitic macular edema, retinal vein occlusion, ocular neovascularization, ocular histoplasmosis, neovascular glaucoma, retinoblastoma, macular degeneration, retrolental fibroplasias, retinal angiomatous proliferation, dry eye disease, uveitis, thyroid eye disease, sickle cell retinopathy, geographic atrophy, choroidal neovascularization, or corneal graft rejection in a subject in need thereof, comprising (a) dissolving in a pharmaceutically acceptable aqueous carrier or vehicle phentolamine mesylate that exhibits an XRPD pattern that is substantially the same as that depicted in **Fig. 1A** or **Fig. 1C** to provide an ophthalmic solution in which the phentolamine mesylate that exhibits an XRPD pattern that is substantially the same as that depicted in **Fig. 1A** or **Fig. 1C** is dissolved and (b) administering to the subject an effective amount of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, diabetic retinal disease is diabetic retinopathy (DR) or diabetic macular edema (DME). In some embodiments, the DR is moderately severe non-proliferative DR or mild proliferative DR. In some embodiments, the DME is DME without loss of central vision. In some embodiments, the administering is topically instilling into one or both eyes of the subject.

In some embodiments, the compound of the invention is useful for inhibiting the contraction of smooth muscle of the iris.

Accordingly, the invention further provides methods for inhibiting the contraction of smooth muscle of the iris, comprising administering to a subject in need thereof an effective amount of a compound of the invention or a composition of the invention. In some embodiments, the administering is topically instilling into one or both eyes of the subject.

The invention further provides methods for inhibiting the contraction of smooth muscle of the iris, comprising administering to a subject in need thereof an effective amount of phentolamine mesylate that exhibits an XRPD pattern comprising a peak at 8.4 ± 0.2 degrees 2-theta and a peak at 16.3 ± 0.2 degrees 2-theta.

The invention further provides methods for inhibiting the contraction of smooth muscle of the iris, comprising administering to a subject in need thereof an effective amount of phentolamine mesylate that exhibits an XRPD pattern that is substantially the same as that depicted in **Fig. 2A****.** In some embodiments, the administering is topically instilling into one or both eyes of the subject.

The invention further provides methods for inhibiting the contraction of smooth muscle of the iris, comprising administering to a subject in need thereof an effective amount of phentolamine mesylate that exhibits an XRPD pattern comprising a **Table 3** peak having a relative peak intensity (%) of greater than 30%. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for inhibiting the contraction of smooth muscle of the iris, comprising administering to a subject in need thereof an effective amount of phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta.

The invention further provides methods for inhibiting the contraction of smooth muscle of the iris, comprising administering to a subject in need thereof an effective amount of phentolamine mesylate that exhibits an XRPD pattern that is substantially the same as that depicted in **Fig. 3A****.** In some embodiments, the administering is topically instilling into one or both eyes of the subject.

The invention further provides methods for inhibiting the contraction of smooth muscle of the iris, comprising administering to a subject in need thereof an effective amount of phentolamine mesylate that exhibits an XRPD pattern comprising a **Table 4** peak having a relative peak intensity (%) of greater than 30%. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for inhibiting the contraction of smooth muscle of the iris, comprising administering to a subject in need thereof an effective amount of a composition of the invention. In some embodiments, the administering is topically instilling into one or both eyes of the subject.

The invention further provides methods for inhibiting the contraction of smooth muscle of the iris, comprising administering to a subject in need thereof an effective amount of a composition comprising (i) phentolamine mesylate that exhibits an XRPD pattern comprising a peak at 8.4 ± 0.2 degrees 2-theta and a peak at 16.3 ± 0.2 degrees 2-theta and (ii) a pharmaceutically acceptable carrier or vehicle. In some embodiments, the administering is topically instilling into one or both eyes of the subject.

The invention further provides methods for inhibiting the contraction of smooth muscle of the iris, comprising administering to a subject in need thereof an effective amount of a composition comprising (i) phentolamine mesylate that exhibits an XRPD pattern that is substantially the same as that depicted in **Fig. 2A** and (ii) a pharmaceutically acceptable carrier or vehicle. In some embodiments, the administering is topically instilling into one or both eyes of the subject.

The invention further provides methods for inhibiting the contraction of smooth muscle of the iris, comprising administering to a subject in need thereof an effective amount of a composition comprising (i) phentolamine mesylate that exhibits an XRPD pattern comprising a **Table 3** peak having a relative peak intensity (%) of greater than 30% and (ii) a pharmaceutically acceptable carrier or vehicle. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for inhibiting the contraction of smooth muscle of the iris, comprising administering to a subject in need thereof an effective amount of a composition comprising (i) phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta and (ii) a pharmaceutically acceptable carrier or vehicle. In some embodiments, the administering is topically instilling into one or both eyes of the subj ect.

The invention further provides methods for inhibiting the contraction of smooth muscle of the iris, comprising administering to a subject in need thereof an effective amount of a composition comprising (i) phentolamine mesylate that exhibits an XRPD pattern that is substantially the same as that depicted in **Fig. 3A** and (ii) a pharmaceutically acceptable carrier or vehicle. In some embodiments, the administering is topically instilling into one or both eyes of the subject.

The invention further provides methods for inhibiting the contraction of smooth muscle of the iris, comprising administering to a subject in need thereof an effective amount of a composition comprising (i) phentolamine mesylate that exhibits an XRPD pattern comprising a **Table 4** peak having a relative peak intensity (%) of greater than 30% and (ii) a pharmaceutically acceptable carrier or vehicle. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for inhibiting the contraction of smooth muscle of the iris, comprising administering to a subject in need thereof an effective amount of phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****.** In some embodiments, the administering is topically instilling into one or both eyes of the subj ect.

The invention further provides methods for inhibiting the contraction of smooth muscle of the iris, comprising administering to a subject in need thereof an effective amount of a composition comprising (i) phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (ii) a pharmaceutically acceptable carrier or vehicle. In some embodiments, the administering is topically instilling into one or both eyes of the subj ect.

The invention further provides methods for inhibiting the contraction of smooth muscle of the iris, comprising administering to a subject in need thereof an effective amount of an ophthalmic solution in which a compound of the invention is dissolved. In some embodiments, the administering is topically instilling into one or both eyes of the subject.

The invention further provides methods for inhibiting the contraction of smooth muscle of the iris, comprising administering to a subject in need thereof an effective amount of an ophthalmic solution in which phentolamine mesylate that exhibits an XRPD pattern comprising a peak at 8.4 ± 0.2 degrees 2-theta and a peak at 16.3 ± 0.2 degrees 2-theta is dissolved. In some embodiments, the administering is topically instilling into one or both eyes of the subject.

The invention further provides methods for inhibiting the contraction of smooth muscle of the iris, comprising administering to a subject in need thereof an effective amount of an ophthalmic solution in which phentolamine mesylate that exhibits an XRPD pattern that is substantially the same as that depicted in **Fig. 2A** is dissolved. In some embodiments, the administering is topically instilling into one or both eyes of the subject.

The invention further provides methods for inhibiting the contraction of smooth muscle of the iris, comprising administering to a subject in need thereof an effective amount of an ophthalmic solution in which phentolamine mesylate that exhibits an XRPD pattern comprising a **Table 3** peak having a relative peak intensity (%) of greater than 30% is dissolved. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for inhibiting the contraction of smooth muscle of the iris, comprising administering to a subject in need thereof an effective amount of an ophthalmic solution in which phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved. In some embodiments, the administering is topically instilling into one or both eyes of the subject.

The invention further provides methods for inhibiting the contraction of smooth muscle of the iris, comprising administering to a subject in need thereof an effective amount of an ophthalmic solution in which phentolamine mesylate that exhibits an XRPD pattern that is substantially the same as that depicted in **Fig. 3A** is dissolved. In some embodiments, the administering is topically instilling into one or both eyes of the subject.

The invention further provides methods for inhibiting the contraction of smooth muscle of the iris, comprising administering to a subject in need thereof an effective amount of an ophthalmic solution in which phentolamine mesylate that exhibits an XRPD pattern comprising a **Table 4** peak having a relative peak intensity (%) of greater than 30% is dissolved. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for inhibiting the contraction of smooth muscle of the iris, comprising administering to a subject in need thereof an effective amount of an ophthalmic solution in which phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13** is dissolved. In some embodiments, the administering is topically instilling into one or both eyes of the subject.

The invention further provides methods for inhibiting the contraction of smooth muscle of the iris in a subject in need thereof, comprising (a) dissolving in a pharmaceutically acceptable aqueous carrier or vehicle a compound of the invention to provide an ophthalmic solution in which the compound of the invention is dissolved and (b) administering to the subject the ophthalmic solution. In some embodiments, the administering is topically instilling into one or both eyes of the subject.

The invention further provides methods for inhibiting the contraction of smooth muscle of the iris in a subject in need thereof, comprising (a) dissolving in a pharmaceutically acceptable aqueous carrier or vehicle phentolamine mesylate that exhibits an XRPD pattern comprising a peak at 8.4 ± 0.2 degrees 2-theta and a peak at 16.3 ± 0.2 degrees 2-theta to provide an ophthalmic solution in which the phentolamine mesylate that exhibits an XRPD pattern comprising a peak at 8.4 ± 0.2 degrees 2-theta and a peak at 16.3 ± 0.2 degrees 2-theta is dissolved and (b) administering to the subject an effective amount of the ophthalmic solution. In some embodiments, the administering is topically instilling into one or both eyes of the subject.

The invention further provides methods for inhibiting the contraction of smooth muscle of the iris in a subject in need thereof, comprising (a) dissolving in a pharmaceutically acceptable aqueous carrier or vehicle phentolamine mesylate that exhibits an XRPD pattern that is substantially the same as that depicted in **Fig. 2A** to provide an ophthalmic solution in which the phentolamine mesylate that exhibits an XRPD pattern that is substantially the same as that depicted in **Fig. 2A** is dissolved and (b) administering to the subject an effective amount of the ophthalmic solution. In some embodiments, the administering is topically instilling into one or both eyes of the subject.

The invention further provides methods for inhibiting the contraction of smooth muscle of the iris in a subject in need thereof, comprising (a) dissolving in a pharmaceutically acceptable aqueous carrier or vehicle phentolamine mesylate that exhibits an XRPD pattern comprising a **Table 3** peak having a relative peak intensity (%) of greater than 30% to provide an ophthalmic solution in which the phentolamine mesylate that exhibits an XRPD pattern comprising a **Table 3** peak having a relative peak intensity (%) of greater than 30% is dissolved and (b) administering to the subject an effective amount of the ophthalmic solution. In some embodiments, the administering is topically instilling into one or both eyes of the subj ect.

The invention further provides for inhibiting the contraction of smooth muscle of the iris in a subject in need thereof, comprising (a) dissolving in a pharmaceutically acceptable aqueous carrier or vehicle phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta to provide an ophthalmic solution in which the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved and (b) administering to the subject an effective amount of the ophthalmic solution. In some embodiments, the administering is topically instilling into one or both eyes of the subject.

The invention further provides methods for inhibiting the contraction of smooth muscle of the iris in a subject in need thereof, comprising (a) dissolving in a pharmaceutically acceptable aqueous carrier or vehicle phentolamine mesylate that exhibits an XRPD pattern that is substantially the same as that depicted in **Fig. 3A** to provide an ophthalmic solution in which the phentolamine mesylate that exhibits an XRPD pattern that is substantially the same as that depicted in **Fig. 3A** is dissolved and (b) administering to the subject an effective amount of the ophthalmic solution. In some embodiments, the administering is topically instilling into one or both eyes of the subject.

The invention further provides methods for inhibiting the contraction of smooth muscle of the iris in a subject in need thereof, comprising (a) dissolving in a pharmaceutically acceptable aqueous carrier or vehicle phentolamine mesylate that exhibits an XRPD pattern comprising a **Table 4** peak having a relative peak intensity (%) of greater than 30% to provide an ophthalmic solution in which the phentolamine mesylate that exhibits an XRPD pattern comprising a **Table 4** peak having a relative peak intensity (%) of greater than 30% is dissolved and (b) administering to the subject an effective amount of the ophthalmic solution. In some embodiments, the administering is topically instilling into one or both eyes of the subj ect.

The invention further provides methods for inhibiting the contraction of smooth muscle of the iris in a subject in need thereof, comprising (a) dissolving in a pharmaceutically acceptable aqueous carrier or vehicle phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (b) administering to the subject an effective amount of the ophthalmic solution. In some embodiments, the administering is topically instilling into one or both eyes of the subject.

In some embodiments, the compound of the invention is useful for reducing pupil diameter.

Accordingly, the invention further provides methods for reducing pupil diameter, comprising administering to a subject in need thereof an effective amount of a compound of the invention or a composition of the invention. In some embodiments, the administering is topically instilling into one or both eyes of the subject.

The invention further provides methods for reducing pupil diameter, comprising administering to a subject in need thereof an effective amount of phentolamine mesylate that exhibits an XRPD pattern comprising a peak at 8.4 ± 0.2 degrees 2-theta and a peak at 16.3 ± 0.2 degrees 2-theta. In some embodiments, the administering is topically instilling into one or both eyes of the subject.

The invention further provides methods for reducing pupil diameter, comprising administering to a subject in need thereof an effective amount of phentolamine mesylate that exhibits an XRPD pattern that is substantially the same as that depicted in **Fig. 2A****.**

The invention further provides methods reducing pupil diameter, comprising administering to a subject in need thereof an effective amount of phentolamine mesylate that exhibits an XRPD pattern comprising a **Table 3** peak having a relative peak intensity (%) of greater than 30%. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for reducing pupil diameter, comprising administering to a subject in need thereof an effective amount of phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta. In some embodiments, the administering is topically instilling into one or both eyes of the subject.

The invention further provides methods for reducing pupil diameter, comprising administering to a subject in need thereof an effective amount of phentolamine mesylate that exhibits an XRPD pattern that is substantially the same as that depicted in **Fig. 3A****.**

The invention further provides methods for reducing pupil diameter, comprising administering to a subject in need thereof an effective amount of phentolamine mesylate that exhibits an XRPD pattern comprising a **Table 4** peak having a relative peak intensity (%) of greater than 30%. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for reducing pupil diameter, comprising administering to a subject in need thereof an effective amount of a composition of the invention. In some embodiments, the administering is topically instilling into one or both eyes of the subject.

The invention further provides methods for reducing pupil diameter, comprising administering to a subject in need thereof an effective amount of a composition comprising (i) phentolamine mesylate that exhibits an XRPD pattern comprising a peak at 8.4 ± 0.2 degrees 2-theta and a peak at 16.3 ± 0.2 degrees 2-theta and (ii) a pharmaceutically acceptable carrier or vehicle. In some embodiments, the administering is topically instilling into one or both eyes of the subject.

The invention further provides methods for reducing pupil diameter, comprising administering to a subject in need thereof an effective amount of a composition comprising (i) phentolamine mesylate that exhibits an XRPD pattern that is substantially the same as that depicted in **Fig. 2A** and (ii) a pharmaceutically acceptable carrier or vehicle. In some embodiments, the administering is topically instilling into one or both eyes of the subject.

The invention further provides methods for reducing pupil diameter, comprising administering to a subject in need thereof an effective amount of a composition comprising (i) phentolamine mesylate that exhibits an XRPD pattern comprising a **Table 3** peak having a relative peak intensity (%) of greater than 30% and (ii) a pharmaceutically acceptable carrier or vehicle. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for reducing pupil diameter, comprising administering to a subject in need thereof an effective amount of a composition comprising (i) phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta and (ii) a pharmaceutically acceptable carrier or vehicle. In some embodiments, the administering is topically instilling into one or both eyes of the subject.

The invention further provides methods for reducing pupil diameter, comprising administering to a subject in need thereof an effective amount of a composition comprising (i) phentolamine mesylate that exhibits an XRPD pattern that is substantially the same as that depicted in **Fig. 3A** and (ii) a pharmaceutically acceptable carrier or vehicle. In some embodiments, the administering is topically instilling into one or both eyes of the subject.

The invention further provides methods for reducing pupil diameter, comprising administering to a subject in need thereof an effective amount of a composition comprising (i) phentolamine mesylate that exhibits an XRPD pattern comprising a **Table 4** peak having a relative peak intensity (%) of greater than 30% and (ii) a pharmaceutically acceptable carrier or vehicle. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for reducing pupil diameter, comprising administering to a subject in need thereof an effective amount of phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****.** In some embodiments, the administering is topically instilling into one or both eyes of the subject.

The invention further provides methods for reducing pupil diameter, comprising administering to a subject in need thereof an effective amount of a composition comprising (i) phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (ii) a pharmaceutically acceptable carrier or vehicle. In some embodiments, the administering is topically instilling into one or both eyes of the subject.

The invention further provides methods for reducing pupil diameter, comprising administering to a subject in need thereof an effective amount of an ophthalmic solution in which a compound of the invention is dissolved. In some embodiments, the administering is topically instilling into one or both eyes of the subject.

The invention further provides methods for reducing pupil diameter, comprising administering to a subject in need thereof an effective amount of an ophthalmic solution in which phentolamine mesylate that exhibits an XRPD pattern comprising a peak at 8.4 ± 0.2 degrees 2-theta and a peak at 16.3 ± 0.2 degrees 2-theta is dissolved. In some embodiments, the administering is topically instilling into one or both eyes of the subject.

The invention further provides methods for reducing pupil diameter, comprising administering to a subject in need thereof an effective amount of an ophthalmic solution in which phentolamine mesylate that exhibits an XRPD pattern that is substantially the same as that depicted in **Fig. 2A** is dissolved. In some embodiments, the administering is topically instilling into one or both eyes of the subject.

The invention further provides methods for reducing pupil diameter, comprising administering to a subject in need thereof an effective amount of an ophthalmic solution in which phentolamine mesylate that exhibits an XRPD pattern comprising a **Table 3** peak having a relative peak intensity (%) of greater than 30% is dissolved. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for reducing pupil diameter, comprising administering to a subject in need thereof an effective amount of an ophthalmic solution in which phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved. In some embodiments, the administering is topically instilling into one or both eyes of the subject.

The invention further provides methods for reducing pupil diameter, comprising administering to a subject in need thereof an effective amount of an ophthalmic solution in which phentolamine mesylate that exhibits an XRPD pattern that is substantially the same as that depicted in **Fig. 3A** is dissolved. In some embodiments, the administering is topically instilling into one or both eyes of the subject.

The invention further provides methods for reducing pupil diameter, comprising administering to a subject in need thereof an effective amount of an ophthalmic solution in which phentolamine mesylate that exhibits an XRPD pattern comprising a **Table 4** peak having a relative peak intensity (%) of greater than 30% is dissolved. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for reducing pupil diameter, comprising administering to a subject in need thereof an effective amount of an ophthalmic solution in which phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13** is dissolved. In some embodiments, the administering is topically instilling into one or both eyes of the subject.

The invention further provides methods for reducing pupil diameter in a subject in need thereof, comprising (a) dissolving in a pharmaceutically acceptable aqueous carrier or vehicle a compound of the invention to provide an ophthalmic solution in which the compound of the invention is dissolved and (b) administering to the subject the ophthalmic solution. In some embodiments, the administering is topically instilling into one or both eyes of the subject.

The invention further provides methods for reducing pupil diameter in a subject in need thereof, comprising (a) dissolving in a pharmaceutically acceptable aqueous carrier or vehicle phentolamine mesylate that exhibits an XRPD pattern comprising a peak at 8.4 ± 0.2 degrees 2-theta and a peak at 16.3 ± 0.2 degrees 2-theta to provide an ophthalmic solution in which the phentolamine mesylate that exhibits an XRPD pattern comprising a peak at 8.4 ± 0.2 degrees 2-theta and a peak at 16.3 ± 0.2 degrees 2-theta is dissolved and (b) administering to the subject an effective amount of the ophthalmic solution. In some embodiments, the administering is topically instilling into one or both eyes of the subject.

The invention further provides methods for reducing pupil diameter in a subject in need thereof, comprising (a) dissolving in a pharmaceutically acceptable aqueous carrier or vehicle phentolamine mesylate that exhibits an XRPD pattern that is substantially the same as that depicted in **Fig. 2A** to provide an ophthalmic solution in which the phentolamine mesylate that exhibits an XRPD pattern that is substantially the same as that depicted in **Fig. 2A** is dissolved and (b) administering to the subject an effective amount of the ophthalmic solution. In some embodiments, the administering is topically instilling into one or both eyes of the subject.

The invention further provides methods for reducing pupil diameter in a subject in need thereof, comprising (a) dissolving in a pharmaceutically acceptable aqueous carrier or vehicle phentolamine mesylate that exhibits an XRPD pattern comprising a **Table 3** peak having a relative peak intensity (%) of greater than 30% to provide an ophthalmic solution in which the phentolamine mesylate that exhibits an XRPD pattern comprising a **Table 3** peak having a relative peak intensity (%) of greater than 30% is dissolved and (b) administering to the subject an effective amount of the ophthalmic solution. In some embodiments, the administering is topically instilling into one or both eyes of the subject.

The invention further provides for reducing pupil diameter in a subject in need thereof, comprising (a) dissolving in a pharmaceutically acceptable aqueous carrier or vehicle phentolamine mesylate that exhibits XRPD pattern comprising a peak at 8.3 ± 0.2 degrees 2-theta, a peak at 13.3 ± 0.2 degrees 2-theta and a peak at 19.7 ± 0.2 degrees 2-theta to provide an ophthalmic solution in which the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved and (b) administering to the subject an effective amount of the ophthalmic solution. In some embodiments, the administering is topically instilling into one or both eyes of the subject.

The invention further provides methods for reducing pupil diameter in a subject in need thereof, comprising (a) dissolving in a pharmaceutically acceptable aqueous carrier or vehicle phentolamine mesylate that exhibits an XRPD pattern that is substantially the same as that depicted in **Fig. 3A** to provide an ophthalmic solution in which the phentolamine mesylate that exhibits an XRPD pattern that is substantially the same as that depicted in **Fig. 3A** is dissolved and (b) administering to the subject an effective amount of the ophthalmic solution. In some embodiments, the administering is topically instilling into one or both eyes of the subject.

The invention further provides methods for reducing pupil diameter in a subject in need thereof, comprising (a) dissolving in a pharmaceutically acceptable aqueous carrier or vehicle phentolamine mesylate that exhibits an XRPD pattern comprising a **Table 4** peak having a relative peak intensity (%) of greater than 30% to provide an ophthalmic solution in which the phentolamine mesylate that exhibits an XRPD pattern comprising a **Table 4** peak having a relative peak intensity (%) of greater than 30% is dissolved and (b) administering to the subject an effective amount of the ophthalmic solution. In some embodiments, the administering is topically instilling into one or both eyes of the subject.

The invention further provides methods for reducing pupil diameter in a subject in need thereof, comprising (a) dissolving in a pharmaceutically acceptable aqueous carrier or vehicle phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (b) administering to the subject an effective amount of the ophthalmic solution. In some embodiments, the administering is topically instilling into one or both eyes of the subj ect.

In some embodiments, the compound of the invention is useful for improving visual contrast sensitivity or visual acuity.

Accordingly, the invention further provides methods for improving visual contrast sensitivity or visual acuity, comprising administering to a subject in need thereof an effective amount of a compound of the invention or a composition of the invention. In some embodiments, the administering is topically instilling into one or both eyes of the subject.

The invention further provides methods for improving visual contrast sensitivity or visual acuity, comprising administering to a subject in need thereof an effective amount of phentolamine mesylate that exhibits an XRPD pattern comprising a peak at 8.4 ± 0.2 degrees 2-theta and a peak at 16.3 ± 0.2 degrees 2-theta. In some embodiments, the administering is topically instilling into one or both eyes of the subject.

The invention further provides methods for improving visual contrast sensitivity or visual acuity, comprising administering to a subject in need thereof an effective amount of phentolamine mesylate that exhibits an XRPD pattern that is substantially the same as that depicted in **Fig. 2A****.** In some embodiments, the administering is topically instilling into one or both eyes of the subject.

The invention further provides methods for improving visual contrast sensitivity or visual acuity, comprising administering to a subject in need thereof an effective amount of phentolamine mesylate that exhibits an XRPD pattern comprising a **Table 3** peak having a relative peak intensity (%) of greater than 30%. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for improving visual contrast sensitivity or visual acuity, comprising administering to a subject in need thereof an effective amount of phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta. In some embodiments, the administering is topically instilling into one or both eyes of the subject.

The invention further provides methods for improving visual contrast sensitivity or visual acuity, comprising administering to a subject in need thereof an effective amount of phentolamine mesylate that exhibits an XRPD pattern that is substantially the same as that depicted in **Fig. 3A****.** In some embodiments, the administering is topically instilling into one or both eyes of the subject.

The invention further provides methods for improving visual contrast sensitivity or visual acuity, comprising administering to a subject in need thereof an effective amount of phentolamine mesylate that exhibits an XRPD pattern comprising a **Table 4** peak having a relative peak intensity (%) of greater than 30%. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for improving visual contrast sensitivity or visual acuity, comprising administering to a subject in need thereof an effective amount of a composition of the invention. In some embodiments, the administering is topically instilling into one or both eyes of the subject.

The invention further provides methods for improving visual contrast sensitivity or visual acuity, comprising administering to a subject in need thereof an effective amount of a composition comprising (i) phentolamine mesylate that exhibits an XRPD pattern comprising a peak at 8.4 ± 0.2 degrees 2-theta and a peak at 16.3 ± 0.2 degrees 2-theta and (ii) a pharmaceutically acceptable carrier or vehicle. In some embodiments, the administering is topically instilling into one or both eyes of the subject.

The invention further provides methods for improving visual contrast sensitivity or visual acuity, comprising administering to a subject in need thereof an effective amount of a composition comprising (i) phentolamine mesylate that exhibits an XRPD pattern that is substantially the same as that depicted in **Fig. 2A** and (ii) a pharmaceutically acceptable carrier or vehicle. In some embodiments, the administering is topically instilling into one or both eyes of the subject.

The invention further provides methods for improving visual contrast sensitivity or visual acuity, comprising administering to a subject in need thereof an effective amount of a composition comprising (i) phentolamine mesylate that exhibits an XRPD pattern comprising a **Table 3** peak having a relative peak intensity (%) of greater than 30% and (ii) a pharmaceutically acceptable carrier or vehicle. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for improving visual contrast sensitivity or visual acuity, comprising administering to a subject in need thereof an effective amount of a composition comprising (i) phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta and (ii) a pharmaceutically acceptable carrier or vehicle. In some embodiments, the administering is topically instilling into one or both eyes of the subject.

The invention further provides methods for improving visual contrast sensitivity or visual acuity, comprising administering to a subject in need thereof an effective amount of a composition comprising (i) phentolamine mesylate that exhibits an XRPD pattern that is substantially the same as that depicted in **Fig. 3A** and (ii) a pharmaceutically acceptable carrier or vehicle. In some embodiments, the administering is topically instilling into one or both eyes of the subject.

The invention further provides methods for improving visual contrast sensitivity or visual acuity, comprising administering to a subject in need thereof an effective amount of a composition comprising (i) phentolamine mesylate that exhibits an XRPD pattern comprising a **Table 4** peak having a relative peak intensity (%) of greater than 30% and (ii) a pharmaceutically acceptable carrier or vehicle. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for improving visual contrast sensitivity or visual acuity, comprising administering to a subject in need thereof an effective amount of phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****.** In some embodiments, the administering is topically instilling into one or both eyes of the subj ect.

The invention further provides methods for improving visual contrast sensitivity or visual acuity, comprising administering to a subject in need thereof an effective amount of a composition comprising (i) phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (ii) a pharmaceutically acceptable carrier or vehicle.

The invention further provides methods for improving visual contrast sensitivity or visual acuity, comprising administering to a subject in need thereof an effective amount of an ophthalmic solution in which a compound of the invention is dissolved. In some embodiments, the administering is topically instilling into one or both eyes of the subject.

The invention further provides methods for improving visual contrast sensitivity or visual acuity, comprising administering to a subject in need thereof an effective amount of an ophthalmic solution in which phentolamine mesylate that exhibits an XRPD pattern comprising a peak at 8.4 ± 0.2 degrees 2-theta and a peak at 16.3 ± 0.2 degrees 2-theta is dissolved. In some embodiments, the administering is topically instilling into one or both eyes of the subject.

The invention further provides methods for improving visual contrast sensitivity or visual acuity, comprising administering to a subject in need thereof an effective amount of an ophthalmic solution in which phentolamine mesylate that exhibits an XRPD pattern that is substantially the same as that depicted in **Fig. 2A** is dissolved. In some embodiments, the administering is topically instilling into one or both eyes of the subject.

The invention further provides methods for improving visual contrast sensitivity or visual acuity, comprising administering to a subject in need thereof an effective amount of an ophthalmic solution in which phentolamine mesylate that exhibits an XRPD pattern comprising a **Table 3** peak having a relative peak intensity (%) of greater than 30% is dissolved. In some embodiments, the phentolamine mesylate exhibits an XRPD pattern comprising a **Table 3** peak having a relative peak intensity (%) of greater than 40%. In some embodiments, the phentolamine mesylate exhibits an XRPD pattern comprising a **Table 3** peak having a relative peak intensity (%) of greater than 50%. In some embodiments, the administering is topically instilling into one or both eyes of the subject.

The invention further provides methods for improving visual contrast sensitivity or visual acuity, comprising administering to a subject in need thereof an effective amount of an ophthalmic solution in which phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved. In some embodiments, the administering is topically instilling into one or both eyes of the subject.

The invention further provides methods for improving visual contrast sensitivity or visual acuity, comprising administering to a subject in need thereof an effective amount of an ophthalmic solution in which phentolamine mesylate that exhibits an XRPD pattern that is substantially the same as that depicted in **Fig. 3A** is dissolved. In some embodiments, the administering is topically instilling into one or both eyes of the subject.

The invention further provides methods for improving visual contrast sensitivity or visual acuity, comprising administering to a subject in need thereof an effective amount of an ophthalmic solution in which phentolamine mesylate that exhibits an XRPD pattern comprising a **Table 4** peak having a relative peak intensity (%) of greater than 30% is dissolved. In some embodiments, the phentolamine mesylate exhibits an XRPD pattern comprising a **Table 4** peak having a relative peak intensity (%) of greater than 40%. In some embodiments, the phentolamine mesylate exhibits an XRPD pattern comprising a **Table 4** peak having a relative peak intensity (%) of greater than 50%. In some embodiments, the administering is topically instilling into one or both eyes of the subject.

The invention further provides methods for improving visual contrast sensitivity or visual acuity, comprising administering to a subject in need thereof an effective amount of an ophthalmic solution in which phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13** is dissolved. In some embodiments, the administering is topically instilling into one or both eyes of the subject.

The invention further provides methods for improving visual contrast sensitivity or visual acuity in a subject in need thereof, comprising (a) dissolving in a pharmaceutically acceptable aqueous carrier or vehicle a compound of the invention to provide an ophthalmic solution in which the compound of the invention is dissolved and (b) administering to the subject the ophthalmic solution. In some embodiments, the administering is topically instilling into one or both eyes of the subject.

The invention further provides methods for improving visual contrast sensitivity or visual acuity in a subject in need thereof, comprising (a) dissolving in a pharmaceutically acceptable aqueous carrier or vehicle phentolamine mesylate that exhibits an XRPD pattern comprising a peak at 8.4 ± 0.2 degrees 2-theta and a peak at 16.3 ± 0.2 degrees 2-theta to provide an ophthalmic solution in which the phentolamine mesylate that exhibits an XRPD pattern comprising a peak at 8.4 ± 0.2 degrees 2-theta and a peak at 16.3 ± 0.2 degrees 2-theta is dissolved and (b) administering to the subject an effective amount of the ophthalmic solution. In some embodiments, the administering is topically instilling into one or both eyes of the subject.

The invention further provides methods for improving visual contrast sensitivity or visual acuity in a subject in need thereof, comprising (a) dissolving in a pharmaceutically acceptable aqueous carrier or vehicle phentolamine mesylate that exhibits an XRPD pattern that is substantially the same as that depicted in **Fig. 2A** to provide an ophthalmic solution in which the phentolamine mesylate that exhibits an XRPD pattern that is substantially the same as that depicted in **Fig. 2A** is dissolved and (b) administering to the subject an effective amount of the ophthalmic solution. In some embodiments, the administering is topically instilling into one or both eyes of the subject.

The invention further provides methods for improving visual contrast sensitivity or visual acuity in a subject in need thereof, comprising (a) dissolving in a pharmaceutically acceptable aqueous carrier or vehicle phentolamine mesylate that exhibits an XRPD pattern comprising a **Table 3** peak having a relative peak intensity (%) of greater than 30% to provide an ophthalmic solution in which the phentolamine mesylate that exhibits an XRPD pattern comprising a **Table 3** peak having a relative peak intensity (%) of greater than 30% is dissolved and (b) administering to the subject an effective amount of the ophthalmic solution. In some embodiments, the administering is topically instilling into one or both eyes of the subj ect.

The invention further provides for improving visual contrast sensitivity or visual acuity in a subject in need thereof, comprising (a) dissolving in a pharmaceutically acceptable aqueous carrier or vehicle phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta to provide an ophthalmic solution in which the phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta is dissolved and (b) administering to the subject an effective amount of the ophthalmic solution. In some embodiments, the administering is topically instilling into one or both eyes of the subject.

The invention further provides methods for improving visual contrast sensitivity or visual acuity in a subject in need thereof, comprising (a) dissolving in a pharmaceutically acceptable aqueous carrier or vehicle phentolamine mesylate that exhibits an XRPD pattern that is substantially the same as that depicted in **Fig. 3A** to provide an ophthalmic solution in which the phentolamine mesylate that exhibits an XRPD pattern that is substantially the same as that depicted in **Fig. 3A** is dissolved and (b) administering to the subject an effective amount of the ophthalmic solution. In some embodiments, the administering is topically instilling into one or both eyes of the subject.

The invention further provides methods for improving visual contrast sensitivity or visual acuity in a subject in need thereof, comprising (a) dissolving in a pharmaceutically acceptable aqueous carrier or vehicle phentolamine mesylate that exhibits an XRPD pattern comprising a **Table 4** peak having a relative peak intensity (%) of greater than 30% to provide an ophthalmic solution in which the phentolamine mesylate that exhibits an XRPD pattern comprising a **Table 4** peak having a relative peak intensity (%) of greater than 30% is dissolved and (b) administering to the subject an effective amount of the ophthalmic solution. In some embodiments, the administering is topically instilling into one or both eyes of the subj ect.

The invention further provides methods for improving visual contrast sensitivity or visual acuity in a subject in need thereof, comprising (a) dissolving in a pharmaceutically acceptable aqueous carrier or vehicle phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****,** and (b) administering to the subject an effective amount of the ophthalmic solution. In some embodiments, the administering is topically instilling into one or both eyes of the subject.

In some embodiments, the administering is topically instilling into one or both eyes of the subject.

In some embodiments, the effective amount is about 0.01 mg to about 100 mg. In some embodiments, the effective amount is about 0.05 mg to about 50 mg. In some embodiments, the effective amount is about 0.1 mg to about 100 mg. In some embodiments, the effective amount is about 1 mg to about 25 mg. In some embodiments, the effective amount is about 5 mg to about 10 mg. In some embodiments, the effective amount is a daily amount.

In some embodiments, the effective amount is about 0.1 mg to about 2.0 mg, about 0.2 mg to about 0.7 mg, about 0.4 mg to about 0.6 mg, or about 0.8 mg to about 1.2 mg. In some embodiments, the effective amount is about 0.5 mg or about 1 mg. In some embodiments, the effective amount is a daily amount.

In some embodiments, the effective amount is about 0.1 mg to about 2.0 mg. In some embodiments, the effective amount is about 0.1 mg to about 1.0 mg. In some embodiments, the effective amount is about 0.2 mg to about 0.7 mg. In some embodiments, the effective amount is about 0.4 mg to about 0.6 mg. In some embodiments, the effective amount is about 0.25 mg, about 0.5 mg or about 1.0 mg. In some embodiments, the effective amount is about 0.5 mg. In some embodiments, the effective amount is a daily amount.

In some embodiments, the effective amount is about 0.1 mg/day to about 20 mg/day. In some embodiments, the effective amount is about 0.1 mg/day to about 10 mg/day. In some embodiments, the effective amount is about 0.1 mg/day to about 5 mg/day. In some embodiments, the effective amount is about 0.1 mg/day to about 2.0 mg/day. In some embodiments, the effective amount is about 0.1 mg/day, about 0.2 mg/day, about 0.3 mg/day, about 0.4 mg/day, about 0.5 mg/day, about 0.6 mg/day, about 0.7 mg/day, about 0.8 mg/day, about 0.9 mg/day, about 1.0 mg/day, about 1.1 mg/day, about 1.2 mg/day, about 1.3 mg/day, about 1.4 mg/day, about 1.5 mg/day, about 1.6 mg/day, about 1.7 mg/day, about 1.8 mg/day, about 1.9 mg/day, or about 2.0 mg/day.

### Compositions

In some embodiments, the composition of the invention or the composition comprising (i) phentolamine mesylate that exhibits an XRPD pattern that is substantially the same as that depicted in **Fig. 1A** or **Fig. 1C****,** and (ii) a pharmaceutically acceptable carrier or vehicle, is an ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the ophthalmic solution is suitable for topical, e.g., ocular instillation; subconjunctival; intravitreal; retrobulbar; intracameral; or systemic administration.

In some embodiments, a composition of the invention or a composition comprising (i) phentolamine mesylate that exhibits an XRPD pattern that is substantially the same as that depicted in **Fig. 1A** or **Fig. 1C****,** and (ii) a pharmaceutically acceptable carrier or vehicle, is an ophthalmic solution, or is coated on or incorporated in an ophthalmic drug delivery device. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

In some embodiments, a compound of the invention or phentolamine mesylate that exhibits an XRPD pattern that is substantially the same as that depicted in **Fig. 1A** or **Fig. 1C** is dissolved in a pharmaceutically acceptable aqueous carrier or vehicle. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

In some embodiments, the compositions disclosed herein are in the form of an ophthalmic solution. In some embodiments, the compound of the invention or phentolamine mesylate that exhibits an XRPD pattern that is substantially the same as that depicted in **Fig. 1A** or **Fig. 1C** is dissolved in the ophthalmic solution in an amount of about 0.5% to about 2% by weight of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the compound of the invention or phentolamine mesylate that exhibits an XRPD pattern that is substantially the same as that depicted in **Fig. 1A** or **Fig. 1C** is dissolved in the ophthalmic solution in an amount that is molar equivalent to about 0.5% to about 5% of phentolamine mesylate by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the compound of the invention or phentolamine mesylate that exhibits an XRPD pattern that is substantially the same as that depicted in **Fig. 1A** or **Fig. 1C** is dissolved in the ophthalmic solution in an amount that is molar equivalent to about 0.35% or about 1% of phentolamine mesylate by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the compound of the invention or phentolamine mesylate that exhibits an XRPD pattern that is substantially the same as that depicted in **Fig. 1A** or **Fig. 1C** is dissolved in the ophthalmic solution in an amount that is molar equivalent to about 0.37% or about 0.5% of phentolamine mesylate by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the compound of the invention or phentolamine mesylate that exhibits an XRPD pattern that is substantially the same as that depicted in **Fig. 1A** or **Fig. 1C** is dissolved in the ophthalmic solution in an amount of about 0.35% or about 0.75% by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the compound of the invention or phentolamine mesylate that exhibits an XRPD pattern that is substantially the same as that depicted in **Fig. 1A** or **Fig. 1C** is dissolved in the ophthalmic solution in an amount of about 0.75% by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the compound of the invention or phentolamine mesylate that exhibits an XRPD pattern that is substantially the same as that depicted in **Fig. 1A** or **Fig. 1C** is dissolved in the ophthalmic solution in an amount of about 0.75% by weight the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the compound of the invention or phentolamine mesylate that exhibits an XRPD pattern that is substantially the same as that depicted in **Fig. 1A** or **Fig. 1C** is dissolved in the ophthalmic solution in an amount of about 0.75% by volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

In some embodiments, the composition comprises about 1% of a compound of the invention or phentolamine mesylate that exhibits an XRPD pattern that is substantially the same as that depicted in **Fig. 1A** or **Fig. 1C** by weight of the composition. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition comprises about 1% of a compound of the invention or phentolamine mesylate that exhibits an XRPD pattern that is substantially the same as that depicted in **Fig. 1A** or **Fig. 1C** by volume of the composition. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution.

In some embodiments, the composition comprises about 0.5% of a compound of the invention or phentolamine mesylate that exhibits an XRPD pattern that is substantially the same as that depicted in **Fig. 1A** or **Fig. 1C** by weight of the composition. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition comprises about 0.5% of a compound of the invention or phentolamine mesylate that exhibits an XRPD pattern that is substantially the same as that depicted in **Fig. 1A** or **Fig. 1C** by volume of the composition. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution.

In some embodiments, the composition is an ophthalmic solution in which a compound of the invention or phentolamine that exhibits an XRPD pattern that is substantially the same as that depicted in **Fig. 1A** or **Fig. 1C** is dissolved in an amount of about 0.5% to about 2% by weight of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which a compound of the invention or phentolamine mesylate that exhibits an XRPD pattern that is substantially the same as that depicted in **Fig. 1A** or **Fig. 1C** is dissolved in an amount that is molar equivalent to about 0.5% to about 5% of phentolamine mesylate by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which a compound of the invention or phentolamine mesylate that exhibits an XRPD pattern that is substantially the same as that depicted in **Fig. 1A** or **Fig. 1C** is dissolved in an amount that is molar equivalent to about 0.35% or about 1% of phentolamine mesylate by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which a compound of the invention or phentolamine mesylate that exhibits an XRPD pattern that is substantially the same as that depicted in **Fig. 1A** or **Fig. 1C** is dissolved in an amount that is molar equivalent to about 0.37% or about 0.5% of phentolamine mesylate by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which a compound of the invention or phentolamine mesylate that exhibits an XRPD pattern that is substantially the same as that depicted in **Fig. 1A** or **Fig. 1C** is dissolved in an amount of about 0.35% or about 0.75% by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which a compound of the invention or phentolamine mesylate that exhibits an XRPD pattern that is substantially the same as that depicted in **Fig. 1A** or **Fig. 1C** is dissolved in an amount of about 0.75% by weight or volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which a compound of the invention or phentolamine that exhibits an XRPD pattern that is substantially the same as that depicted in **Fig. 1A** or **Fig. 1C** is dissolved in an amount of about 0.75% by weight the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which a compound of the invention or phentolamine mesylate that exhibits an XRPD pattern that is substantially the same as that depicted in **Fig. 1A** or **Fig. 1C** is dissolved in an amount of about 0.75% by volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

In some embodiments, the composition is an ophthalmic solution in which about 1% of a compound of the invention or phentolamine mesylate that exhibits an XRPD pattern that is substantially the same as that depicted in **Fig. 1A** or **Fig. 1C** is dissolved by weight of the composition. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the composition is an ophthalmic solution in which about 1% of a compound of the invention or phentolamine mesylate that exhibits an XRPD pattern that is substantially the same as that depicted in **Fig. 1A** or **Fig. 1C** by volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

In some embodiments, the composition is an ophthalmic solution in which about 0.5% of a compound of the invention or phentolamine mesylate that exhibits an XRPD pattern that is substantially the same as that depicted in **Fig. 1A** or **Fig. 1C** is dissolved by weight of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

In some embodiments, the composition is an ophthalmic solution in which about 0.5% of a compound of the invention or phentolamine mesylate that exhibits an XRPD pattern that is substantially the same as that depicted in **Fig. 1A** or **Fig. 1C** is dissolved by volume of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

The present invention further provides methods for making an ophthalmic solution, comprising dissolving in a pharmaceutically acceptable aqueous carrier or vehicle: a compound of the invention; phentolamine mesylate that exhibits an XRPD pattern that is substantially the same as that depicted in **Fig. 1A** or **Fig. 1C****;** phentolamine mesylate that exhibits an XRPD pattern comprising a peak at 8.4 ± 0.2 degrees 2-theta and a peak at 16.3 ± 0.2 degrees 2-theta; phentolamine mesylate that exhibits an XRPD pattern that is substantially the same as that depicted in **Fig. 2A****;** phentolamine mesylate that exhibits an XRPD pattern comprising a **Table 3** peak having a relative peak intensity (%) of greater than 30%; phentolamine mesylate that exhibits an XRPD pattern comprising a peak at 8.3 ± 0.2 degrees 2-theta, a peak at 13.3 ± 0.2 degrees 2-theta and a peak at 19.7 ± 0.2 degrees 2-theta; phentolamine mesylate that exhibits an XRPD pattern that is substantially the same as that depicted in **Fig. 3A****;** or phentolamine mesylate that exhibits an XRPD pattern comprising a **Table 4** peak having a relative peak intensity (%) of greater than 30%. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.**

The present invention further provides methods for making an ophthalmic solution, comprising dissolving in a pharmaceutically acceptable aqueous carrier or vehicle phentolamine mesylate that exhibits an XRPD pattern that is substantially the same as that depicted in **Fig. 4A****,** **Fig. 5A****,** **Fig. 6A****,** **Fig. 7****,** **Fig. 8****,** **Fig. 9****,** **Fig. 10****,** **Fig. 11****,** **Fig. 12****,** or **Fig. 13****.**

In some embodiments, the ophthalmic solution is suitable for ocular administration or ophthalmic use. In some embodiments, the ophthalmic solution is suitable for topical, subconjunctival, intravitreal, retrobulbar, intracameral or systemic administration.

In some embodiments, an ophthalmic solution in which a compound of the invention is dissolved comprises water, and the compound of the invention is present in the ophthalmic solution at a concentration of about 1% w/v of the ophthalmic solution. In some embodiments, the ophthalmic solution further comprises mannitol, and the mannitol is present in the ophthalmic solution at a concentration of about 4% w/v of the ophthalmic solution. In some embodiments, the ophthalmic solution further comprises sodium acetate trihydrate, and the sodium acetate trihydrate is present in the ophthalmic solution at a concentration of about 0.041% w/v of the ophthalmic solution. In some embodiments, the ophthalmic solution has a pH ranging from about 4.5 to about 5.3.

In some embodiments, an ophthalmic solution in which phentolamine mesylate that exhibits an XRPD pattern that is substantially the same as that depicted in **Fig. 1A** or **Fig. 1C** is dissolved comprises water, and the phentolamine is present in the ophthalmic solution at a concentration of about 1% w/v of the ophthalmic solution. In some embodiments, the phentolamine mesylate exhibits an XRPD pattern that is substantially the same as that depicted in **Fig. 1A****.** In some embodiments, the phentolamine mesylate exhibits an XRPD pattern that is substantially the same as that depicted in **Fig. 1C****.** In some embodiments, the ophthalmic solution further comprises mannitol, and the mannitol is present in the ophthalmic solution at a concentration of about 4% w/v of the ophthalmic solution. In some embodiments, the ophthalmic solution further comprises sodium acetate trihydrate, and the sodium acetate trihydrate is present in the ophthalmic solution at a concentration of about 0.041% w/v of the ophthalmic solution. In some embodiments, the ophthalmic solution has a pH ranging from about 4.5 to about 5.3.

In some embodiments, the pharmaceutically acceptable carrier or vehicle is a stabilizer, binder, filler, diluent, disintegrant, wetting agent, lubricant, glidant, coloring agent, dye-migration inhibitor, sweetening agent, flavoring agent, viscosity modifying agent, pH adjusting agent, buffer, osmotic agent, chelating agent, surfactants, or co-solvent.

In some embodiments, a viscosity modifying agent is polyvinyl alcohol, poloxamers, hyaluronic acid, carbomers, and polysaccharides, that is, cellulose derivatives, gellan gum, or xanthan gum.

In some embodiments, the pharmaceutically acceptable aqueous carrier or vehicle is sterile water, sterile buffer solution, or sterile saline.

In some embodiments, the pharmaceutically acceptable carrier or vehicle comprises or is mannitol or sodium acetate.

In some embodiments, the compositions disclosed herein comprise a preservative. In some embodiments, the preservative is benzalkonium chloride, cetrimide, polyquaternium-1, thimerosal, sodium perborate, stabilized oxychloro complex, stabilized chlorite peroxide, chlorhexidine, chlorobutanol, phenylethanol or methylparaben.

In some embodiments, the compositions disclosed herein do not comprise a preservative. In some embodiments, the compositions are preservative free.

In some embodiments, the compositions disclosed herein are sterile.

In some embodiments, the compositions disclosed herein have a pH in the range of about 4 to about 6. In some embodiments, the compositions of the invention have a pH in the range of about 4.5 to about 5.3. In some embodiments, the compositions have a pH of about 4.5, about 4.6, about 4.7, about 4.8, about 4.9, about 5.0, about 5.1, about 5.2, or about 5.3.

In some embodiments, a compound or composition disclosed herein is contained in a sealed container. In some embodiments, the sealed container further contains an inert gas. In some embodiments, the inert gas is argon or nitrogen.

### EXAMPLES

### General Procedures:

Unless otherwise noted, XRPD data was collected using a PANanalytical EMPYREAN vs. 8.2 20201404 diffractometer with a Cu-Kα radiation (λ = 1.541874 Å) source operating at 45 kV and 40 mA in transmission theta-theta configuration. Samples were scanned from 2° to 40° 2-theta with a step size of 0.013° 2-theta and a time per step of 78.795 sec. Data collection was performed with DATA COLLECTOR vs. 7.1 and processed with Data Viewer vs. 2.1 and Profex 5.2.4 software.

Unless otherwise noted, DSC analyses were performed using a Thermal Analysis (TA) Discovery instrument model DSC 25. The samples, typically weighing 1-3 mg, were heated in a sealed standard Tzero aluminum pan at a heating rate of 10 °C/min under a nitrogen glass flow of 50 mL/min. A sealed empty Tzero aluminum pan was used as a reference. Data collection was performed with TRIOS software and processed with TRIOS vs. 5.5 software.

Unless otherwise noted, thermogravimetric analysis (TGA) was conducted in a Thermal Analysis (TA) Discovery instrument model TGA 550 using a nitrogen atmosphere with a gas flow of 60 mL/min and a heating rate of 10 °C/min. Approximately 1 to 5 mg of sample was used. Data collection was performed with TRIOS software and processed with TRIOS vs. 5.5 software.

Unless otherwise noted, proton nuclear magnetic resonance (¹H NMR) analyses were performed using an Agilent Mercury400 NMR Spectrometer. Between 5-15 mg of the sample were dissolved in 0.5 mL-0-7 mL deuterated DMSO.

Unless otherwise noted, temperature-controlled experiments were performed with a Polar Bear Plus heating and cooling platform from Cambridge Reactor Design.

### Reference Example A.

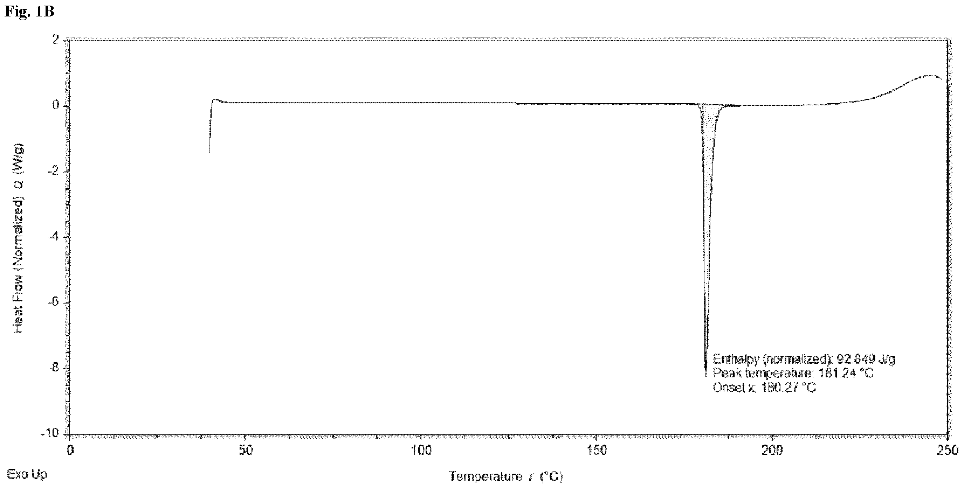

**Fig. 1A** shows an XRPD pattern of Reference A, and **Table 1** lists XRPD peaks represented in **Fig. 1A****.** **Fig. 1B** shows a DSC thermogram of Reference A.

**Table 1. XRPD Data for Reference A**

| **2-theta Angle (°)** | **d Value** (**Å**) | **Height (Counts)** | **Rel. Intensity (%)** |
|---|---|---|---|
| 6.9 | 12.8 | 4569.7 | 13.0 |
| 8.5 | 10.4 | 1462.4 | 4.2 |
| 10.1 | 8.7 | 796.6 | 2.3 |
| 11.6 | 7.6 | 13570.5 | 38.5 |
| 12.6 | 7.0 | 631.6 | 1.8 |
| 14.6 | 6.1 | 1336.8 | 3.8 |
| 14.7 | 6.0 | 1391.1 | 4.0 |
| 15.4 | 5.8 | 3872.7 | 11.0 |
| 16.7 | 5.3 | 3218.9 | 9.1 |
| 16.9 | 5.2 | 526.2 | 1.5 |
| 18.5 | 4.8 | 8810.6 | 25.0 |
| 18.9 | 4.7 | 3519.3 | 10.0 |
| 19.1 | 4.6 | 823.6 | 2.3 |
| 19.4 | 4.6 | 258.0 | 0.7 |
| 20.3 | 4.4 | 35229.3 | 100.0 |
| 20.6 | 4.3 | 442.9 | 1.3 |
| 21.1 | 4.2 | 11928.3 | 33.9 |
| 21.3 | 4.2 | 8730.1 | 24.8 |
| 21.7 | 4.1 | 6250.9 | 17.7 |
| 22.1 | 4.0 | 1805.9 | 5.1 |
| 23.2 | 3.8 | 2572.0 | 7.3 |
| 23.3 | 3.8 | 2066.8 | 5.9 |
| 23.8 | 3.7 | 4070.9 | 11.6 |
| 23.9 | 3.7 | 4369.4 | 12.4 |
| 24.5 | 3.6 | 2569.7 | 7.3 |
| 24.6 | 3.6 | 983.7 | 2.8 |
| 25.4 | 3.5 | 93.0 | 0.3 |
| 26.1 | 3.4 | 1376.5 | 3.9 |
| 26.4 | 3.4 | 384.1 | 1.1 |
| 27.1 | 3.3 | 453.7 | 1.3 |
| 27.6 | 3.2 | 3268.4 | 9.3 |
| 27.9 | 3.2 | 995.8 | 2.8 |
| 28.9 | 3.1 | 899.9 | 2.6 |
| 29.5 | 3.0 | 1543.0 | 4.4 |
| 29.9 | 3.0 | 186.9 | 0.5 |

### Reference Example B.

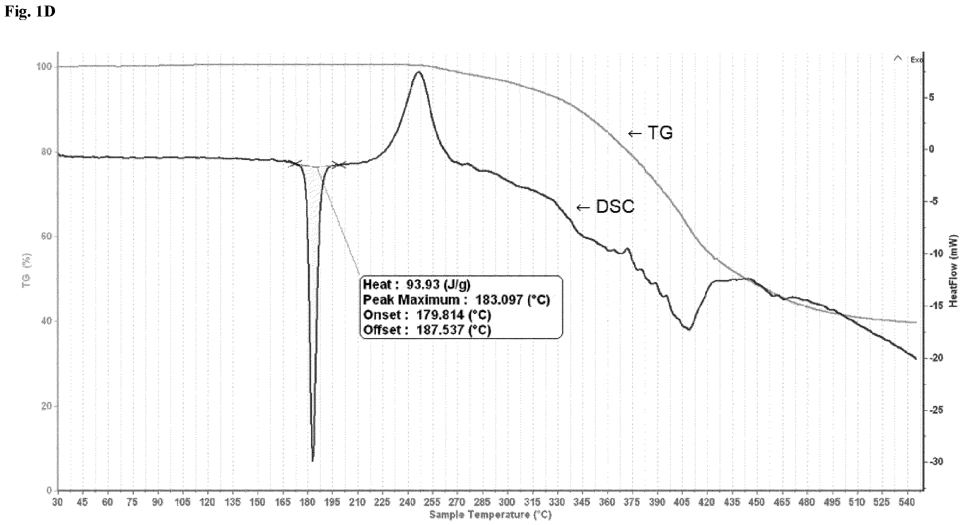

The XRPD analysis and TG/DSC analysis of Reference B were also performed. **Fig. 1C** shows an XRPD diffractogram of Reference B, and **Table 2** lists XRPD peaks represented in **Fig. 1C****.** **Fig. 1D** shows an overlay of a TG thermogram of Reference B and a DSC thermogram of Reference B with one endothermic event (peak maximum at about 183 °C) and one exothermic event.

XRPD analysis for **Fig. 1C** and **Table 2** was carried out using a Bruker D8 Discover diffractometer with DAVINCI configuration, in transmission mode (scan type: TwoTheta or Offset Coupled TwoTheta/Theta) scanning about 5 mg of Reference B at between 1.5 and 45° 2θ angles, and using the following measurements characteristics: acquisition time was 53 minutes, increment per step was 0.01°, time per step was 0.7 s, and generator voltage / generator amperage was 40 mA / 40 kV to reach 1.6 kW power. The raw data was imported in the Diffrac.EVA5.0 software and it was processed using the subsequent parameters: background subtraction and Kα2 stripping were performed before peak determination, and the peak search operation was performed with a threshold of 1 and a peak width of 0.153 for the sample. Only the resulting peaks having relative intensity of greater than 2% were considered. The degree of crystallinity was calculated using the Diffrac.EVA5.0 software option. The degree of crystallinity of Reference B was 85.1 % with 14.9 % amorphous phase.

**Table 2. XRPD Data for Reference B**

| **Angle 2θ (°)** | **Net Intensity (counts)** | **Rel. Intensity (%)** |
|---|---|---|
| 6.67 | 287.05 | 6.10 |
| 6.92 | 656.31 | 13.95 |
| 8.26 | 113.50 | 2.41 |
| 8.57 | 380.56 | 8.09 |
| 10.17 | 128.57 | 2.73 |
| 11.22 | 258.56 | 5.50 |
| 11.71 | 1718.23 | 36.53 |
| 14.73 | 328.32 | 6.98 |
| 15.44 | 662.52 | 14.09 |
| 16.76 | 797.82 | 16.96 |
| 16.94 | 125.17 | 2.66 |
| 17.80 | 127.99 | 2.72 |
| 18.14 | 149.55 | 3.18 |
| 18.59 | 1108.99 | 23.58 |
| 18.94 | 1423.19 | 30.26 |
| 19.22 | 267.97 | 5.70 |
| 19.54 | 577.37 | 12.28 |
| 20.40 | 4703.14 | 100.00 |
| 20.66 | 373.47 | 7.94 |
| 21.15 | 1970.68 | 41.90 |

| **Angle 2θ (°)** | **Net Intensity (counts)** | **Rel. Intensity (%)** |
|---|---|---|
| 21.42 | 3983.23 | 84.69 |
| 21.76 | 877.82 | 18.66 |
| 22.14 | 575.15 | 12.23 |
| 22.33 | 148.16 | 3.15 |
| 22.91 | 124.08 | 2.64 |
| 23.33 | 750.03 | 15.95 |
| 23.93 | 1355.51 | 28.82 |
| 24.58 | 488.33 | 10.38 |
| 24.70 | 303.72 | 6.46 |
| 26.15 | 225.95 | 4.80 |
| 26.53 | 163.51 | 3.48 |
| 27.19 | 185.76 | 3.95 |
| 27.69 | 797.21 | 16.95 |
| 27.99 | 224.00 | 4.76 |
| 29.07 | 126.74 | 2.69 |
| 29.57 | 195.09 | 4.15 |
| 31.27 | 167.71 | 3.57 |
| 31.99 | 228.62 | 4.86 |
| 32.39 | 331.96 | 7.06 |
| 32.64 | 259.17 | 5.51 |
| 33.23 | 140.20 | 2.98 |
| 33.41 | 210.77 | 4.48 |
| 34.31 | 97.99 | 2.08 |
| 37.97 | 102.05 | 2.17 |
| 38.53 | 94.74 | 2.01 |
| 40.43 | 196.96 | 4.19 |

### Example 1.

*Procedure A:* Phentolamine mesylate (3.45 g, 1 equiv.) prepared according to Example 2 of U.S. Patent No. 11,566,005 was added to a 100 mL reaction vessel having a torispherical bottom and equipped mechanical stirring. The reaction vessel was charged with acetone (37 mL), water (3.2 mL), and methane sulfonic acid (0.055 ml, 0.1 equiv.) and allowed to stir at 25°C for 0.5 h. The resultant reaction mixture was a purple suspension. The reaction vessel was heated to 45 °C and allowed to stir for 0.5 h at 45 °C. The reaction mixture became a solution. The reaction vessel was cooled to 25 °C over 20 minutes. The reaction mixture remained a solution. t-Butyl methyl ether (TBME, 34.5 mL) was added to the reaction vessel, and a white solid precipitated. The reaction vessel was cooled to 0 °C at 1.33 °C/min and dark droplets were observed. The reaction mixture was stirred for 1 h at 0 °C. The reaction vessel was further cooled to -20 °C at 1 °C/min then allowed to stir for 3 hours at -20 °C.

The reaction mixture was filtered under vacuum, and the resultant filter cake, off-white solid with light purple tinge, was washed with TBME (2 × 10 mL) and air dried under vacuum. The resultant solid was further dried in in a rotary evaporator at 40 °C for 48 h. The further dried phentolamine mesylate was analyzed by XRPD and DSC.

**Fig. 2A** shows an XRPD diffractogram of the phentolamine mesylate obtained according to this Example 1, and **Table 3** lists XRPD peaks represented in **Fig. 2A****.**

**Table 3. XRPD Data for Phentolamine Mesylate from this Example 1**

| **2-theta Angle (°)** | **d Value** (**Å**) | **Height (Counts)** | **Rel. Intensity (%)** |
|---|---|---|---|
| 8.4 | 10.5 | 13087.5 | 54.7 |
| 8.9 | 9.9 | 7267.0 | 30.4 |
| 10.3 | 8.6 | 734.9 | 3.1 |
| 10.9 | 8.1 | 1534.1 | 6.4 |
| 11.1 | 7.9 | 2538.4 | 10.6 |
| 11.5 | 7.7 | 3563.9 | 14.9 |
| 13.1 | 6.7 | 23928.7 | 100.0 |
| 15.8 | 5.6 | 1622.0 | 6.8 |
| 16.3 | 5.4 | 5164.0 | 21.6 |
| 16.9 | 5.3 | 8619.7 | 36.0 |
| 18.4 | 4.8 | 9063.7 | 37.9 |
| 18.8 | 4.7 | 14551.2 | 60.8 |
| 19.2 | 4.6 | 5768.1 | 24.1 |
| 19.4 | 4.6 | 17489.2 | 73.1 |
| 20.0 | 4.4 | 14398.7 | 60.2 |
| 20.5 | 4.3 | 4527.6 | 18.9 |
| 20.8 | 4.3 | 19619.5 | 82.0 |
| 21.1 | 4.2 | 1468.2 | 6.1 |
| 21.3 | 4.2 | 1041.1 | 4.4 |
| 21.5 | 4.1 | 1356.4 | 5.7 |
| 21.9 | 4.0 | 2909.9 | 12.2 |
| 22.2 | 4.0 | 7680.3 | 32.1 |
| 22.9 | 3.9 | 1817.8 | 7.6 |
| 23.2 | 3.8 | 2789.5 | 11.7 |
| 23.5 | 3.8 | 2324.4 | 9.7 |
| 24.0 | 3.7 | 4321.5 | 18.1 |
| 24.3 | 3.7 | 2614.2 | 10.9 |
| 24.9 | 3.6 | 329.8 | 1.4 |
| 25.4 | 3.5 | 3062.8 | 12.8 |
| 26.3 | 3.4 | 2586.7 | 10.8 |
| 27.0 | 3.3 | 4128.9 | 17.3 |
| 27.4 | 3.3 | 2272.9 | 9.5 |
| 28.3 | 3.2 | 484.2 | 2.0 |
| 28.5 | 3.1 | 2800.0 | 11.7 |
| 28.7 | 3.1 | 1827.4 | 7.6 |
| 29.3 | 3.0 | 1772.2 | 7.4 |

| **2-theta Angle (°)** | **d Value** (**Å**) | **Height (Counts)** | **Rel. Intensity (%)** |
|---|---|---|---|
| 30.4 | 2.9 | 2102.5 | 8.8 |
| 31.2 | 2.9 | 709.6 | 3.0 |
| 31.5 | 2.8 | 1127.5 | 4.7 |
| 31.9 | 2.8 | 1198.1 | 5.0 |
| 32.1 | 2.8 | 1592.9 | 6.7 |
| 35.1 | 2.6 | 2940.6 | 12.3 |
| 36.3 | 2.5 | 1266.5 | 5.3 |

The DSC analysis of the phentolamine mesylate obtained according to this Example 1 showed two large endothermic events: a first event which onset at about 113 °C (peak maximum at about 120 °C) and a second event which onset at about 130 °C (peak maximum at about 133 °C) **(****Fig. 2B****).** The DSC analysis also showed one small endothermic event which onset at about 179 °C (peak maximum at about 180 °C).

*Procedure B*: Phentolamine mesylate (20 mg) prepared according to Example 2 of U.S. Patent No. 11,566,005 was suspended in dichloromethane (800 µL). The suspension was subjected to heat-cool cycles between 25 °C and 50 °C (2 h at each temperature, with heating and cooling ramps of 1 °C/min) for about 3 days. The resultant reaction mixture was centrifuged at 4000 rpm at 25 °C for 10-15 minutes and the resultant supernatant was decanted. The residual solid was dried under vacuum at 25 °C for 16 h to provide an off-white/pink solid. The solid's XRPD pattern was consistent with the XRPD pattern depicted in **Fig. 2A****.**

*Procedure C*: Phentolamine mesylate (963 mg) prepared according to Example 2 of U.S. Patent No. 11,566,005 was dissolved in methyl ethyl ketone (MEK):water 96:4 w/w (6 ml) at 80°C. The solution was cooled to 45°C at 0.5 °C/min. Seeds of phentolamine mesylate obtained according to Procedure B of this Example 1 were added (approximately 2 mg) and the resultant mixture was cooled further to 10 °C and allowed to stir for 30 minutes. The resulting suspension was heated to 35 °C and vacuum was applied to remove approximately 1/3 of the volume. Then, the vacuum was removed and the suspension was cooled to 10 °C and allowed to stir for 40 minutes. The resultant solid was isolated by vacuum filtration, washed with cold MEK (0.5 ml) and dried for 16 h under vacuum at about 0.24 bar at ambient temperature to provide an off-white/pink solid (677 mg). The solid's XRPD pattern was consistent with the XRPD pattern depicted in **Fig. 2A****.**

*Procedure D*: Phentolamine mesylate (93.4 mg) prepared according to Example 2 of U.S. Patent No. 11,566,005 was suspended in MEK:water 96:4 w:w (497 mg) at room temperature. The mixture was subjected to three heat-cool cycles between 10 °C and 77°C using heating and cooling rates of 0.5 °C/min (with no holding time at 10 °C or 77 °C). At the end of the third cycle, the temperature was held at 10 °C for about 7 hours. The resultant solid was isolated by filtration to provide an off-white/pink solid. The solid's XRPD pattern was consistent with the XRPD pattern depicted in **Fig. 2A****.**

### Example 2.

Phentolamine mesylate (12.7 mg) obtained according to Example 1 above was heated to 126 °C at 10 °C/min and was allowed to cool to room temperature. The resultant phentolamine mesylate was analyzed by XRPD and DSC.

**Fig. 3A** shows an XRPD diffractogram of the phentolamine mesylate obtained by this Example 2, and **Table 4** lists XRPD peaks represented in **Fig. 3A****.** The XRPD diffractogram's peak at 6.9 degrees 2-theta and peak at 11.6 degrees 2-theta indicate a presence of a small amount of phentolamine mesylate having XRPD peaks of Reference A.

**Table 4. XRPD Data for Phentolamine Mesylate Obtained According to this Example 2**

| **2-theta Angle (°)** | **d Value (Å)** | **Height (Counts)** | **Rel. Intensity (%)** |
|---|---|---|---|
| 6.9 | 12.9 | 1140.7 | 9.9 |
| 8.3 | 10.6 | 11494.5 | 100.0 |
| 9.0 | 9.8 | 1139.3 | 9.9 |
| 10.3 | 8.6 | 334.1 | 2.9 |
| 11.3 | 7.8 | 1462.3 | 12.7 |
| 11.6 | 7.6 | 545.7 | 4.8 |
| 13.3 | 6.7 | 8206.1 | 71.4 |
| 13.8 | 6.4 | 276.2 | 2.4 |
| 14.7 | 6.0 | 353.2 | 3.1 |
| 15.4 | 5.8 | 243.4 | 2.1 |
| 15.7 | 5.6 | 1166.8 | 10.2 |
| 16.7 | 5.3 | 6842.4 | 59.5 |
| 17.1 | 5.2 | 644.3 | 5.6 |
| 18.5 | 4.8 | 739.8 | 6.4 |
| 18.8 | 4.7 | 4347.5 | 37.8 |
| 18.9 | 4.7 | 5387.5 | 46.9 |
| 19.1 | 4.6 | 1570.0 | 13.7 |
| 19.4 | 4.6 | 1421.0 | 12.4 |
| 19.7 | 4.5 | 6097.0 | 53.0 |
| 20.3 | 4.4 | 6631.7 | 57.7 |
| 21.0 | 4.2 | 2408.3 | 21.0 |
| 21.4 | 4.2 | 1303.7 | 11.3 |
| 22.0 | 4.0 | 2075.0 | 18.1 |
| 22.5 | 3.9 | 1615.1 | 14.1 |
| 23.0 | 3.9 | 877.9 | 7.6 |
| 23.5 | 3.8 | 1890.5 | 16.5 |
| 24.7 | 3.6 | 411.1 | 3.6 |
| 25.2 | 3.5 | 768.2 | 6.7 |
| 25.4 | 3.5 | 1037.7 | 9.0 |
| 25.9 | 3.4 | 491.2 | 4.3 |
| 27.3 | 3.3 | 1502.4 | 13.1 |
| 29.9 | 3.0 | 471.4 | 4.1 |
| 30.4 | 2.9 | 525.7 | 4.6 |

DSC analysis of the phentolamine mesylate obtained according to this Example 2 showed a small endothermic event having an onset at about 129 °C (peak maximum at about 133 °C) and a small exothermic event having an onset at about 135 °C (peak maximum at about 138 °C) **(****Fig. 3B****).** The DSC analysis also showed a sharp endothermic event having an onset at about 178 °C (peak maximum at about 180 °C).

### Example 3.

A suspension of Reference A (20 mg) in acetone (50 µL) was subjected to heat-cool cycles between 25 °C and 50 °C (2 h at each temperature, with heating and cooling ramps of 1 °C/min) for up to three days. The resultant reaction mixture was centrifuged at 4000 rpm at 25 °C for 10-15 minutes, and the resultant supernatant was decanted. The residual solid was taken with a spatula and analyzed wet by XRPD.

**Fig. 4A** shows an XRPD diffractogram of the phentolamine mesylate obtained according to this Example 3, and **Table 5** below lists XRPD peaks represented in **Fig. 4A****.**

**Table 5. XRPD Data for Phentolamine Mesylate Obtained According to Example 3**

| **2-theta Angle (°)** | **d Value (Å)** | **Height (Counts)** | **Rel. Intensity (%)** |
|---|---|---|---|
| 7.1 | 12.4 | 4741.1 | 54.3 |
| 8.9 | 10.0 | 2153.4 | 24.7 |
| 10.9 | 8.1 | 331.2 | 3.8 |
| 11.8 | 7.5 | 388.3 | 4.5 |
| 13.0 | 6.8 | 3116.0 | 35.7 |
| 14.3 | 6.2 | 740.7 | 8.5 |
| 14.7 | 6.0 | 626.7 | 7.2 |
| 15.1 | 5.9 | 954.4 | 10.9 |
| 16.2 | 5.5 | 598.4 | 6.9 |
| 16.5 | 5.4 | 628.2 | 7.2 |
| 17.1 | 5.2 | 489.1 | 5.6 |
| 17.8 | 5.0 | 1450.0 | 16.6 |
| 19.0 | 4.7 | 2307.1 | 26.4 |
| 19.9 | 4.5 | 8733.6 | 100.0 |

| **2-theta Angle (°)** | **d Value (Å)** | **Height (Counts)** | **Rel. Intensity (%)** |
|---|---|---|---|
| 20.2 | 4.4 | 1626.0 | 18.6 |
| 20.4 | 4.4 | 765.2 | 8.8 |
| 21.0 | 4.2 | 1854.5 | 21.2 |
| 21.4 | 4.1 | 743.3 | 8.5 |
| 21.9 | 4.1 | 2216.7 | 25.4 |
| 22.4 | 4.0 | 451.8 | 5.2 |
| 22.6 | 3.9 | 978.2 | 11.2 |
| 23.5 | 3.8 | 849.1 | 9.7 |
| 24.3 | 3.7 | 740.1 | 8.5 |
| 24.5 | 3.6 | 257.3 | 3.0 |
| 25.2 | 3.5 | 168.2 | 1.9 |
| 25.6 | 3.5 | 659.6 | 7.6 |
| 26.1 | 3.4 | 400.5 | 4.6 |
| 26.3 | 3.4 | 443.1 | 5.1 |
| 26.9 | 3.3 | 387.2 | 4.4 |
| 27.3 | 3.3 | 324.6 | 3.7 |
| 28.4 | 3.1 | 145.9 | 1.7 |
| 29.2 | 3.1 | 871.2 | 10.0 |
| 30.7 | 2.9 | 305.5 | 3.5 |
| 32.0 | 2.8 | 218.6 | 2.5 |
| 33.1 | 2.7 | 180.7 | 2.1 |
| 33.9 | 2.6 | 442.3 | 5.1 |
| 34.4 | 2.6 | 339.1 | 3.9 |
| 35.0 | 2.6 | 142.9 | 1.6 |
| 36.6 | 2.5 | 201.5 | 2.3 |

DSC analysis of the phentolamine mesylate obtained according to this Example 3 showed two endothermic events: a first event having an onset at about 99 °C (peak maximum at about 100 °C) and a second event having an onset at about 179 °C (peak maximum at about 181 °C) (**Fig. 4B**).

A mass loss of about 7% was observed on the TG curve, corresponding to the first endothermic event (**Fig. 4C**).

A ¹H-NMR spectrum of the phentolamine mesylate obtained according to this Example 3 was obtained (**Fig. 4D**).

Based on the TG/DSC and ¹H-NMR data, the phentolamine mesylate obtained according to this Example 3 is an acetone solvate.

### Example 4.

Reference A (50 mg) was suspended in MeCN (500 µl) and slurried at 5 °C for 1 week. The resultant solid was isolated after 1 week by vacuum filtration at 0.24 bar at 25 °C and analyzed by XRPD.

**Fig. 5A** shows an XRPD diffractogram of the phentolamine mesylate obtained according to this Example 4. DSC analysis of the phentolamine mesylate obtained according to this Example 4 showed multiple events (**Fig. 5B**): a broad endothermic event having an onset at about 41 °C (peak maximum at about 52 °C); a broad endothermic event having an onset at about 91 °C (peak maximum at about 99 °C); an endothermic event having an onset at about 110 °C (peak maximum at about 114 °C); an exothermic event having an onset at about 129°C (peak maximum at about 133 °C); and a sharp endothermic event having an onset at about 178 °C (peak maximum at about 180 °C).

A mass loss of about 3% and a mass loss of about 5% was observed according to the TG curve (**Fig. 5C**).

The phentolamine mesylate obtained according to this Example 4 was further dried at 0.24 bar and 25 °C for 16 hours. The XRPD analysis of the further dried material indicated that the phentolamine mesylate form exhibiting XRPD pattern of **Fig. 5A****,** upon further drying, converted to a mixture of the phentolamine mesylate having XRPD peaks of Reference A and the phentolamine mesylate obtained according to Example 1.

### Example 5.

Reference A (20 mg) was placed in a 2 mL HPLC vial equipped with 3 stainless steel 3 mm diameter balls. 1,4-Dioxane (20 µL) was added to the vial at room temperature and the phentolamine mesylate/1,4-dioxane mixture was ground using a Fritsch Pulverisette planetary mill at 400 rpm for 3 h. The resultant solid was dried under vacuum at 0.24 bar for 16 h and analyzed by XRPD.

**Fig. 6A** shows an XRPD diffractogram of the phentolamine mesylate obtained according to this Example 5, and **Table 6** below lists XRPD peaks represented in **Fig. 6A****.**

**Table 6. XRPD Data for Phentolamine Mesylate Obtained According to Example 5**

| **2-theta Angle (°)** | **d Value (Å)** | **Height (Counts**) | **Rel. Intensity (%)** |
|---|---|---|---|
| 7.4 | 12.0 | 16829.9 | 40.1 |
| 9.2 | 9.6 | 7349.4 | 17.5 |
| 10.8 | 8.2 | 5357.7 | 12.8 |
| 11.3 | 7.8 | 577.3 | 1.4 |
| 12.7 | 7.0 | 1174.1 | 2.8 |
| 13.1 | 6.8 | 14276.7 | 34.0 |
| 14.7 | 6.0 | 6144.0 | 14.6 |
| 14.8 | 6.0 | 3609.2 | 8.6 |
| 15.8 | 5.6 | 5730.2 | 13.7 |
| 16.1 | 5.5 | 1909.1 | 4.6 |
| 16.6 | 5.3 | 782.1 | 1.9 |

| **2-theta Angle (°)** | **d Value (Å)** | **Height (Counts)** | **Rel. Intensity (%)** |
|---|---|---|---|
| 17.5 | 5.1 | 1751.3 | 4.2 |
| 18.4 | 4.8 | 11291.1 | 26.9 |
| 18.7 | 4.7 | 4141.9 | 9.9 |
| 19.5 | 4.6 | 6051.5 | 14.4 |
| 19.8 | 4.5 | 41962.1 | 100.0 |
| 20.2 | 4.4 | 1880.3 | 4.5 |
| 20.7 | 4.3 | 14457.0 | 34.5 |
| 20.9 | 4.3 | 33417.6 | 79.6 |
| 21.2 | 4.2 | 3378.1 | 8.1 |
| 21.7 | 4.1 | 2336.7 | 5.6 |
| 21.9 | 4.1 | 3102.7 | 7.4 |
| 22.2 | 4.0 | 2030.7 | 4.8 |
| 22.7 | 3.9 | 4311.6 | 10.3 |
| 23.0 | 3.9 | 1937.4 | 4.6 |
| 23.3 | 3.8 | 2502.8 | 6.0 |
| 24.2 | 3.7 | 804.3 | 1.9 |
| 24.6 | 3.6 | 3357.1 | 8.0 |
| 24.9 | 3.6 | 377.2 | 0.9 |
| 25.4 | 3.5 | 518.7 | 1.2 |
| 25.8 | 3.5 | 2029.5 | 4.8 |
| 26.3 | 3.4 | 1766.4 | 4.2 |
| 26.6 | 3.4 | 1271.4 | 3.0 |
| 26.8 | 3.3 | 1551.2 | 3.7 |
| 27.2 | 3.3 | 2051.6 | 4.9 |
| 27.4 | 3.3 | 1193.4 | 2.8 |
| 27.6 | 3.2 | 548.3 | 1.3 |
| 28.8 | 3.1 | 2364.3 | 5.6 |
| 28.9 | 3.1 | 2656.7 | 6.3 |
| 29.1 | 3.1 | 1499.2 | 3.6 |
| 29.4 | 3.0 | 770.9 | 1.8 |
| 29.8 | 3.0 | 1082.0 | 2.6 |
| 30.3 | 2.9 | 2090.0 | 5.0 |
| 30.6 | 2.9 | 1585.7 | 3.8 |

DSC analysis of the phentolamine mesylate obtained according to this Example 5 showed one endothermic event having an onset at about 140 °C (peak maximum at about 143 °C) (**Fig. 6B**).

A mass loss of about 9.5% was observed according to the TG curve, corresponding to the endothermic event (**Fig. 6C**).

A ¹H-NMR spectrum of the phentolamine mesylate obtained according to this Example 5 was obtained (**Fig. 6D**).

Based on the TG/DSC and ¹H-NMR data, the phentolamine mesylate obtained according to this Example 5 is a 1,4-dioxane solvate.

### Example 6.

Reference A (20 mg) was suspended in THF (100 µl) and slurried at 40 °C for 24 h. The resultant solid was centrifuged at 4000 rpm at 25 °C for 10-15 minutes, and the resultant supernatant was decanted. The residual solid was taken with a spatula and analyzed wet by XRPD.

**Fig. 7** shows an XRPD diffractogram of the phentolamine mesylate obtained according to this Example 6.

The phentolamine mesylate obtained according to this Example 6 was further dried at 25°C at 0.24 bar for 16 hours. The XRPD analysis of the further dried material indicated that the phentolamine mesylate form exhibiting XRPD pattern of **Fig. 7****,** upon further drying, converted to the phentolamine mesylate obtained according to Example 1.

### Example 7.

Reference A (50 mg) was suspended in THF (500 µl) and slurried at 25 °C for 1 week. The resultant solid was centrifuged at 4000 rpm at 25 °C for 10-15 minutes, and the supernatant was decanted. The solid was analyzed wet by XRPD.

**Fig. 8** shows an overlay of XRPD pattern of the phentolamine mesylate obtained according to this Example 7 (top) with an XRPD pattern of the phentolamine mesylate obtained according to Example 6 (bottom). **Fig. 8** indicates that the phentolamine mesylate obtained according to this Example 7 is a mixture that includes the phentolamine mesylate obtained according to Example 6.

The phentolamine mesylate obtained according to this Example 7 was further dried at 0.24 bars at 25 °C for 16 hours. The XRPD analysis of the further dried material indicated that the mixture converted to the phentolamine mesylate obtained according to Example 1 above.

### Example 8.

Reference A (20 mg) was dissolved in 2 vol of MeOH. Aliquots of isobutyl acetate (100 µL) were added until a suspension was obtained. The resultant solid was centrifuged at 4000 rpm at 25 °C for 10-15 minutes, and the supernatant was decanted. The residual wet solid was taken with a spatula and analyzed wet by XRPD.

**Fig. 9** shows an XRPD diffractogram of the phentolamine mesylate obtained according to this Example 8.

### Example 9.

Reference A (20 mg) was placed in an open, uncapped 2 mL HPLC vial, which was subsequently placed into a 20 mL vial containing 1 mL of dichloromethane. The 20 mL vial was capped and stored at room temperature for 2 weeks while the Reference A in the smaller vial was exposed to dichloromethane vapor. The resultant solid was collected and analyzed wet by XRPD.

**Fig. 10** shows an overlay of an XRPD pattern of the phentolamine mesylate obtained according to this Example 9 (bottom) with an XRPD pattern of the phentolamine mesylate obtained according to Example 1 above (top). **Fig. 10** indicates that the phentolamine mesylate obtained according to this Example 9 is a mixture that includes the phentolamine mesylate obtained according to Example 1 above.

### Example 10.

Reference A (20 mg) was placed into an open, uncapped 2 mL HPLC vial, which was subsequently placed into a 20 mL vial containing 1 mL of chloroform. The 20 mL vial was capped and stored at room temperature for 2 weeks while the Reference A in the smaller vial was exposed to chloroform vapor. The resultant solid was collected and analyzed wet by XRPD.

**Fig. 11** shows an XRPD diffractogram of the phentolamine mesylate obtained according to this Example 10.

### Example 11.

Reference A (20 mg) was dissolved in 2 vol of MeOH. Aliquots of ethyl acetate (100 µL) were added until a suspension was obtained. The resultant solid was centrifuged at 4000 rpm at 25 °C for 10-15 minutes, and the resultant supernatant was decanted. The residual solid was taken with a spatula and analyzed wet by XRPD.

**Fig. 12** shows an XRPD diffractogram of the phentolamine mesylate obtained according to this Example 11.

The phentolamine mesylate obtained according to this Example 11 was further dried at 25°C and 0.24 bar for 16 hours. The XRPD analysis of the further dried material indicated that the phentolamine mesylate obtained according to this Example 11 converted to the phentolamine mesylate obtained according to Example 1 above.

### Example 12.

Reference A (20 mg) was dissolved in 2 vol of DMSO. Aliquots of methyl ethyl ketone (100 µL) were added until a suspension was obtained. The resultant solid was centrifuged at 4000 rpm at 25 °C for 10-15 minutes, and the resultant supernatant was decanted. The residual solid was taken with a spatula and analyzed wet by XRPD.

**Fig. 13** shows an XRPD diffractogram of the phentolamine mesylate obtained according to this Example 12.

The phentolamine mesylate obtained according to this Example 12 was further dried at 25°C and 0.24 bar for 16 hours. The XRPD analysis of the further dried material indicated that the phentolamine mesylate obtained according to this Example 12 converted to the phentolamine mesylate obtained according to Example 1 above.

## Claims

1. Phentolamine mesylate that exhibits an X-ray powder diffraction (XRPD) pattern comprising a peak at 8.4 ± 0.2 degrees 2-theta and a peak at 16.3 ± 0.2 degrees 2-theta.

2. The phentolamine mesylate of claim 1, wherein the XRPD pattern further comprises a peak at 8.4 ± 0.2 degrees 2-theta, a peak at 19.4 ± 0.2 degrees 2-theta, or a peak at 20.8 ± 0.2 degrees 2-theta.

3. The phentolamine mesylate of claim 1 or 2, wherein the XRPD pattern further comprises a peak at 18.8 ± 0.2 degrees 2-theta or a peak at 20.0 ± 0.2 degrees 2-theta.

4. Phentolamine mesylate that exhibits an XRPD pattern that is substantially the same as that depicted in **Fig. 2A.**

5. Phentolamine mesylate that exhibits an XRPD pattern comprising the peaks of **Table 3** having at least 50% relative intensity.

6. Phentolamine mesylate that exhibits an X-ray powder diffraction (XRPD) pattern comprising a peak at 8.3 ± 0.2 degrees 2-theta, a peak at 13.3 ± 0.2 degrees 2-theta and a peak at 19.7 ± 0.2 degrees 2-theta.

7. The phentolamine mesylate of claim 6, wherein the XRPD pattern further comprises a peak at 16.7 ± 0.2 degrees 2-theta or a peak at 20.3 ± 0.2 degrees 2-theta.

8. The phentolamine mesylate of claim 6 or 7, wherein the XRPD pattern further comprises a peak at 18.8 ± 0.2 degrees 2-theta.

9. Phentolamine mesylate that exhibits an XRPD pattern that is substantially the same as that depicted in **Fig. 3A.**

10. Phentolamine mesylate that exhibits an XRPD pattern comprising the peaks of **Table 4** having at least 50% relative intensity.

11. The phentolamine mesylate of any one of claims 1-10 comprising less than 5% by weight of phentolamine mesylate that exhibits an XRPD pattern that is substantially the same as that depicted in **Fig. 1A** or **Fig. 1C.**

12. The phentolamine mesylate of any one of claims 1-10 comprising less than 3% by weight of phentolamine mesylate that exhibits an XRPD pattern that is substantially the same as that depicted in **Fig. 1A** or **Fig. 1C.**

13. The phentolamine mesylate of any one of claims 1-10 comprising less than 1% by weight of phentolamine mesylate that exhibits an XRPD pattern that is substantially the same as that depicted in **Fig. 1A** or **Fig. 1C.**

14. The phentolamine mesylate of any one of claims 1-10, wherein the phentolamine mesylate does not comprising phentolamine mesylate that exhibits an XRPD pattern that is substantially the same as that depicted in **Fig. 1A** or **Fig. 1C.**

15. The phentolamine mesylate of any one of claims 1-14, wherein the phentolamine mesylate is purified and isolated.

16. The phentolamine mesylate of any one of claims 1-15, wherein the phentolamine mesylate comprises less than 2% of an impurity by weight of the phentolamine mesylate.

17. The phentolamine mesylate of any one of claims 1-15, wherein the phentolamine mesylate comprises less than 1% of an impurity by weight of the phentolamine mesylate.

18. The phentolamine mesylate of any one of claims 1-15, wherein the phentolamine mesylate comprises less than 0.5% of an impurity by weight of the phentolamine mesylate.

19. A composition comprising the phentolamine mesylate of any one of claims 1-18, and a pharmaceutically acceptable carrier or excipient.

20. The composition of claim 19, wherein the composition is in the form of a solution, a suspension, an emulsion, a tablet, a capsule, a powder, a cream, or a gel.

21. The composition of claim 19, wherein the composition is an ophthalmic solution, or the composition is coated on or incorporated in an ophthalmic drug delivery device.

22. The composition of any one of claims 19-21, wherein the composition comprises the phentolamine mesylate in an amount of about 0.25% to about 3% by weight or volume of the composition.

23. The composition of any one of claims 19-21, wherein the composition comprises the phentolamine mesylate in an amount of about 0.5% to about 2% by weight or volume of the composition.

24. The composition of any one of claims 19-21, wherein the composition comprises the phentolamine mesylate in an amount of about 1% by weight of the composition.

25. The composition of any one of claims 19-21, wherein the composition comprises the phentolamine mesylate in an amount of about 1% by volume of the composition.

26. The composition of any one of claims 19-21, wherein the composition comprises the phentolamine mesylate in an amount of about 0.5% by weight of the composition.

27. The composition of any one of claims 19-21, wherein the composition comprises the phentolamine mesylate in an amount of about 0.5% by volume of the composition.

28. A composition comprising a pharmaceutically acceptable aqueous carrier or vehicle in which the phentolamine mesylate of any one of claims 1-18 is dissolved.

29. The composition of claim 28, wherein phentolamine mesylate is dissolved in an amount of about 0.25% to about 3% by weight or volume of the composition.

30. The composition of any one of claim 29, wherein phentolamine mesylate is dissolved in an amount of about 0.5% to about 2% by weight or volume of the composition.

31. The composition of any one of claims 30, wherein phentolamine mesylate is dissolved in an amount of about 1% by weight of the composition.

32. The composition of any one of claims 30, wherein phentolamine mesylate is dissolved in an amount of about 1% by volume of the composition.

33. The composition of any one of claims 30, wherein phentolamine mesylate is dissolved in an amount of about 0.5% by weight of the composition.

34. The composition of any one of claims 30, wherein phentolamine mesylate is dissolved in an amount of about 0.5% by volume of the composition.

35. A sealed container containing the phentolamine mesylate of any one of claims 1-18 and an inert gas.

36. The sealed container of claim 35, wherein the inert gas is argon or nitrogen.

37. A method for treating or reversing pharmacologically induced mydriasis, comprising administering to a subject in need thereof an effective amount of the phentolamine mesylate of any one of claims 1-18 or the composition of any one of claims 19-34.

38. The method of claim 37, wherein the pharmacologically induced mydriasis is produced by an adrenergic agonist or a parasympatholytic agent.

39. The method of claim 38, wherein the pharmacologically induced mydriasis is produced by an adrenergic agonist, and the adrenergic agonist is phenylephrine.

40. The method of claim 38, wherein the pharmacologically induced mydriasis is produced by a parasympatholytic agent, and the parasympatholytic agent is tropicamide.

41. A method for inhibiting contraction of smooth muscle of a subject's iris, comprising administering to a subject in need thereof an effective amount of the phentolamine mesylate of any one of claims 1-18 or the composition of any one of claims 19-34.

42. A method for reducing a subject's pupil diameter, comprising administering to a subject in need thereof an effective amount of the phentolamine mesylate of any one of claims 1-18 or the composition of any one of claims 19-34.

43. A method for improving visual contrast sensitivity or visual acuity, comprising administering to a subject in need thereof an effective amount of the phentolamine mesylate of any one of claims 1-18 or the composition of any one of claims 19-34.

44. A method for treating or preventing an ocular disease or condition, comprising administering to a subject in need thereof an effective amount of the phentolamine mesylate of any one of claims 1-18 or the composition of any one of claims 19-34.

45. The method of claim 44, wherein the ocular disease or condition is a dim or night vision disturbance.

46. The method of claim 44, wherein the ocular disease or condition is presbyopia.

47. The method of claim 44, wherein the ocular disease or condition is diabetic retinal disease.

48. The method of claim 44 or 47, wherein the ocular disease or condition is diabetic retinopathy (DR) or diabetic macular edema (DME).

49. The method of claim 48, wherein the ocular disease or condition is DR, and the DR is moderately severe non-proliferative DR or mild proliferative DR.

50. The method of claim 48, wherein the ocular disease or condition is DME, and the DME is DME without loss of central vision.

51. The method of claim 44, wherein the ocular disease or condition is retinopathy of prematurity, diabetic retinopathy, pathological myopia, hypertensive retinopathy, occlusive vasculitis, polypoidal choroidal vasculopathy, diabetic macular edema, uveitic macular edema, retinal vein occlusion, ocular neovascularization, ocular histoplasmosis, neovascular glaucoma, retinoblastoma, macular degeneration, retrolental fibroplasias, retinal angiomatous proliferation, dry eye disease, uveitis, thyroid eye disease, or sickle cell retinopathy.

52. The method of claim 51, wherein the ocular disease or condition is diabetic retinopathy, and the diabetic retinopathy is proliferative diabetic retinopathy.

53. The method of claim 51, wherein the ocular disease or condition is macular degeneration, and the macular degeneration is advanced macular degeneration.

54. The method of claim 51, wherein the ocular disease or condition is macular degeneration, and the macular degeneration is wet age-related macular degeneration or dry age-related macular degeneration.

55. The method of claim 51, wherein the ocular disease or condition is ocular neovascularization, and the ocular neovascularization is corneal neovascularization or retinal neovascularization.

56. The method of claim 51, wherein the ocular disease or condition is retinal vein occlusion, and the retinal vein occlusion is central retinal vein occlusion or branch retinal vein occlusion.

57. The method of claim 44, wherein the ocular disease or condition is geographic atrophy, choroidal neovascularization, or corneal graft rejection.

58. A method for treating or preventing an ocular disease or condition, comprising administering to a subject in need thereof an effective amount of:
phentolamine mesylate that exhibits an XRPD pattern that is substantially the same as that depicted in **Fig. 1A** or **Fig. 1C;**
a composition comprising (i) phentolamine mesylate that exhibits an XRPD pattern that is substantially the same as that depicted in **Fig. 1A** or **Fig. 1C** and (ii) a pharmaceutically acceptable carrier or vehicle; or
a composition comprising a pharmaceutically acceptable aqueous carrier or vehicle in which phentolamine mesylate that exhibits an XRPD pattern that is substantially the same as that depicted in **Fig. 1A** or **Fig. 1C** is dissolved,
wherein the ocular disease or condition is diabetic retinal disease, retinopathy of prematurity, pathological myopia, hypertensive retinopathy, occlusive vasculitis, polypoidal choroidal vasculopathy, diabetic macular edema, uveitic macular edema, retinal vein occlusion, ocular neovascularization, ocular histoplasmosis, neovascular glaucoma, retinoblastoma, macular degeneration, retrolental fibroplasias, retinal angiomatous proliferation, dry eye disease, uveitis, thyroid eye disease, sickle cell retinopathy, geographic atrophy, choroidal neovascularization, or corneal graft rejection.

59. The method of claim 58, wherein the ocular disease or condition is diabetic retinal disease the diabetic retinal disease is diabetic retinopathy (DR) or diabetic macular edema (DME).

60. The method of claim 59, wherein the DR is moderately severe non-proliferative DR or mild proliferative DR.

61. The method of claim 59, wherein the DR is proliferative diabetic retinopathy.

62. The method of claim 59, wherein the DME is DME without loss of central vision.

63. The method of claim 58, wherein the ocular disease or condition is macular degeneration, and the macular degeneration is advanced macular degeneration.

64. The method of claim 58, wherein the ocular disease or condition is macular degeneration, and the macular degeneration is wet age-related macular degeneration or dry age-related macular degeneration.

65. The method of any one of claims 37-64, wherein the administering to is topically instilling into the subject's eye.

66. The method of any one of claims 37-65, wherein the composition is an ophthalmic solution.

67. The method of claim 66, wherein the ophthalmic solution is suitable for topical, subconjunctival, intravitreal, retrobulbar, intracameral or systemic administration.

68. The method of any one of claims 37-65, wherein the composition is an ophthalmic solution, or the composition is coated on or incorporated in an ophthalmic drug delivery device.

69. The method of any one of claims 37-68, comprising dissolving the phentolamine mesylate in a pharmaceutically acceptable aqueous carrier or vehicle to provide the composition in which the phentolamine mesylate is dissolved.

70. The method of any one of claims 58-68, comprising dissolving the phentolamine mesylate that exhibits an XRPD pattern that is substantially the same as that depicted in **Fig. 1A** or **Fig. 1C** in a pharmaceutically acceptable aqueous carrier or vehicle to provide the composition in which the phentolamine mesylate that exhibits an XRPD pattern that is substantially the same as that depicted in **Fig. 1A** or **Fig. 1C** is dissolved.

71. A method for treating or reversing pharmacologically induced mydriasis, comprising dissolving the phentolamine mesylate of any one of claims 1-18 in a pharmaceutically acceptable aqueous carrier or vehicle to provide an ophthalmic solution and administering to a subject in need of treatment or reversal of pharmacologically induced mydriasis an effective amount of the ophthalmic solution.

72. A method for treating or reversing pharmacologically induced mydriasis, comprising administering to a subject in need thereof an effective amount of an ophthalmic solution in which the phentolamine mesylate of any one of claims 1-18 is dissolved.

73. The method of claim 71 or 72, wherein the pharmacologically induced mydriasis is produced by an adrenergic agonist or a parasympatholytic agent.

74. The method of claim 73, wherein the pharmacologically induced mydriasis is produced by an adrenergic agonist, and the adrenergic agonist is phenylephrine.

75. The method of claim 73, wherein the pharmacologically induced mydriasis is produced by a parasympatholytic agent, and the parasympatholytic agent is tropicamide.

76. A method for treating or reversing pharmacologically induced mydriasis, comprising dissolving phentolamine mesylate that exhibits an X-ray powder diffraction (XRPD) pattern comprising a peak at 8.4 ± 0.2 degrees 2-theta and a peak at 16.3 ± 0.2 degrees 2-theta in a pharmaceutically acceptable aqueous carrier or vehicle to provide an ophthalmic solution and administering to a subject in need of treatment or reversal of pharmacologically induced mydriasis an effective amount of the ophthalmic solution.

77. A method for treating or reversing pharmacologically induced mydriasis, comprising administering to a subject in need thereof an effective amount of an ophthalmic solution in which phentolamine mesylate that exhibits an X-ray powder diffraction (XRPD) pattern comprising a peak at 8.4 ± 0.2 degrees 2-theta and a peak at 16.3 ± 0.2 degrees 2-theta is dissolved.

78. A method for treating or reversing pharmacologically induced mydriasis, comprising dissolving phentolamine mesylate that exhibits an X-ray powder diffraction (XRPD) pattern comprising a peak at 8.3 ± 0.2 degrees 2-theta, a peak at 13.3 ± 0.2 degrees 2-theta and a peak at 19.7 ± 0.2 degrees 2-theta in a pharmaceutically acceptable aqueous carrier or vehicle to provide an ophthalmic solution and administering to a subject in need of treatment or reversal of pharmacologically induced mydriasis an effective amount of the ophthalmic solution.

79. A method for treating or reversing pharmacologically induced mydriasis, comprising administering to a subject in need thereof an effective amount of an ophthalmic solution in which phentolamine mesylate that exhibits an X-ray powder diffraction (XRPD) pattern comprising a peak at 8.3 ± 0.2 degrees 2-theta, a peak at 13.3 ± 0.2 degrees 2-theta and a peak at 19.7 ± 0.2 degrees 2-theta is dissolved.

80. The method of any one of claims 76-79, wherein the pharmacologically induced mydriasis is produced by an adrenergic agonist or a parasympatholytic agent.

81. The method of claim 80, wherein the pharmacologically induced mydriasis is produced by an adrenergic agonist, and the adrenergic agonist is phenylephrine.

82. The method of claim 80, wherein the pharmacologically induced mydriasis is produced by a parasympatholytic agent, and the parasympatholytic agent is tropicamide.

83. A method for making phentolamine mesylate of claim 1, comprising:
(a) heating a mixture comprising phentolamine mesylate comprising an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta; methane sulfonic acid; acetone; and water at about 45 °C for about thirty minutes to provide a heated mixture;
(b) allowing the heated mixture to cool to room temperature to provide a cooled mixture;
(c) diluting the cooled mixture with t-butyl methyl ether to provide a t-butyl methyl ether mixture; and
(d) cooling the t-butyl methyl ether mixture to about -20 °C to precipitate the phentolamine mesylate that exhibits an XRPD pattern comprising a peak at 8.4 ± 0.2 degrees 2-theta and a peak at 16.3 ± 0.2 degrees 2-theta.

84. The method of claim 83 further comprising:
(e) isolating the phentolamine mesylate of claim 1.

85. A method for making the phentolamine mesylate of claim 1, comprising:
(a) heating a mixture comprising phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta and dichloromethane at about 50 °C for about two hours to provide a heated mixture;
(b) allowing the heated mixture to cool at about 25 °C for about two hours to provide cooled phentolamine mesylate; and
(c) repeating steps (a) and (b) for up to about three days to provide the phentolamine mesylate that exhibits an XRPD pattern comprising a peak at 8.4 ± 0.2 degrees 2-theta and a peak at 16.3 ± 0.2 degrees 2-theta.

86. The method of claim 85 further comprising:
(d) isolating the phentolamine mesylate of claim 1.

87. A method for making the phentolamine mesylate of claim 1, comprising:
(a) dissolving phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta in a mixture comprising methyl ethyl ketone and water at about 80 °C to provide a solution;
(b) cooling the solution to about 45 °C and adding one or more seeds of phentolamine mesylate that exhibits an XRPD pattern comprising a peak at 8.4 ± 0.2 degrees 2-theta and a peak at 16.3 ± 0.2 degrees 2-theta to provide a seeded mixture;
(c) cooling the seeded mixture at 10 °C for about thirty minutes to provide a cooled mixture;
(d) heating the cooled mixture at 35°C under vacuum to reduce the volume of the mixture by about 33% and provide a reduced mixture;
(e) cooling the reduced mixture at 10 °C for about forty minutes to provide the phentolamine mesylate that exhibits an XRPD pattern comprising a peak at 8.4 ± 0.2 degrees 2-theta and a peak at 16.3 ± 0.2 degrees 2-theta.

88. The method of claim 87 further comprising:
(f) isolating the phentolamine mesylate of claim 1.

89. A method for making the phentolamine mesylate of claim 1, comprising:
(a) heating a mixture comprising phentolamine mesylate that exhibits an XRPD pattern comprising a peak at 6.87 ± 0.2 degrees 2-theta, a peak at 11.65 ± 0.2 degrees 2-theta, a peak at 13.15 ± 0.2 degrees 2-theta, a peak at 18.86 ± 0.2 degrees 2-theta, a peak at 20.32 ± 0.2 degrees 2-theta, a peak at 20.85 ± 0.2 degrees 2-theta, a peak at 21.07 ± 0.2 degrees 2-theta, and a peak at 21.36 ± 0.2 degrees 2-theta, methyl ethyl ketone, and water to about 77 °C to provide a heated mixture;
(b) cooling the heated mixture to about 10 °C to provide a cooled mixture; and
(c) repeating steps (a) and (b) twice to provide the phentolamine mesylate that exhibits an XRPD pattern comprising a peak at 8.4 ± 0.2 degrees 2-theta and a peak at 16.3 ± 0.2 degrees 2-theta.

90. The method of claim 89 further comprising:
(d) isolating the phentolamine mesylate of claim 1.

91. A method for making the phentolamine mesylate of claim 6, comprising:
(a) heating phentolamine mesylate that exhibits an XRPD pattern comprising a peak at 8.4 ± 0.2 degrees 2-theta and a peak at 16.3 ± 0.2 degrees 2-theta to about 126 °C to provide heated phentolamine mesylate; and
(b) allowing the heated phentolamine mesylate to cool to room temperature to provide the phentolamine mesylate of claim 6.

92. The method of claim 91, further comprising:
(c) isolating the phentolamine mesylate of claim 6.

93. A method for making phentolamine mesylate that exhibits an XRPD pattern that is substantially the same as that depicted in **Fig. 4A,** comprising:
(a) heating a mixture comprising phentolamine mesylate that exhibits an XRPD pattern that is substantially the same as that depicted in **Fig. 1A** or **Fig. 1C** and acetone at about 50 °C for about two hours to provide a heated mixture;
(b) allowing the heated mixture to cool at about 25 °C for about two hours to provide cooled phentolamine mesylate; and
(c) repeating steps (a) and (b) for up to about three days to provide the phentolamine mesylate that exhibits an XRPD pattern that is substantially the same as that depicted in **Fig. 4A.**

94. The method of claim 93, further comprising:
(d) isolating the phentolamine mesylate that exhibits an XRPD pattern that is substantially the same as that depicted in **Fig. 4A.**

95. A method for making phentolamine mesylate that exhibits an XRPD pattern that is substantially the same as that depicted in **Fig. 6A,** comprising:
(a) pulverizing phentolamine mesylate that exhibits an XRPD pattern that is substantially the same as that depicted in **Fig. 1A** or **Fig. 1C** in the presence of an excess of 1,4-dioxane solvent to provide a pulverized mixture; and
(b) removing the 1,4-dioxane solvent from the pulverized mixture to provide the phentolamine mesylate that exhibits an XRPD pattern that is substantially the same as that depicted in **Fig. 6A.**

96. The method of claim 95, further comprising:
(c) isolating the phentolamine mesylate that exhibits an XRPD pattern that is substantially the same as that depicted in **Fig. 6A.**

97. The phentolamine mesylate of any one of claims 1-18 comprising less than 5% by weight of phentolamine mesylate that exhibits an XRPD pattern that is substantially the same as that depicted in **Fig. 1A.**

98. The phentolamine mesylate of any one of claims 1-18 comprising less than 3% by weight of phentolamine mesylate that exhibits an XRPD pattern that is substantially the same as that depicted in **Fig. 1A.**

99. The phentolamine mesylate of any one of claims 1-18 comprising less than 1% by weight of phentolamine mesylate that exhibits an XRPD pattern that is substantially the same as that depicted in **Fig. 1A.**

100. The phentolamine mesylate of any one of claims 1-18, wherein the phentolamine mesylate does not comprising phentolamine mesylate that exhibits an XRPD pattern that is substantially the same as that depicted in **Fig. 1A.**

101. The phentolamine mesylate of any one of claims 1-18 comprising less than 5% by weight of phentolamine mesylate that exhibits an XRPD pattern that is substantially the same as that depicted in **Fig. 1C.**

102. The phentolamine mesylate of any one of claims 1-18 comprising less than 3% by weight of phentolamine mesylate that exhibits an XRPD pattern that is substantially the same as that depicted in **Fig. 1C.**

103. The phentolamine mesylate of any one of claims 1-18 comprising less than 1% by weight of phentolamine mesylate that exhibits an XRPD pattern that is substantially the same as that depicted in **Fig. 1C.**

104. The phentolamine mesylate of any one of claims 1-18, wherein the phentolamine mesylate does not comprising phentolamine mesylate that exhibits an XRPD pattern that is substantially the same as that depicted in **Fig. 1C.**

105. The method of any one of claims 58-70 comprising administering to the subject in need thereof an effective amount of:
the phentolamine mesylate that exhibits an XRPD pattern that is substantially the same as that depicted in **Fig. 1A;**
the composition comprising (i) the phentolamine mesylate that exhibits an XRPD pattern that is substantially the same as that depicted in **Fig. 1A** and (ii) a pharmaceutically acceptable carrier or vehicle; or
the composition comprising a pharmaceutically acceptable aqueous carrier or vehicle in which the phentolamine mesylate that exhibits an XRPD pattern that is substantially the same as that depicted in **Fig. 1A** is dissolved.

106. The method of any one of claims 58-70 comprising administering to the subject in need thereof an effective amount of:
the phentolamine mesylate that exhibits an XRPD pattern that is substantially the same as that depicted in **Fig. 1C;**
the composition comprising (i) the phentolamine mesylate that exhibits an XRPD pattern that is substantially the same as that depicted in **Fig. 1C** and (ii) a pharmaceutically acceptable carrier or vehicle; or
the composition comprising a pharmaceutically acceptable aqueous carrier or vehicle in which the phentolamine mesylate that exhibits an XRPD pattern that is substantially the same as that depicted in **Fig. 1C** is dissolved.
